# EUROPEAN PATENT SPECIFICATION

(11) **EP 2 443 228 B1**
(45) Date of publication and mention of the grant of the patent: **20.07.2016**
(21) Application number: 10789964.3
(22) Date of filing: 10.06.2010
(51) Int. Cl.: C12N 1/21, C12P 7/06, C12P 21/00

(54) **ZYMOMONAS WITH IMPROVED ARABINOSE UTILIZATION**
ZYMOMONE MIT VERBESSERTER ARABINOSE-NUTZUNG
ZYMOMONAS AVEC UTILISATION D'ARABINOSE AMÉLIORÉE

(30) Priority: 18.06.2009 US 218164 P; 18.06.2009 US 218166 P
(43) Date of publication of application: 25.04.2012
(73) Proprietor: E. I. du Pont de Nemours and Company, Wilmington, DE 19805 (US)
(72) Inventor: YANG, Jianjun, Hockessin, DE 19707 (US)
(74) Representative: Wilkins, Christopher George
(86) International application number: PCT/US2010/038121
(87) International publication number: WO 2010/147835

(56) References cited:
- WO-A1-03/095627
- JP-A- 2009 050 236
- US-A1- 2003 162 271
- US-A1- 2006 141 586
- HASONA ET AL.: 'Pyruvate Formate Lyase and Acetate Kinase Are Essential for Anaerobic Growth of Escherichia coli on Xylose.' J BACTERIOL. vol. 186, no. 22, 2004, pages 7593 - 7600, XP002634712
- DEANDA ET AL.: 'Development of an Arabinose-Fermenting Zymomonas mobilis Strain by Metabolic Pathway Engineering.' APPL ENVIRON MICROBIOL. vol. 62, no. 12, December 1996, pages 4465 - 4470, XP002912122
- KENT ET AL.: 'Biological Research Survey for the Efficient Conversion of Biomass to Biofuels.' SANDIA REPORT, [Online] 2007, XP008154243 Retrieved from the Internet: <URL:http://prod.sandia.gov/techlib/access- controLcgU2006/067221.pdf> [retrieved on 2010-07-14]
- DARUWALLA ET AL.: 'Energization of the transport systems for arabinose and comparison with galactose transport in Escherichia coli.' BIOCHEM. J. vol. 200, 1981, pages 611 - 627, XP008154244

## Description

### STATEMENT OF GOVERNMENT RIGHTS

This invention was made with United States Government support under Contract No. DE-FC36-07GO17056 awarded by the Department of Energy. The U.S. Government has certain rights in this invention.

### FIELD OF THE INVENTION

The invention relates to the fields of microbiology and fermentation. More specifically, engineering of *Zymomonas* strains to confer improved arabinose utilization, and methods of making ethanol using the strains are described.

### BACKGROUND OF THE INVENTION

Production of ethanol by microorganisms provides an alternative energy source to fossil fuels and is therefore an important area of current research. It is desirable that microorganisms producing ethanol, as well as other useful products, be capable of using xylose and arabinose as carbon sources since these are the predominant pentose sugars in hydrolyzed lignocellulosic materials, which can provide an abundantly available, low cost source of carbon substrate for biocatalysts to use in fermentation.

*Zymomonas mobilis* and other bacterial ethanologens which do not naturally utilize xylose and arabinose may be genetically engineered for utilization of these sugars. To provide for xylose utilization, strains have been engineered to express genes encoding the following proteins: 1) xylose isomerase, which catalyses the conversion of xylose to xylulose; 2) xylulokinase, which phosphorylates xylulose to form xylulose 5-phosphate; 3) transketolase; and 4) transaldolase (US 5514583, US 6566107; Zhang et al. (1995) Science 267:240-243). To provide for arabinose utilization, additional genes encoding the following proteins have been introduced: 1) L-arabinose isomerase to convert L-arabinose to L-ribulose, 2) L-ribulokinase to convert L-ribulose to L-ribulose-5-phosphate, and 3) L-ribulose-5-phosphate-4-epimerase to convert L-ribulose-5-phosphate to D-xylulose (US5843760).

Though some strains of *Z mobilis* have been engineered for arabinose utilization, typically only a low percentage of the arabinose present in a fermentation medium is utilized by these engineered strains. There remains a need to improve arabinose utilization in *Zymomonas* and other bacterial ethanologens to enhance ethanol production when fermentation is in arabinose containing media.

### SUMMARY OF THE INVENTION

The present invention relates to strains of *Zymomonas* and *Zymobacter* that are genetically engineered to have improved ability to use arabinose by introducing a gene for expression of an arabinose-proton symporter, and to production of ethanol using these strains. These strains have improved production of ethanol when grown in media containing arabinose.

Accordingly, the invention provides a recombinant microorganism of the genus *Zymomonas* or *Zymobacter* that utilizes arabinose to produce ethanol, said microorganism comprising at least one heterologous gene encoding an arabinose-proton symporter, wherein said symporter is expressed by said microorganism.

In addition, the invention provides a process for generating a recombinant microorganism of the genus *Zymomonas* or *Zymobacter* that has increased arabinose utilization comprising:
a) providing a recombinant *Zymomonas* or *Zymobacter* strain that utilizes arabinose to produce ethanol under suitable conditions; and
b) introducing at least one gene encoding a heterologous arabinose-proton symporter to the strain of (a) for expression of said smyporter.

In another embodiment the invention provides a process for producing ethanol comprising:
a) providing a recombinant *Zymomonas* or *Zymobacter* strain that utilizes arabinose to produce ethanol, said strain comprising at least one heterologous gene encoding an arabinose-proton symporter, wherein said symporter is expressed by said microorganism;
b) culturing the strain of (a) in a medium comprising arabinose whereby arabinose is converted by said strain to ethanol.

In another embodiment the invention provides a method for improving arabinose utilization by an arabinose-utilizing microorganism comprising:
(a) providing an arabinose-utlizing microorganism wherein said microorganism is selected from the group consisting of a recombinant *Zymomonas* or *Zymobacter* strain that utilizes arabinose to produce ethanol;
(b) introducing into the genome of said microorganism at least one heterologous gene encoding an arabinose-proton symporter wherein said symporter is expressed by said microorganism; and
(c) contacting the microorganism of (b) with a medium comprising arabinose, wherein said microorganism metabolizes said arabinose at an increased rate as compared to said microorganism that is lacking the arabinose-proton symporter.

### BRIEF DESCRIPTION OF THE FIGURES AND SEQUENCE DESCRIPTIONS

The invention can be more fully understood from the following detailed description, the Figures, and the accompanying sequence descriptions that form a part of this application.
Figure 1 shows a diagram of the ethanol fermentation pathway in *Zymomonas* engineered for xylose and arabinose utilization, where glf means glucose-facilitated diffusion transporter.
Figure 2 is a drawing of a plasmid map of pARA205.
Figure 3 is a drawing of a plasmid map of pARA354.
Figure 4 shows graphs of growth and metabolite profiles of ZW705 (A), ZW705-ara354 (B), and ZW705-ara354A7 (C) in MRM3A5 during a 96-hour time course.
Figure 5 shows graphs of growth and metabolite profiles of ZW705 (A), ZW705-ara354 (B), and ZW705-ara354A7 (C) in MRM3A2.5X2.5G5 during a 96-hour time course.
Figure 6 is a drawing of a plasmid map of pARA112.
Figure 7 is a drawing of a plasmid map of pARA113.
Figure 8 shows graphs of growth and metabolite profiles of ZW705-ara354A7 (A), ZW705-ara354A7-ara112-2 (B), and ZW705-ara354A7-ara112-3 (C) in MRM3A5 during a 96-hour time course.
Figure 9 shows graphs of growth and metabolite profiles of ZW705-ara354A7 (A), ZW705-ara354A7-ara112-2 (B), and ZW705-ara354A7-ara112-3 (C) in MRM3A2.5X2.5G5 during a 96-hour time course
Figure 10 shows graphs of growth and metabolite profiles of ZW705-ara354 (A), ZW705-ara354-ara112-1 (B), and ZW705-ara354-ara112-2 (C) in MRM3A5 during a 96-hour time course.
Figure 11 shows graphs of growth and metabolite profiles of ZW705-ara354 (A), ZW705-ara354-ara112-1 (B), and ZW705-ara354-ara112-2 (C) in MRM3A2.5X2.5G5 during a 96-hour time course.
Figure 12 shows graphs of growth and metabolite profiles of ZW801-ara354 (A), ZW801-ara354-ara112-5 (B), and ZW801-ara354-ara112-6 (C) in MRM3A5 during a 96-hour time course.
Figure 13 shows graphs of growth and metabolite profiles of ZW801-ara354 (A), ZW801-ara354-ara112-5 (B), and ZW801-ara354-ara112-6 (C) in MRM3A2.5X2.5G5 during a 96-hour time course.

The following sequences conform with 37 C.F.R. 1.821-1.825 ("Requirements for Patent Applications Containing Nucleotide Sequences and/or Amino Acid Sequence Disclosures - the Sequence Rules") and consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

**Table 1. Protein and coding region SEQ ID NOs for arabinose-proton symporters encoded by araE**

| Organism | SEQ ID NO: coding region | SEQ ID NO: peptide |
|---|---|---|
| *E. coli* | 1 | 2 |
| *Shigella flexneri* | 3 | 4 |
| *Shigella boydii* | 5 | 6 |
| *Shigella dysenteriae* | 7 | 8 |
| *Salmonella typhimurium* | 9 | 10 |
| *Salmonella enterica* | 11 | 12 |
| *Klebsiella pneumoniae* | 13 | 14 |
| *Klebsiella oxytoca* | 15 | 16 |
| *Enterobacter cancerogenus* | 17 | 18 |
| *Bacillus amyloliquefaciens* | 19 | 20 |

SEQ ID NOs:21 and 22 are the amino acid sequence and coding region, respectively, for the araA gene of *E. coli.*
SEQ ID NOs:23 and 24 are the amino acid sequence and coding region, respectively, for the araB gene of *E. coli.*
SEQ ID NOs:25 and 26 are the amino acid sequence and coding region, respectively, for the araD gene of *E*. *coli.*
SEQ ID NO:27 is the nucleotide sequence of the araB-araA DNA fragment PCR product.
SEQ ID NOs:28 and 29 are the nucleotide sequences of primers for PCR amplification of the araB-araA DNA fragment.
SEQ ID NO:30 is the nucleotide sequence of the araD DNA fragment PCR product, ilncluding RBS and 3' UTR.
SEQ ID NOs:31 and 32 are the nucleotide sequences of primers for PCR amplification of the araD DNA fragment, Including RBS and 3' UTR.
SEQ ID NO:33 is the nucleotide sequence of the Pgap promoter of *Z*. *mobilis.*
SEQ ID NOs:34 and 35 are the nucleotide sequences of primers for PCR amplification of the Pgap promoter DNA fragment.
SEQ ID NO:36 is the nucleotide sequence of the Pgap promoter DNA fragment PCR product.
SEQ ID NOs:37 and 38 are the nucleotide sequences of primers for PCR amplification of the spectinomycin resistance cassette.
SEQ ID NOs:39 and 40 are the nucleotide sequences of primers for mutagenesis of Pgap to remove the added Ncol site.
SEQ ID NO:41 is the nucleotide sequence of the pARA205 plasmid.
SEQ ID NOs:42 and 43 are the nucleotide sequences of primers for PCR amplification of the LDH-L DNA fragment.
SEQ ID NO:44 is the nucleotide sequence of the LDH-L DNA fragment PCR product.
SEQ ID NOs:45 and 46 are the nucleotide sequences of primers for PCR amplification of the LDH-R DNA fragment.
SEQ ID NO:47 is the nucleotide sequence of the LDH-R DNA fragment PCR product.
SEQ ID NO:48 is the nucleotide sequence of the LoxPw-aadA-LoxPw DNA fragment PCR product.
SEQ ID NO:49 is the nucleotide sequence of the pARA354 plasmid.
SEQ ID NOs:50 and 51 are the nucleotide sequences of primers for PCR amplification to check 5' integration of P_{gap}-araBAD-aadA.
SEQ ID NOs:52 and 53 are the nucleotide sequences of primers for PCR amplification to check 3' integration of P_{gap}-araBAD-aadA.
SEQ ID NOs:54 and 55 are the nucleotide sequences of primers for PCR amplification of the araE coding region DNA fragment.
SEQ ID NO:56 is the nucleotide sequence of the araE DNA fragment PCR product.
SEQ ID NOs:57 and 58 are the nucleotide sequences of primers for PCR amplification of the araFGH DNA fragment.
SEQ ID NO:59 is the nucleotide sequence of the araFGH DNA fragment PCR product.
SEQ ID NOs:60 and 61 are the nucleotide sequences of primers for PCR amplification of the *Actinoplanes missouriensis* P_{gi} DNA fragment.
SEQ ID NO:62 is the nucleotide sequence of the *Actinoplanes missouriensis* GI promoter in the plasmid used as PCR template.
SEQ ID NO:63 is the nucleotide sequence of the *Actinoplanes missouriensis* P_{gi} DNA fragment PCR product.
SEQ ID NO:64 is the nucleotide sequence of the chloramphenicol resistance marker.
SEQ ID NO:65 is the nucleotide sequence of the pARA112 plasmid.
SEQ ID NO:66 is the nucleotide sequence of the pARA113 plasmid.

### DETAILED DESCRIPTION

The present invention describes improved arabinose-utilizing recombinant *Zymomonas* or *Zymobacter* strains that are further engineered to express an arabinose-proton symporter, and a process for engineering the strains by introducing a gene encoding an arabinose-proton symporter. In other aspects, the present invention describes processes for improving arabinose utilization, and for producing ethanol in media comprising arabinose, using said strains. The arabinose-utilizing strains expressing an arabinose-proton symporter have improved arabinose utilization and are useful for producing ethanol in media comprising arabinose.

Ethanol produced by the present strains with improved arabinose utilization may be used as an alternative energy source to fossil fuels.

The following abbreviations and definitions will be used for the interpretation of the specification and the claims.

As used herein, the terms "comprises," "comprising," "includes," "induding," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

"Gene" refers to a nucleic acid fragment that expresses a specific protein, which may include regulatory sequences preceding (5' non-coding sequences) and following (3' non-coding sequences) the coding sequence. "Native gene" or "wild type gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes.

The term *"araE'* refers to a gene or genetic construct that encodes a bacterial arabinose-proton symporter protein which is a low affinity and high capacity arabinose transporter with a Km of 1.25 x 10⁻⁴ M. Genes encoding the arabinose-proton symporter protein may be isolated from a multiplicity of bacteria and those from enteric bacteria, such as *Escherichia, Klebsiella, Salmonella,* and *Shigella* are particularly useful in the present invention.

The term "arabinose utilization" when used in the context of a microorganism refers to the ability of that microorganism to utilize arabinose for the production of products, particularly ethanol.

The term "adapted strain" refers to a microorganism that has been selected for growth on a particular carbon source in order to improve it's ability use that carbon source for the production of products. An "arabinose adapted strain" for example is a strain of microorganism that has been selected for growth on high concentrations of arabinose.

The term "genetic construct" refers to a nucleic acid fragment that encodes for expression of one or more specific proteins. In the genetic construct the gene may be native, chimeric, or foreign in nature. Typically a genetic construct will comprise a "coding sequence". A "coding sequence" refers to a DNA sequence that codes for a specific amino acid sequence.

"Promoter" or "Initiation control regions" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed in most cell types at most times are commonly referred to as "constitutive promoters".

The term "expression", as used herein, refers to the transcription and stable accumulation of sense (mRNA) or antisense RNA derived from a gene. Expression may also refer to translation of mRNA into a polypeptide. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts or fragments capable of suppressing the expression of identical or substantially similar foreign or endogenous genes (U.S. 5,231,020).

The term "transformation" as used herein, refers to the transfer of a nucleic acid fragment into a host organism, resulting in genetically stable inheritance. The transferred nucleic acid may be in the form of a plasmid maintained in the host cell, or some transferred nucleic acid may be integrated into the genome of the host cell. Host organisms containing the transformed nucleic acid fragments are referred to as "transgenic" or "recombinant" or "transformed" organisms.

The terms "plasmid" and "vector" as used herein, refer to an extra chromosomal element often carrying genes which are not part of the central metabolism of the cell, and usually in the form of circular double-stranded DNA molecules. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

The term "operably linked" refers to the association of nucleic acid sequences on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "selectable marker" means an identifying factor, usually an antibiotic or chemical resistance gene, that is able to be selected for based upon the marker gene's effect, i.e., resistance to an antibiotic, wherein the effect is used to track the inheritance of a nucleic acid of interest and/or to identify a cell or organism that has inherited the nucleic acid of interest.

As used herein the term "codon degeneracy" refers to the nature in the genetic code permitting variation of the nucleotide sequence without affecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

The term "codon-optimized" as it refers to genes or coding regions of nucleic acid molecules for transformation of various hosts, refers to the alteration of codons in the gene or coding regions of the nucleic acid molecules to reflect the typical codon usage of the host organism without altering the polypeptide encoded by the DNA.

The term "carbon source" refers to sugars such as oligosaccharides and monosaccharides that can be used by a microorganism in a fermentation process ("fermentable sugar") to produce a product suh as ethanol. A microorganism may have the ability to use a single carbon source for the production of a product and as such the carbon source is refereed to herein as a "sole" carbon source.

The term "lignocellulosic" refers to a composition comprising both lignin and cellulose. Lignocellulosic material may also comprise hemicellulose.

The term "cellulosic" refers to a composition comprising cellulose and additional components, including hemicellulose.

The term "saccharification" refers to the production of fermentable sugars or carbon sources from polysaccharides.

The term "pretreated biomass" means biomass that has been subjected to pretreatment prior to saccharification.

"Biomass" refers to any cellulosic or lignocellulosic material and includes materials comprising cellulose, and optionally further comprising hemicellulose, lignin, starch, oligosaccharides and/or monosaccharides. Biomass may also comprise additional components, such as protein and/or lipid. Biomass may be derived from a single source, or biomass can comprise a mixture derived from more than one source; for example, biomass could comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn cobs, crop residues such as corn husks, corn stover, grasses, wheat, wheat straw, barley straw, hay, rice straw, switchgrass, waste paper, sugar cane bagasse, sorghum bagasse or stover, soybean stover, components obtained from milling of grains, trees, branches, roots, leaves, wood chips, sawdust, shrubs and bushes, vegetables, fruits, flowers and animal manure.

"Biomass hydrolysate" refers to the product resulting from saccharification of biomass. The biomass may also be pretreated or preprocessed prior to saccharification.

The term "heterologous" means not naturally found in the location of interest. For example, a heterologous gene refers to a gene that is not naturally found in the host organism, but that is introduced into the host organism by gene transfer. For example, a heterologous nucleic acid molecule that is present in a chimeric gene is a nucleic acid molecule that is not naturally found associated with the other segments of the chimeric gene, such as the nucleic acid molecules having the coding region and promoter segments not naturally being associated with each other.

As used herein, an "isolated nucleic acid molecule" is a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.

A nucleic acid fragment is "hybridizable" to another nucleic acid fragment, such as a cDNA, genomic DNA, or RNA molecule, when a single-stranded form of the nucleic acid fragment can anneal to the other nucleic acid fragment under the appropriate conditions of temperature and solution ionic strength. Hybridization and washing conditions are well known and exemplified in Sambrook, J., Fritsch, E. F. and Maniatis, T. Molecular Cloning: A Laboratoy Manual, 2nd ed., Cold Spring Harbor Laboratory: Cold Spring Harbor, NY (1989), particularly Chapter 11 and Table 11.1 therein. The
conditions of temperature and ionic strength determine the "stringency" of the hybridization. Stringency conditions can be adjusted to screen for moderately similar fragments (such as homologous sequences from distantly related organisms), to highly similar fragments (such as genes that duplicate functional enzymes from closely related organisms). Post-hybridization washes determine stringency conditions. One set of preferred conditions uses a series of washes starting with 6X SSC, 0.5% SDS at room temperature for 15 min, then repeated with 2X SSC, 0.5% SDS at 45 °C for 30 min, and then repeated twice with 0.2X SSC, 0.5% SDS at 50 °C for 30 min. A more preferred set of stringent conditions uses higher temperatures in which the washes are identical to those above except for the temperature of the final two 30 min washes in 0.2X SSC, 0.5% SDS was increased to 60 °C. Another preferred set of highly stringent conditions uses two final washes in 0.1 X SSC, 0.1% SDS at 65 °C. An additional set of stringent conditions include hybridization at 0.1 X SSC, 0.1 % SDS, 65 °C and washes with 2X SSC, 0.1 % SDS followed by 0.1 X SSC, 0.1% SDS, for example.

Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridizations with shorter nucleic acids, i.e., oligonucleotides, the position of mismatches becomes more important, and the length of the oligonucleotide determines its specificity (see Sambrook et al., supra, 11.7-11.8). In one embodiment the length for a hybridizable nucleic acid is at least about 10 nucleotides. Preferably a minimum length for a hybridizable nucleic acid is at least about 15 nucleotides; more preferably at least about 20 nucleotides; and most preferably the length is at least about 30 nucleotides. Furthermore, the skilled artisan will recognize that the temperature and wash solution salt concentration may be adjusted as necessary according to factors such as length of the probe.

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Altschul, S. F., et al., J. Mol. Biol., 215:403-410 (1993)). In general, a sequence of ten or more contiguous amino acids or thirty or more nucleotides is necessary in order to putatively identify a polypeptide or nucleic acid sequence as homologous to a known protein or gene. Moreover, with respect to nucleotide sequences, gene specific oligonucleotide probes comprising 20-30 contiguous nucleotides may be used in sequence-dependent methods of gene identification (e.g., Southern hybridization) and isolation (e.g., in situ hybridization of bacterial colonies or bacteriophage plaques). In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence. The instant specification teaches the complete amino acid and nucleotide sequence encoding particular fungal proteins. The skilled artisan, having the benefit of the sequences as reported herein, may now use all or a substantial portion of the disclosed sequences for purposes known to those skilled in this art.

The term "complementary" is used to describe the relationship between nucleotide bases that are capable of hybridizing to one another. For example, with respect to DNA, adenosine is complementary to thymine and cytosine is complementary to guanine.

The terms "homology" and "homologous" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments disclosed herein such as deletion or insertion of one or more nucleotides that do not substantially alter the functional properties of the resulting nucleic acid fragment relative to the initial, unmodified fragment. It is therefore understood, as those skilled in the art will appreciate, that more than the specific exemplary sequences may be used in the strains and methods of the invention.

Moreover, the skilled artisan recognizes that homologous nucleic acid sequences disclosed herein are also defined by their ability to hybridize, under moderately stringent conditions (e.g., 0.5 X SSC, 0.1% SDS, 60 °C) with the sequences exemplified herein, or to any portion of the nucleotide sequences disclosed herein and which are functionally equivalent to any of the nucleic acid sequences disclosed herein.

The term "percent identity", as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in: 1.) Computational Molecular Biology (Lesk, A. M., Ed.) Oxford University: NY (1988); 2.) Biocomputing: Informatics and Genome Projects (Smith, D. W., Ed.) Academic: NY (1993); 3.) Computer Analysis of Sequence Data, Part I (Griffin, A. M., and Griffin, H. G., Eds.) Humania: NJ (1994); 4.) Sequence Analysis in Molecular Biology (von Heinje, G., Ed.) Academic (1987); and
5.) Sequence Analysis Primer (Gribskov, M. and Devereux, J., Eds.) Stockton: NY (1991).

Preferred methods to determine identity are designed to give the best match between the sequences tested. Methods to determine identity and similarity are codified in publicly available computer programs. Sequence alignments and percent identity calculations may be performed using the MegAlign^{™} program of the LASERGENE bioinformatics computing suite (DNASTAR Inc., Madison, WI). Multiple alignment of the sequences is performed using the "Clustal method of alignment" which encompasses several varieties of the algorithm including the "Clustal V method of alignment" corresponding to the alignment method labeled Clustal V (described by Higgins and Sharp, CABIOS. 5:151-153 (1989); Higgins, D.G. et al., Comput. Appl. Biosci., 8:189-191 (1992)) and found in the MegAlign^{™} program of the LASERGENE bioinformatics computing suite (DNASTAR Inc.). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program. Additionally the "Clustal W method of alignment" is available and corresponds to the alignment method labeled Clustal W (described by Higgins and Sharp, CABIOS. 5:151-153 (1989); Higgins, D.G. et al., Comput. Appl. Biosci. 8:189-191 (1992)) and found in the MegAlign^{™} v6.1 program of the LASERGENE bioinformatics computing suite (DNASTAR Inc.). Default parameters for multiple alignment (GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

It is well understood by one skilled in the art that many levels of sequence identity are useful in identifying polypeptides, from other species, wherein such polypeptides have the same or similar function or activity. Useful examples of percent identities include, but are not limited to: 24%, 30%, 35%, 40%, 45%, 50%, 55%, 60%, 65%, 70%, 75%, 80%, 85%, 90%, or 95%, or any integer percentage from 24% to 100% may be useful in describing the present invention, such as 25%, 26%, 27%, 28%, 29%, 30%, 31%, 32%, 33%, 34%, 35%, 36%, 37%, 38%, 39%, 40%, 41 %, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98% or 99%. Suitable nucleic acid fragments not only have the above homologies but typically encode a polypeptide having at least 50 amino acids, preferably at least 100 amino acids, more preferably at least 150 amino acids, still more preferably at least 200 amino acids, and most preferably at least 250 amino acids.

The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1.) the GCG suite of programs (Wisconsin Package Version 9.0, Genetics Computer Group (GCG), Madison, WI); 2.) BLASTP, BLASTN, BLASTX (Altschul et al., J. Mol. Biol., 215:403-410 (1990)); 3.) DNASTAR (DNASTAR, Inc. Madison, WI); 4.) Sequencher (Gene Codes Corporation, Ann Arbor, MI); and 5.) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

Standard recombinant DNA and molecular cloning techniques used here are well known in the art and are described by Sambrook, J., Fritsch, E. F. and Maniatis, T. Molecular Cloning: A Laboratory Manual, 2nd ed.; Cold Spring Harbor Laboratory: Cold Spring Harbor, New York, 1989 (hereinafter "Maniatis"); and by Silhavy, T. J., Bennan, M. L. and Enquist, L. W. Experiments with Gene Fusions; Cold Spring Harbor Laboratory: Cold Spring Harbor, New York, 1984; and by Ausubel, F. M. et al., In Current Protocols in Molecular Biology, published by Greene Publishing and Wiley-Interscience, 1987.

The present invention relates to engineered strains of arabinose-utilizing *Zymomonas* or *Zymobacter* that have improved arabinose utilization when fermented in arabinose containing media, and to processes for ethanol production using the strains. A challenge for improving ethanol production by fermentation of a biocatalyst in media that includes biomass hydrolysate, produced typically by pretreatment and saccharification of biomass, is obtaining efficient utilization of arabinose. Arabinose is one of the predominant pentose sugars in hydrolyzed lignocellulosic materials, the other being xylose. Applicants have discovered that expression of an arabinose-proton symporter leads to increased efficiency in arabinose utilization by arabinose-utilizing strains, and thus to higher ethanol yields when fermentation is in arabinose containing media.

### Arabinose-utilizing host strain

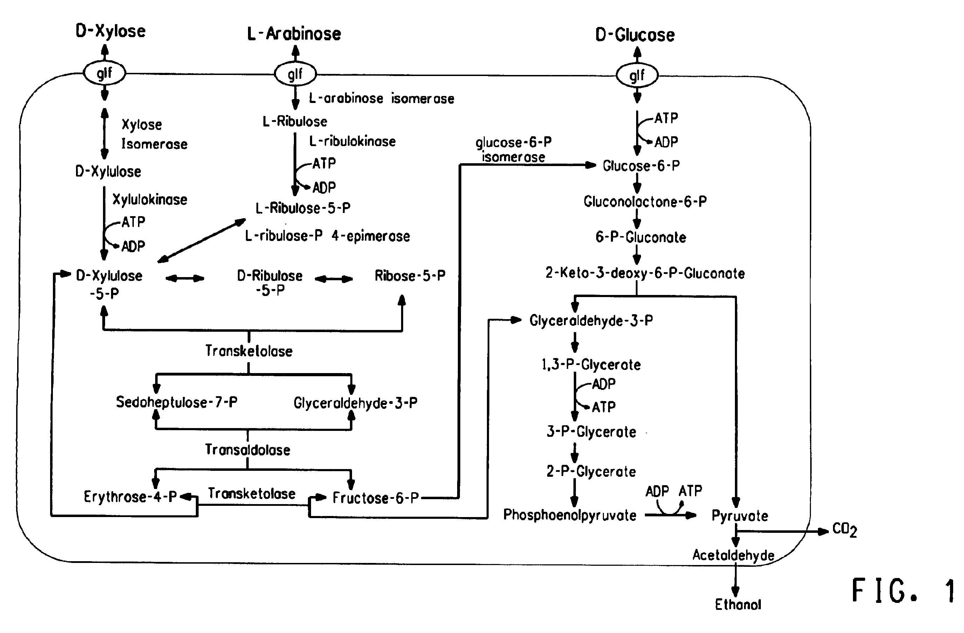

Any strain of *Zymomonas* or *Zymobacter* that is able to utilize arabinose as a carbon source may be used as a host for preparing the strains of the present invention. Strains of *Zymomonas,* such as Z. *mobilis* that have been engineered for arabinose fermentation to ethanol are particularly useful. *Zymomonas* has been engineered for arabinose utilization by introducing genes encoding 1) L-arabinose isomerase to convert L-arabinose to L-ribulose, 2) L-ribulokinase to convert L-ribulose to L-ribulose-5-phosphate, and 3) L-ribulose-5-phosphate-4-epimerase to convert L-ribulose-5-phosphate to D-xylulose (US5843760 and described in Examples 1 and 2 herein; see diagram in Figure 1). DNA sequences encoding these enzymes may be obtained from any microorganisms that are able to metabolize arabinose. Sources for the coding regions include *Klebsiella, Escherichia, Rhizobium, Agrobacterium,* and *Salmonella.* Particularly useful are the coding regions of *E. coli* which are for L-arabinose isomerase: coding region of araA (coding region SEQ ID NO:21; protein SEQ ID NO:22), for L-ribulokinase: coding region of araB (coding region SEQ ID NO:23; protein SEQ ID NO:24), and for L-ribulose-5-phosphate-4-epimerase: coding region of araD (coding region SEQ ID NO:25; protein SEQ ID NO:26). These proteins and their coding regions may be readily identified in other arabinose utilizing microorganisms, such as those listed above, by one skilled in the art using bioinformatics or experimental methods as described below for araE.

In addition, transketolase and transaldolase activities are used in the biosynthetic pathway from arabinose to ethanol (see Figure 1). Transketolase and transaldolase are two enzymes of the pentose phosphate pathway that convert xylulose 5-phosphate to intermediates that couple pentose metabolism to the glycolytic Entner-Douderoff pathway permitting the metabolism of arabinose or xylose to ethanol. These may be endogenous activities, or endogenous activities may complement introduced activities for these enzymes.

Typically, arabinose-utilizing *Zymomonas* is also engineered for xylose utilization. Typically four genes have been introduced into *Z mobilis* for expression of four enzymes involved in xylose metabolism (Figure 1) as described in US 5514583. These include genes encoding transketolase and transaldolase as described above, as well as xylose isomerase, which catalyzes the conversion of xylose to xylulose and xylulokinase, which phosphorylates xylulose to form xylulose 5-phosphate (see Figure 1). DNA sequences encoding these enzymes may be obtained from any of numerous
microorganisms that are able to metabolize xylose, such as enteric bacteria, and some yeasts and fungi. Sources for the coding regions include *Xanthomonas, Klebsiella, Escherichia, Rhodobacter, Flavobacterium, Acetobacter, Gluconobacter, Rhizobium, Agrobacterium, Salmonella, Pseudomonads,* and *Zymomonas.* Particularly useful are the coding regions of *E. coli.*

For expression, the encoding DNA sequences for arabinose-utilizing proteins and xylose-utilizing protiens are operably linked to promoters that are expressed in *Z*. *mobilis* cells, and transcription terminators. Examples of promoters that may be used include the promoters of the Z. *mobilis* glyceraldehyde-3-phosphate dehydrogenase encoding gene (GAP promoter; Pgap), of the *Z*. *mobilis* enolase encoding gene (ENO promoter; Peno), and of the *Actinoplanes missouriensis* xylose isomerase encoding gene (GI promoter, Pgi). The coding regions may be individually expressed from a promoter typically as a chimeric gene, or two or more coding regions may be joined in an operon with expression from the same promoter. The resulting chimeric genes and/or operons are typically constructed in or transferred to a vector for further manipulations.

Vectors are well known in the art. Particularly useful for expression in *Zymomonas* are vectors that can replicate in both *E. coli* and *Zymomonas,* such as pZB188 which is described in U.S. Pat. No. 5,514,583. Vectors may include plasmids for autonomous replication in a cell, and plasmids for carrying constructs to be integrated into the cell genome. Plasmids for DNA integration may include transposons, regions of nucleic acid sequence homologous to the target cell genome, site-directed integration sequences, or other sequences supporting integration. In homologous recombination, DNA sequences flanking a target integration site are placed bounding a spectinomycin-resistance gene, or other selectable marker, and the desired chimeric gene leading to insertion of the selectable marker and chimeric gene into the target genomic site as described in Example 2 herein. In addition, the selectable marker may be bounded by site-specific recombination sites, so that after expression of the corresponding site-specific recombinase, the resistance gene may be excised from the genome.

Xylose-utilizing strains that are of particular use include CP4(pZB5) (US 5514583), ATCC31821/pZBS (US 6566107), 8b (US 20030162271; Mohagheghi et al., (2004) Biotechnol. Lett. 25; 321-325), and ZW658 with derivatives ZW800 and ZW801-4 (commonly owned and co-pending US Patent App. Pub. #US20080286870; deposited, ATTCC # PTA-7858). Also ZW705 may be used, which is described in commonly owned and co-pending US Patent App. Pub. #US 20110014670. Arabinose utilizing strains that may be used are disclosed in US5843760, as well as being described herein in Examples 1 and 2.

### Adaptation for Arabinose utilization

A *Z*. *mobilis* strain engineered for xylose and arabinose utilization as described above was found by Applicants to utilize about 33% of arabinose in media where arabinose is the sole carbon source (at 50 g/L), and about 68% of arabinose in media including mixed sugars of 25 g/L arabinose, 25 g/L xylose, and 50 g/L glucose in test growth conditions. In an attempt to derive a strain with improved arabinose utilization, applicants adapted cells from the xylose and arabinose utilizing strain by serial growth in media with 50 g/L arabinose as the sole carbon source as described herein in Example 2. Using this process, isolated strains were obtained that had a substantial improvement in arabinose utilization in media where arabinose is the sole carbon source, which are arabinose-adapted strains. For example, one strain used about 83% of arabinose in media where 50 g/L arabinose is the sole carbon source. In mixed sugars media containing 25 g/L arabinose, 25 g/L xylose, and 50 g/L glucose, there was less improvement: about 74% of arabinose was used. Also in mixed sugars media arabinose utilization was delayed as compared to utilization of glucose and xylose.

To obtain strains with improved arabinose utilization, strains engineered for expression of arabinose utilization genes as described above may be adapted by serial growth in media containing arabinose as the sole carbon source in concentrations between about 20 g/L and 100 g/L, or higher. Adaptation may be in lower concentrations of arabinose, but with initial growth in about 20 g/L or higher. Serial growth is typically for at least about 25 doublings. Adaptation may be before or after introducing a heterologous arabinose-proton symporter, that is described below, to an arabinose utilizing strain. In addition, cells may be adapted both before and after introduction of a heterologous arabinose-proton symporter.

### Discovery for engineering improved arabinose utilization

Applicants engineered xylose and arabinose utilizing strains of *Zymomonas* for expression of the two different arabinose transport systems present in *E. coli.* The two systems are 1) an ABC transporter consisting of three proteins encoded by araFGH: 33 kD preiplasmic arabinose binding protein encoded by araF, 55 kD membrane bound ATPase encoded by araG, and 34 kD membrane bound protein encoded by araH; and 2) an arabinose-proton symporter consisting of one protein: 52 kD arabinose-proton symporter encoded by araE. The ABC transporter is a high affinity and low capacity arabinose transporter with a Km of 3 x 10⁻⁴ M, while the arabinose-proton symporter is a low affinity and high capacity arabinose transporter with a Km of 1.25 x 10⁻⁴ M. Applicants found that expression of the ABC transporter actually resulted in reduced arabinose utilization in arabinose only media. Expression of the arabinose-proton symporter increased arabinose utilization in both arabinose only media and mixed sugars media. Thus applicants have discovered that the *E. coli* ABC transporter does not improved arabinose utilization while the arabinose-proton symporter does improve arabinose utilization in *Zymomonas.* With expression of the arabinose-proton symporter, arabinose utilization was greatly increased in both arabinose only media and in mixed sugars media.

Expression of an arabinose-proton symporter increased arabinose utilization in all strains tested. These include an arabinose and xylose utilizing *Z*. *mobilis* strain with no adaptation, an arabinose and xylose utilizing *Z*. *mobilis* strain that had been adapted for xylose utilization in stress conditions (disclosed in commonly owned and co-pending US Patent App. Pub. #US 20110014670), and
an arabinose and xylose utilizing *Z*. *mobilis* strain that had been adapted for xylose utilization in stress conditions and also for arabinose utilization as described herein above and in Example 2. In strains without arabinose adaptation, arabinose utilization was increased by at least about 28% in arabinose only media as well as in mixed sugars media. Also in an arabinose adapted strain, arabinose utilization was increased by at least about 28% in mixed sugars media. In arabinose only media the level of arabinose utilization in the arabinose adapted parental strain without expression of the arabinose-proton symporter is already at about 80%, and therefore the increase in arabinose utilization cannot exceed 20%, and is about 18%.

Thus any *Zymomonas* or *Zymobacter* strain that is capable of utilizing arabinose, also called an arabinose utilizing strain, may be used to create the present strains. Particularly useful are strains that additionally utilize xylose and glucose. In these strains arabinose utilization is improved by at least about 10% by expressing an arabinose-proton symporter. Arabinose utilization may be improved by at least about 10%, 12%, 16%,18%, 20%, 24%, 28%, or more. The % improvement may vary depending on the growth conditions used including the type of media and the parental microorganism used for engineering expression of the arabinose-proton symporter, as well as the specific resulting engineered strain. Factors causing variation include level of expression of the introduced arabinose-proton symporter and resulting transporter activity level, which may vary between transformants.

### Expression of an arabinose-proton symporter

In the present engineered *Zymomonas* or *Zymobacter* cells any bacterial arabinose-proton symporter may be expressed to provide increased arabinose utilization. Bacterial arabinose-proton symporter proteins and their encoding sequences for expression in *Zymomonas* or *Zymobacter* are heterologous, as they are not naturally found in *Zymomonas* or *Zymobacter.* Examples of arabinose-proton symporter protein and encoding sequences that may be expressed include those encoded by the araE genes of *E. coli* (coding region SEQ ID NO:1; protein SEQ ID NO:2), *Shigella flexneri* (coding region SEQ ID NO:3; protein SEQ ID NO:4), *Shigella boydii* (coding region SEQ ID NO:5; protein SEQ ID NO:6), *Shigella dysenteriae* (coding region SEQ ID NO:7; protein SEQ ID NO:8), *Salmonella typhimurium* (coding region SEQ ID NO:9; protein SEQ ID NO:10), *Salmonella enterica* (coding region SEQ ID N0:11; protein SEQ ID NO:12), *Klebsiella pneumoniae* (coding region SEQ ID NO13; protein SEQ ID NO:14), *Klebsiella oxytoca* (coding region SEQ ID NO:15; protein SEQ ID NO:16), *Enterobacter cancerogenus* (coding region SEQ ID NO:17; protein SEQ ID NO:18) and *Bacillus amyloliquefaciens* (coding region SEQ ID NO:19; protein SEQ ID NO:20).

Because the sequences of arabinose-proton symporter coding regions and the encoded proteins are well known, as exemplified in the SEQ ID NOs listed above and given in Table 1, additional suitable arabinose-proton symporters may be readily identified by one skilled in the art on the basis of sequence similarity using bioinformatics approaches. Typically BLAST (described above) searching of publicly available databases with known arabinose-proton symporter amino acid sequences, such as those provided herein, is used to identify additional arabinose-proton symporters, and their encoding sequences, that may be used in the present strains. These proteins may have at least about 80-85%, 85%-90%, 90%- 95% or 95%-99% sequence identity to any of the arabinose-proton symporters of SEQ ID NOS:2, 4, 6, 8, 10, 12, 14, 16, 18, or 20 while having arabinose-proton symporter activity. Identities are based on the Clustal W method of alignment using the default parameters of GAP PENALTY=10, GAP LENGTH PENALTY=0.1, and Gonnet 250 series of protein weight matrix.

In addition to using protein or coding region sequence and bioinformatics methods to identify additional arabinose-proton symporters, the sequences described herein or those recited in the art may be used to experimentally identify other homologs in nature. For example each of the arabinose-proton symporter encoding nucleic acid fragments described herein may be used to isolate genes encoding homologous proteins. Isolation of homologous genes using sequence-dependent protocols is well known in the art. Examples of sequence-dependent protocols include, but are not limited to: 1.) methods of nucleic acid hybridization; 2.) methods of DNA and RNA amplification, as exemplified by various uses of nucleic acid amplification technologies [e.g., polymerase chain reaction (PCR), Mullis et al., U.S. Patent 4,683,202; ligase chain reaction (LCR), Tabor, S. et al., Proc. Acad. Sci. USA 82:1074 (1985); or strand displacement amplification (SDA), Walker, et al., Proc. Natl. Acad. Sci. U.S.A., 89:392 (1992)]; and 3.) methods of library construction and screening by complementation.

For example, coding regions for similar proteins or polypeptides to the arabinose-proton symporter encoding sequences described herein could be isolated directly by using all or a portion of the instant nucleic acid fragments as DNA hybridization probes to screen libraries from any desired organism using methodology well known to those skilled in the art. Specific oligonucleotide probes based upon the disclosed nucleic acid sequences can be designed and synthesized by methods known in the art (Maniatis, *supra*)*.* Moreover, the entire sequences can be used directly to synthesize DNA probes by methods known to the skilled artisan (e.g., random primers DNA labeling, nick translation or end-labeling techniques), or RNA probes using available *in vitro* transcription systems. In addition, specific primers can be designed and used to amplify a part of (or full-length of) the instant sequences. The resulting amplification products can be labeled directly during amplification reactions or labeled after amplification reactions, and used as probes to isolate full-length DNA fragments by hybridization under conditions of appropriate stringency.

Typically, in PCR-type amplification techniques, the primers have different sequences and are not complementary to each other. Depending on the desired test conditions, the sequences of the primers should be designed to provide for both efficient and faithful replication of the target nucleic acid. Methods of PCR primer design are common and well known in the art (Thein and Wallace, "The use of oligonucleotides as specific hybridization probes in the Diagnosis of Genetic Disorders", in Human Genetic Diseases: A Practical Approach, K. E. Davis Ed., (1986) pp 33-50, IRL: Hemdon, VA; and Rychlik, W., In Methods in Molecular Biology, White, B. A. Ed., (1993) Vol. 15, pp 31-39, PCR Protocols: Current Methods and Applications. Humania: Totowa, NJ).

Generally two short segments of the described sequences may be used in polymerase chain reaction protocols to amplify longer nucleic acid fragments encoding homologous genes from DNA or RNA. The polymerase chain reaction may also be performed on a library of cloned nucleic acid fragments wherein the sequence of one primer is derived from the described nucleic acid fragments, and the sequence of the other primer takes advantage of the presence of the polyadenylic acid tracts to the 3' end of the mRNA precursor encoding microbial genes.

Alternatively, the second primer sequence may be based upon sequences derived from the cloning vector. For example, the skilled artisan can follow the RACE protocol (Frohman et al., PNAS USA 85:8998 (1988)) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (e.g., BRL, Gaithersburg, MD), specific 3' or 5' cDNA fragments can be isolated (Ohara et al., PNAS USA 86:5673 (1989); Loh et al., Science 243:217 (1989)).

Alternatively, the described arabinose-proton symporter encoding sequences may be employed as hybridization reagents for the identification of homologs. The basic components of a nucleic acid hybridization test include a probe, a sample suspected of containing the gene or gene fragment of interest, and a specific hybridization method. Probes are typically single-stranded nucleic acid sequences that are complementary to the nucleic acid sequences to be detected. Probes are "hybridizable" to the nucleic acid sequence to be detected. The probe length can vary from 5 bases to tens of thousands of bases, and will depend upon the specific test to be done. Typically a probe length of about 15 bases to about 30 bases is suitable. Only part of the probe molecule need be complementary to the nucleic acid sequence to be detected. In addition, the complementarity between the probe and the target sequence need not be perfect. Hybridization does occur between imperfectly complementary molecules with the result that a certain fraction of the bases in the hybridized region are not paired with the proper complementary base.

Hybridization methods are well defined. Typically the probe and sample must be mixed under conditions that will permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and sample nucleic acid may occur. The concentration of probe or target in the mixture will determine the time necessary for hybridization to occur. The higher the probe or target concentration, the shorter the hybridization incubation time needed. Optionally, a chaotropic agent may be added. The chaotropic agent stabilizes nucleic acids by inhibiting nuclease activity. Furthermore, the chaotropic agent allows sensitive and stringent hybridization of short oligonucleotide probes at room temperature (Van Ness and Chen, Nucl. Acids Res. 19:5143-5151 (1991)). Suitable chaotropic agents include guanidinium chloride, guanidinium thiocyanate, sodium thiocyanate, lithium tetrachloroacetate, sodium perchlorate, rubidium tetrachloroacetate, potassium iodide and cesium trifluoroacetate, among others. Typically, the chaotropic agent will be present at a final concentration of about 3 M. If desired, one can add formamide to the hybridization mixture, typically 30-50% (v/v).

Various hybridization solutions can be employed. Typically, these comprise from about 20 to 60% volume, preferably 30%, of a polar organic solvent. A common hybridization solution employs about 30-50% v/v formamide, about 0.15 to 1 M sodium chloride, about 0.05 to 0.1 M buffers (e.g., sodium citrate, Tris-HCl, PIPES or HEPES (pH range about 6-9)), about 0.05 to 0.2% detergent (e.g., sodium dodecylsulfate), or between 0.5-20 mM EDTA, FICOLL (Pharmacia Inc.) (about 300-500 kdal), polyvinylpyrrolidone (about 250-500 kdal) and serum albumin. Also included in the typical hybridization solution will be unlabeled carrier nucleic acids from about 0.1 to 5 mg/mL, fragmented nucleic DNA (e.g., calf thymus or salmon sperm DNA, or yeast RNA), and optionally from about 0.5 to 2% wt/vol glycine. Other additives may also be included, such as volume exclusion agents that include a variety of polar water-soluble or swellable agents (e.g., polyethylene glycol), anionic polymers (e.g., polyacrylate or polymethylacrylate) and anionic saccharidic polymers (e.g., dextran sulfate).

Nucleic acid hybridization is adaptable to a variety of assay formats. One of the most suitable is the sandwich assay format. The sandwich assay is particularly adaptable to hybridization under non-denaturing conditions. A primary component of a sandwich-type assay is a solid support. The solid support has adsorbed to it or covalently coupled to it immobilized nucleic acid probe that is unlabeled and complementary to one portion of the sequence.

Expression of an arabinose-proton symporter is achieved by transforming with a sequence encoding an arabinose-proton symporter. As known in the art, there may be variations in DNA sequences encoding an amino acid sequence due to the degeneracy of the genetic code. The coding sequence may be codon-optimized for maximal expression in the target *Zymomonas* or *Zymobacter* host cell, as well known to one skilled in the art. Typically a chimeric gene including a promoter active in *Zymomonas* cells that is operably linked to the desired coding region, as well as a transcription terminator, is used for expression. Any promoter that is active in *Zymomonas* cells may be used, such as the examples cited above for expression of proteins for arabinose utilization. A chimeric gene constructed with a promoter and arabinose-symporter coding region is a heterologous gene for expression in *Zymomonas* or *Zymobacter* since the coding region is from a different organism as described above. Vectors for expression and/or integration are as described above for expression of proteins for arabinose utilization.

### Improved Ethanol Production

The present strains have improved arabinose utilization in media with arabinose as the only carbohydrate source and in media with mixed sugars including arabinose. The present strains also have improved ethanol production. As compared to the parental strain prior to introduction of an arabinose-proton symporter expression gene, ethanol production of the strain expressing an arabinose-proton symporter is increased. The increase in ethanol production may vary depending on the media and growth conditions used in fermentation as well as the arabinose-proton symporter expressing strain used as the biocatalyst. Typically ethanol production may be increased by at least about 10%, and may be increased by about 10%, 12%, 16%,18%, 20%, 24%, 28%, or more.

### Fermentation of improved arabinose-utilizing strain

An engineered arabinose-utilizing strain expressing an arabinose-proton symporter and genes or operons for expression of L-arabinose isomerase, L-ribulokinase, L-ribulose-5-phosphate-4-epimerase, transaldolase and transketolase may be used in fermentation to produce a product that is a natural product of the strain, or a product that the strain is engineered to produce. For example, *Zymomonas mobilis* and *Zymobacter palmae* are natural ethanolagens. Preferred are strains that also utilize xylose and are engineered in addition for expression of xylose isomerase and xylulokinase. As an example, production of ethanol by a Z. *mobilis* strain of the invention, that utilizes xylose and arabinose, is described. Z *mobilis* also utilizes glucose naturally.

For production of ethanol, recombinant xylose and arabinose-utilizing Z. *mobilis* expressing an arabinose-proton symporter is brought in contact with medium that contains arabinose. Typically the medium contains mixed sugars including arabinose, xylose, and glucose. The medium may contain biomass hydrolysate that includes these sugars that are derived from treated cellulosic or lignocellulosic biomass.

When the mixed sugars concentration is high such that growth is inhibited, the medium includes sorbitol, mannitol, or a mixture thereof as disclosed in commonly owned and co-pending US Patent Pub. #US20080081358 A1. Galactitol or ribitol may replace or be combined with sorbitol or mannitol. The *Z. mobilis* grows in the medium where fermentation occurs and ethanol is produced. The fermentation is run without supplemented air, oxygen, or other gases (which may include conditions such as anaerobic, microaerobic, or microaerophilic fermentation), for at least about 24 hours, and may be run for 30 or more hours. The timing to reach maximal ethanol production is variable, depending on the fermentation conditions. Typically, if inhibitors are present in the medium, a longer fermentation period is required. The fermentations may be run at temperatures that are between about 30° C and about 37° C, at a pH of about 4.5 to about 7.5.

The present *Z*. *mobilis* may be grown in medium containing mixed sugars including arabinose in laboratory scale fermenters, and in scaled up fermentation where commercial quantities of ethanol are produced. Where commercial production of ethanol is desired, a variety of culture methodologies may be applied. For example, large-scale production from the present *Z*. *mobilis* strains may be produced by both batch and continuous culture methodologies. A classical batch culturing method is a closed system where the composition of the medium is set at the beginning of the culture and not subjected to artificial alterations during the culturing process. Thus, at the beginning of the culturing process the medium is inoculated with the desired organism and growth or metabolic activity is permitted to occur adding nothing to the system. Typically, however, a "batch" culture is batch with respect to the addition of carbon source and attempts are often made at controlling factors such as pH and oxygen concentration. In batch systems the metabolite and biomass compositions of the system change constantly up to the time the culture is terminated. Within batch cultures cells moderate through a static lag phase to a high growth log phase and finally to a stationary phase where growth rate is diminished or halted. If untreated, cells in the stationary phase will eventually die. Cells in log phase are often responsible for the bulk of production of end product or intermediate in some systems. Stationary or post-exponential phase production can be obtained in other systems.

A variation on the standard batch system is the Fed-Batch system. Fed-Batch culture processes are also suitable for growth of the present Z. *mobilis* strains and comprise a typical batch system with the exception that the substrate is added in increments as the culture progresses. Fed-Batch systems are useful when catabolite repression is apt to inhibit the metabolism of the cells and where it is desirable to have limited amounts of substrate in the medium. Measurement of the actual substrate concentration in Fed-Batch systems is difficult and is therefore estimated on the basis of the changes of measurable factors such as pH and the partial pressure of waste gases such as CO₂. Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA, or Deshpande, Mukund V., Appl. Biochem. Biotechnol., 36, 227, (1992).

Commercial production of ethanol may also be accomplished with a continuous culture. Continuous cultures are open systems where a defined culture medium is added continuously to a bioreactor and an equal amount of conditioned medium is removed simultaneously for processing. Continuous cultures generally maintain the cells at a constant high liquid phase density where cells are primarily in log phase growth. Alternatively, continuous culture may be practiced with immobilized cells where carbon and nutrients are continuously added, and valuable products, by-products or waste products are continuously removed from the cell mass. Cell immobilization may be performed using a wide range of solid supports composed of natural and/or synthetic materials as is known to one skilled in the art.

Continuous or semi-continuous culture allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by medium turbidity, is kept constant. Continuous systems strive to maintain steady state growth conditions and thus the cell loss due to medium being drawn off must be balanced against the cell growth rate in the culture. Methods of modulating nutrients and growth factors for continuous culture processes as well as techniques for maximizing the rate of product formation are well known in the art of industrial microbiology and a variety of methods are detailed by Brock, *supra.*

Particularly suitable for ethanol production is a fermentation regime as follows. The desired *Z. mobilis* strain of the present invention is grown in shake flasks in semi-complex medium at about 30 °C to about 37 °C with shaking at about 150 rpm in orbital shakers and then transferred to a 10 L seed fermentor containing similar medium. The seed culture is grown in the seed fermentor anaerobically until OD₆₀₀ is between 3 and 6, when it is transferred to the production fermentor where the fermentation parameters are optimized for ethanol production. Typical inoculum volumes transferred from the seed tank to the production tank range from about 2% to about 20% v/v. Typical fermentation medium contains minimal medium components such as potassium phosphate (1.0 - 10.0 g/L), ammonium sulfate (0- 2.0 g/L), magnesium sulfate (0 - 5.0 g/L), a complex nitrogen source such as yeast extract or soy based products (0 - 10 gL). A final concentration of about 5 mM sorbitol or mannitol is present in the medium. Mixed sugars including arabinose and at least one additional sugar such as glucose (or sucrose), providing a carbon source, are continually added to the fermentation vessel on depletion of the initial batched carbon source (50-200 g/l) to maximize ethanol rate and titer. Carbon source feed rates are adjusted dynamically to ensure that the culture is not accumulating glucose in excess, which could lead to build up of toxic byproducts such as acetic acid. In order to maximize yield of ethanol produced from substrate utilized, biomass growth is restricted by the amount of phosphate that is either batched initially or that is fed during the course of the fermentation. The fermentation is controlled at pH 5.0 - 6.0 using caustic solution (such as ammonium hydroxide, potassium hydroxide, or sodium hydroxide) and either sulfuric or phosphoric acid. The temperature of the fermentor is controlled at 30 °C - 35 °C. In order to minimize foaming, antifoam agents (any class- silicone based, organic based etc) are added to the vessel as needed. An antibiotic, for which there is an antibiotic resistant marker in the strain, such as kanamycin, may be used optionally to minimize contamination.

In addition, fermentation may be concurrent with saccharification using an SSF (simultaneous saccharification and fermentation) process. In this process sugars are produced from biomass as they are metabolized by the production biocatalyst.

Any set of conditions described above, and additionally variations in these conditions that are well known in the art, are suitable conditions for production of ethanol by an arabinose-utilizing recombinant *Zymomonas* or *Zymobacter* strain that is engineered to express an arabinose-proton symporter by introducing a heterologous coding region of an arabinose-proton symporter.

### EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention and can make various changes and modifications of the invention to adapt it to various uses and conditions that fall within the scope of the appended claims.

### GENERAL METHODS

Standard recombinant DNA and molecular cloning techniques used here are well known in the art and are described by Sambrook, J., Fritsch, E. F. and Maniatis, T., Molecular Cloning: A Laboratory Manual, 2nd ed., Cold Spring Harbor Laboratory: Cold Spring Harbor, NY (1989) (hereinafter "Maniatis"); and by Silhavy, T. J., Bennan, M. L. and Enquist, L. W., Experiments with Gene Fusions, Cold Spring Harbor Laboratory: Cold Spring Harbor, NY (1984); and by Ausubel, F. M. et al., Current Protocols in Molecular Biology, published by Greene Publishing Assoc. and Wiley-Interscience, Hoboken, NJ (1987).

The meaning of abbreviations is as follows: "kb" means kilobase(s), "bp" means base pairs, "nt" means nucleotide(s), "hr" means hour(s), "min" means minute(s), "sec" means second(s), "d" means day(s), "L" means liter(s), "ml" means milliliter(s), "µL" means microliter(s), "µg" means microgram(s), "ng" means nanogram(s), "mM" means millimolar, "µM" means micromolar, "nm" means nanometer(s), "µmol" means micromole(s), "pmol" means picomole(s), "Cm" means chloramphenicol, "Cm^{r}" means chloramphenicol resistant, "Cm^{s}" means chloramphenicol sensitive, "Sp^{r}" means spectinomycin resistance, "Sp^{s}" means spectinomycin sensitive, "UTR" means untranslated region, "RBS" means ribosome binding site.

Primers were synthesized by Sigma (St. Luis, MO) unless otherwise specified

### Example 1

### Construction and Expression of Operon for Arabinose Utilization Proteins in Zymomonas

To engineer *Zymomonas mobilis* for arabinose utilization, the *E*. *coli* araA, araB, and araC coding regions were constructed in an operon with a *Z. mobilis* promoter and expressed on a plasmind in *Z. mobilis* cells. AraB, araA, and araD encode the proteins L-ribulose kinase, L-arabinose isomerase, and L-ribulose-5-phosphate-4-epimerase, respectively, which provide an arabinose assimilation pathway, in conjunction with transketolase and transaldolase activities (see Figure 1).

### 1. Cloning E. coli araBAD coding sequences and Z. mobilis P_{gap} promoter

The araB, araA, and araD coding regions of *E*. *coli* (SEQ ID NOs:23, 21, and 25, respectively) are present in the araBAD operon. An araB-araA DNA fragment (araBA; SEQ ID NO:27) was prepared using oligonucleotide primers ara1 (SEQ ID NO:28) and ara2 (SEQ ID NO:29) which are forward and reverse primers, respectively. Primer ara1 adds the nucleotides CC before the start codon ATG of the araB coding region to create an Ncol site. Primer ara2 adds an XbaI site after the stop codon of the araA coding region. An araD DNA fragment (SEQ ID NO:30) was prepared using oligonucleotide primers ara3 (SEQ ID NO:31) and primer ara4 (SEQ ID NO:32) which are forward and reverse primers , respectively. Primer ara3 adds an Xba site at the 5' end of the ribosome binding site (RBS) sequence 5' to the araD coding region. Primer ara4 adds a Hindlll site after the 3'untranslated region (UTR) that is 3' to the araD coding region. Each pair of primers was used in a standard PCR reaction, including 50 µL AccuPrime Pfx SuperMix (Invitrogene, Carlsbad, CA), 1 µL of 10 µM forward and reverse primers, and 2 µL (approx. 50 to 100 ng) *E*. *coli* genomic DNA prepared from MG1655 (ATCC# 700926; a K12 strain) using a Wizard Genomic DNA Purification Kit (Promega, Madison, WI). A reaction using primers ara1 and ara2 was carried out for 5 min at 95°C, followed by 35 cycles of 30 sec at 95°C/30 sec at 56°C/3.5 min at 68 °C, and ended for 7 min at 68 °C. It resulted in a 3226-bp araB-araA fragment with a 5' Ncol site and a 3' Xbal site (SEQ ID NO:27). Another reaction using primers ara3 and ara4 was carried out using a similar program, except the extension time at 68 °C was shortened to 1.5 min. It produced an 889-bp araD fragment (including the araD 3' UTR) with a 5' XbaI site and a 3' HindIII site (SEQ ID NO:30).

The native *E. coli* promoter for the araBAD operon is an inducible promoter that is not suitable for the desired expression in Z. *mobilis.* The *Z. mobilis* GAP (Glyceraldehydes-3-phosphate dehydrogenase) promoter (P_{gap}; SEQ ID NO:33) was used since it is a strong constitutive promoter for expression in *Z. mobilis.* A DNA fragment containing the *Z*. *mobilis* P_{gap} was prepared using oligonucleotide primers ara10 and ara11. Primer ara10 (SEQ ID NO:34) is a forward primer that adds a SacI and an Apel site at the 5' end of the promoter DNA fragment. Primer ara11 (SEQ ID NO:35) is a reverse primer that changes the last two nucleotides of the promoter from AC to CC, thus it adds an Ncol site at the 3' end of the promoter DNA fragment. These two primers were used in a standard PCR reaction, as described above, using a plasmid containing the P_{gap} as the DNA template to produce a 323-bp P_{gap} promoter DNA fragment with 5' Sacl and Spel sites and a 3' NcoI site (SEQ ID NO:36).

Each of these PCR products was cloned into the TOPO Blunt Zero Vector (Invitrogen, Calsbad, CA) by following the manufacturer's instructions. The resultant plasmids pTP-araB-araA, pTP-araD and pTP-P_{gap} were propagated in *E*. *coli* DH5a cells (Invitrogen) and each was prepared using a Qiagen DNA Miniprep Kit. Their sequences were confirmed by DNA sequencing. 2. Assembling P_{gap}-araBAD operon in a shuttle vector

A P_{gap}-araBAD operon was assembled in a *Zymomonas-E. coli* shuttle vector called pZB188aada, which is based on the vector pZB188 (Zhang et al. (1995) Science 267:240-243; US 5514583) which includes a 2,582 bp Z. *mobilis* genomic DNA fragment containing a replication region allowing the vector to replicate in *Zymomonas* cells. In pZB188aada the tetracycline resistance cassette (Tc^{r}-cassette) of pZB188 was replaced with a spectinomycin resisance cassette (Spec^{r}-cassette). The Spec^{r}-cassette was generated by PCR using plasmid pHP15578 (Cahoon et al, (2003) Nature Biotechnology 21: 1082-1087) as a template and Primers 1 (SEQ ID NO:32 from CL4236) and 2 (SEQ ID NO:33 from CL4236). Plasmid pHP15578 contains the complete nucleotide sequence for the Spec^{r}-cassette and its promoter, which is based on the published sequence of the Tranposon Tn7 aadA gene (GenBank accession number X03043) that codes for 3' (9)-O-nucleotidyltransferase.
Primer 1 (SEQ ID NO:37):
   CTACTCATTTatcgatGGAGCACAGGATGACGCCT
Primer 2 (SEQ ID NO:38):
   CATCTTACTacgcgtTGGCAGGTCAGCAAGTGCC

The underlined bases of Primer 1 (forward primer) hybridize just upstream from the promotor for the SpeC^{r}-Cassette (to nts 4-22 of GenBank accession number X03043), while the lower case letters correspond to a Clal site that was added to the 5' end of the primer. The underlined bases of Primer 2 (reverse primer) hybridize about 130 bases downstream from the stop codon for the SpeC^{r}-cassette (to nts 1002-1020 of GenBank accession number X03043), while the lower case letters correspond to an AflIII site that was added to the 5' end of the primer. The 1048 bp PCR-generated Spec^{r}-cassette was double-digested with Clal and AflIII, and the resulting DNA fragment was purified using the QIAquick PCR Purification Kit (Qiagen, Cat. No. 28104) and the vendor's recommended protocol. Plasmid pZB188 (isolated from *E*. *coli* SSC110 (dcm⁻, dam⁻) in order to obtain non-methylated plasmid DNA for cutting with Clal (which is sensitive to dam methylation) was double-digested with Clal and BssHII to remove the Tc^{r}-cassette, and the resulting large vector fragment was purified by agarose gel electrophoresis. This DNA fragment and the cleaned up PCR product were then ligated together, and the transformation reaction mixture was introduced into *E. coli* JM110 using chemically competent cells that were obtained from Stratagene (Cat. No. 200239). Note that BssHII and AflIII generate compatible "sticky ends", but both sites are destroyed when they are ligated together. Transformants were plated on LB medium that contained spectinomycin (100 µg/ml) and grown at 37 °C. A spectinomycin-resistant transformant that contained a plasmid with the correct size insert was identified by restriction digestion analysis with Notl and named pZB188/aada.

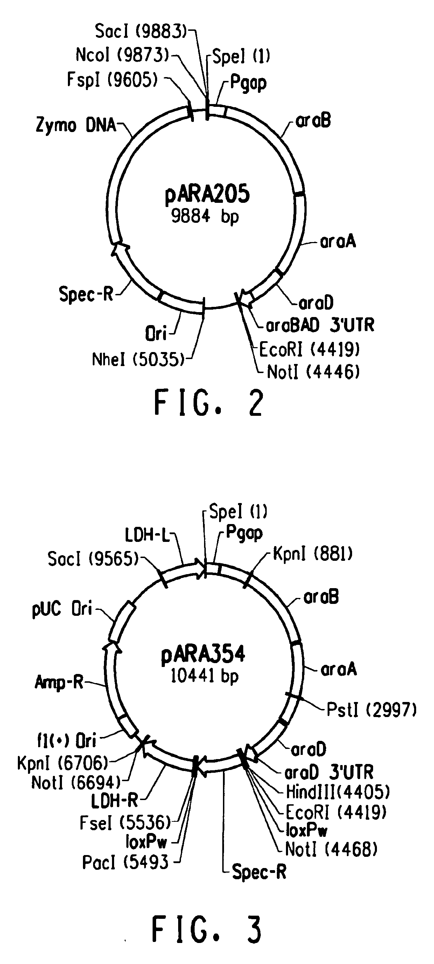

The pTP-P_{gap} Spel - NcoI P_{gap} fragment, the pTP-araB-araA Ncol - XbaI araB-araA fragment, and the pTP-araD Xbal - Notl araD fragment were all cloned into a NotI-SpeI pZB188/aada vector, forming a pZB188aada-based shuttle vector that contained a P_{gap}-araBAD operon. The resulting plasmid, named pARA201, was propagated in E. coli DH5a and prepared using a Qiagen DNA Miniprep Kit. pARA205 (Figure 2; SEQ ID NO:41) was prepared from pARA201 by restoring the nucleotides at the 3' end of P_{gap} from CC back to the original AC nucleotides. This was done using a QickChange XL Site-Directed Mutagenesis Kit (Stratagene, La Jolla, CA). For this mutagenesis, the forward primer ara31 (SEQ ID NO:30) and the reverse primer ara32 (SEQ ID NO:40) were used to make the changes by following the manufacturer's instructions. pARA205 was propagated in E. coli DH5a and prepared using a Qiagen DNA Miniprep Kit.

### 3. Expressing araBAD in Z. mobilis

To confirm that P_{gap}-araBAD is a functional operon in *Z. mobilis*, pARA205 was introduced into *Z. mobilis* strain ZW801-4 for expression. ZW801-4 is a xylose-utilizing strain of *Z. mobilis*. The construction and characterization of strains ZW658, ZW800 and ZW801-4 was described in commonly owned and co-pending U.S. Patent Application Publication US20080286870 A1. ZW658 (ATCC # PTA-7858) was constructed by integrating two operons, *P_{gap}xyIAB* and *P_{gap}taitkt*, containing four xylose-utilizing genes encoding xylose isomerase, xylulokinase, transaldolase and transketolase, into the genome of ZW1 (ATCC #31821) via sequential transposition events, and followed by adaptation on selective media containing xylose. ZW800 is a derivative of ZW658 which has a double-crossover insertion of a spectinomycin resistance cassette in the sequence encoding the glucose-fructose oxidoreductase (GFOR) enzyme to knockout this activity. ZW801-4 is a derivative of ZW800 in which the spectinomycin resistance cassette was deleted by site-specific recombination leaving an in-frame stop codon that prematurely truncates the protein.

Competent cells of ZW801-4 were prepared by growing the seed cells overnight in MRM3G5 (1 % yeast extract, 15 mM KH₂PO₄, 4 mM MgSO₄, and 50 g/L glucose) at 30°C with 150 rpm shaking, up to an OD₆₀₀ value near 5. Cells were harvested and resuspended in fresh medium to an OD₆₀₀ value of 0.05. They were grown further under the same conditions to early or middle log phase (OD₆₀₀ near 0.5). Cells were harvested and washed twice with ice-cold water and then once with ice-cold 10% glycerol. The resultant competent cells were collected and resuspended in ice-cold 10% glycerol to an OD₆₀₀ value near 100. Since transformation of Z. mobilis requires non-methylated DNA, pARA205 plasmid was transformed into *E. coli* SCS110 competent cells (Stratagene). One colony of transformed cells was grown in 10 mL LB-Amp100 (LB broth containing 100 mg/L ampicillin) overnight at 37°C. DNA was prepared from the 10 mL-culture, using a Qiagen DNA Miniprep Kit.

Approximately 500 ng of non-methylated pARA205 plasmid DNA was mixed with 50 µL of ZW801-4 competent cells in a 1 MM Electroporation Cuvette (VWR, West Chester, PA). The plasmid DNA was electroporated into the cells at 2.0 KV using a BT720 Transporater Plus (BTX-Genetronics, San Diego, CA). The transformed cells were recovered in 1 mL MMG5 medium (50 g/L glucose, 10 g/L yeast extract, 5 g/L tryptone, 2.5 g/L (NH₄)₂SO₄, 0.2 g/L K₂HPO₄, and 1 mM MgSO₄) for 4 hours at 30°C and grown on MMG5-Spec250 plates (MMG5 with 250 mg/L spectinomycin and 15 g/L agar) for 2 days at 30°C, inside an anaerobic jar with an AnaeroPack (Mitsubishi Gas Chemical, New York, NY). Individual colonies were streaked onto a MMA5-Spec250 plate (as same as MMG5-Spec250 but glucose was replaced by 50 g/L arabinose) and a new MMG5-Spec250 plate in duplicate. Under the same conditions as described above, the streaks grew well although growth on the MMA5-Spec250 plate took longer time. This indicated that the P_{gap}-araBAD operon was expressed.

Two streaks of the transformed cells growing on the MMG5-Spec250 plate (ZW801-ara205-4 and ZW801-ara205-5) were selected for a 72-hour growth assay. In the assay, cells from each streak were grown overnight in 2 mL MRM3G5-Spec250 (MRM3G5 with 250 mg/L spectinomycin) at 30°C with 150 rpm shaking. Cells were harvested, washed with MRM3A5 (same as MRM3G5 but glucose was replaced by arabinose), and resuspended in MRM3A5-Spec250 (MRM3A5 containing 250 mg/L spectinomycin) to have a start OD₆₀₀ at 0.1. Four mL of the suspension were placed in a 14 mL capped Falcon tube and grown for 72 hours at 30°C with 150 rpm shaking. At the end of growth, OD₆₀₀ was measured. Then, 1 mL of the culture was centrifuged at 10,000x g to remove cells. The supernatant was filtered through a 0.22 µm Costar Spin-X Centrifuge Tube Filter (Corning Inc, Corning, NY) and analyzed by running through a BioRad Aminex HPX-A7H ion exclusion column (BioRad, Hercules, CA) with 0.01 N H₂SO₄ at a speed of 0.6 mL/min at 55°C on an Agilent 1100 HPLC system (Agilent Technologies, Santa Clara, CA) to determine ethanol and sugar concentrations. In parallel, ZW801-4 was grown (without antibiotics) and analyzed as a control. The results given in Table 2 demonstrate that expression of araBAD enabled *Z. mobilis* ZW801-4 to grow and produce ethanol using arabinose as the sole carbon source.

**Table 2 72-hour growth assay for ZW801-ara205 strains in MRM3A5**

| Strain | Growth (OD₆₀₀) | Ethanol (g/L) | Arabinoase (g/L) |
|---|---|---|---|
| ZW801-4 | 0.106 | 0 | 51.20 |
| ZW801-ara205-4 | 1.75 | 7.22 | 33.15 |
| ZW801-ara205-5 | 1.96 | 10.68 | 27.16 |

### Example 2

### Integration of Arabinose Utilization Operon into the Z. mobilis Genome and Characterization of Resulting Strains

This example describes stable integration of the P_{gap}-araBAD operon into two xylose-utilizing strains of Z. mobilis. 1. Building P_{gap}-araBAD operon into a suicide vector.

To integrate the P_{gap}-araBAD operon into the genome of *Z. mobilis*, a suicide vector for DCO (double cross over) homologous recombination was prepared. Besides P_{gap}-araBAD, this vector included DCO homologous recombination fragments to direct integration of P_{gap}-araBAD and an aadA gene to provide a selective marker for spectinomycin resistance. We chose the IdhA locus as the insertion site. Two IdhA DNA fragments for DCO, LDH-L and LDH-R, were synthesized by PCR using *Z. mobilis* ZW801-4 DNA as template. The reaction used AccuPrime Mix and followed the standard PCR procedure described in Example 1. The LDH-L DNA fragment was synthesized using forward primer ara20 (SEQ ID NO:42) and reverse primer ara21 (SEQ ID NO:43). The resulting product was an 895-bp DNA fragment including sequence 5' to the IdhA coding region and nucleotides 1-493 of the IdhA coding region, with a 5' SacI site and a 3' Spel site (SEQ ID NO:44). The LDH-R DNA fragment was synthesized using forward primer ara22 (SEQ ID NO:45) and reverse primer ara23 (SEQ ID NO:46). The resulting product was a 1169 bp fragment including nucleotides 494-996 of the IdhA coding region and sequence 3' to the IdhA coding region, with a 5' EcoRI site and a 3' NotI site (SEQ ID NO:47).

pBS SK(+) (a Bluescript plasmid; Stratagene) was used as a suicide vector since pBS vectors cannot replicate in *Zymomonas.* pARA354 (SEQ ID NO:49) was constructed by cloning the P_{gap}-araBAD operon of pARA205, the LDH-L fragment, and the LDH-R fragment into pBS SK(+). In addition a DNA fragment containing the aadA marker (for spectinomycin resistance) bounded by wild type LoxP sites (LoxPw-aadA-LoxPw fragment; SEQ ID NO:48) was included in pARA354. pARA354 has the P_{gap} -araBAD operon and LoxPw-aadA-LoxPw marker fragment located between the LDH-L and LDH-R sequences.

Figure 3 shows a map of the 10,441 bp pARA354. It has an f1(+) origin and an ampicillin resistance gene for plasmid propagation in *E*. *coli.* Since LDH-L and LDH-R contained the first 493 base pairs and the remaining 503 base pairs of the IdhA coding sequence, respectively, pARA354 was designed to direct insertion of P_{gap}-araBAD and aadA into the IdhA coding sequence of *Z. mobilis* between nucleotides #493 and #494 by crossover recombination.

### 2. Developing the P_{gap}-araBAD integration strains

*Z. mobilis* strain ZW705 is an engineered strain of *Z. mobilis,* with improved xylose utilization in stress conditions that was derived from ZW801-4 by adaptation in continuous culture as described in co-pending and commonly owned US Patent Application US 20110014670. ZW801-4 xylose-utilizing *Zymomonas* cells were continuously grown in medium comprising at least about 50 g/L xylose to produce a culture comprising ethanol, then ammonia and acetic acid were added creating a stress culture. The cells were further continuously grown in the stress culture and cells with improved xylose utilization were isolated, including the ZW705 strain.

To transform pARA354 into both ZW705 and ZW801-4 strains, 800 ng non-methylated plasmid DNA was electroporated into 50 µL competent cells prepared from each strain. DNA demethylation, competent cell preparation, and electroporation were performed as described in Example 1. Colonies of transformed cells of each strain were grown on a MMG5-Spec250 plate for 2 days at 30°C inside an anaerobic jar with an AnaeroPack. Because pARA354 could not replicate in Z. mobilis, spectinomycin resistance indicated these colonies were integration strains. The colonies were streaked on to a new MMG5-Spec250 plate and a MMA5-Spec250 plate, in duplicate, and grown for 2 days and 4 days respectively. Their growth on the MMA5-Spec250 plate also indicated the integration. To further demonstrate the integration, the junctions between the P_{gap}-araBAD-aadA fragment and *Z. mobilis* genomic DNA were inspected by the standard 35-cycle PCR reaction, containing PCR Super Mix (Invitrogen), a pair of primers, and the tested transformed cells. One PCR cycle included 45 seconds denaturing at 95°C, 45 seconds annealing at 58°C, and 2 minutes extension at 72°C. Primer ara45 (SEQ ID NO:50) and primer ara42 (SEQ ID NO:51) were a forward primer located at upstream of the LDH-L sequence in the *Z. mobilis* genomic DNA and a reverse primer located in the araB gene of pARA354, respectively. This pair of primers amplified a 1694-bp fragment from all colonies inspected by PCR. Also used were primer ara46 (SEQ ID NO:52) and primer ara43 (SEQ ID NO:53) which area forward primer located in the aadA gene of pARA354 and a reverse primer located downstream of the LDH-R sequence in Z. mobilis genomic DNA, respectively. This pair of primers amplified a 1521-bp fragment from all colonies inspected by PCR. Therefore, the P_{gap}-araBAD-aadA fragment had been integrated into ZW801-4 and ZW705 genomes successfully by the DCO approach. Because DCO homologous recombination was a target specific integration, every colony resulting from the integration in ZW801-4 or ZW705 would have the identical genotype. A colony from each of the integrations was grown in 5 mL MRMG5-Spec250 overnight at 30°C with 150 rpm shaking. Cells were collected by centrifugation, resuspended in 0.5 mL 50% glycerol, and then stored at -80°C. The strains were named ZW705-ara354 and ZW801-ara354.

To further improve function of the integrated P_{gap}-araBAD operon, the ZW705-ara354 strain was subjected to adaptation. For this purpose, an overnight culture of ZW705-ara354 was collected by centrifugation, washed with MRM3A5, and resuspended in MRM3A5-Spec250 with OD₆₀₀ at 0.1. Four mL of this suspension was placed in a 14 mL Falcon capped tube and grown for 72 hours in a 30°C 150 rpm shaker, until the OD₆₀₀ was above 1. Then the culture was inoculated to a new falcon tube containing 4 mL fresh MRM3A5-Spec250 to reach a starting OD₆₀₀ near 0.1 for a second run of growth. Totally, 9 successive runs were completed. Each run brought the OD₆₀₀ from approximately 0.1 to above 1 and took 3 to 4 days, except the 4^{th} run which took 6 days since the cells grew much more slowly. In order to characterize the adapted strains, the 9^{th} run was diluted 100-fold, and 10 µL of the dilution was spread and grown on a MMA5-Spec250 plate for 3 days at 30°C in an anaerobic jar with an AnaeroPack. Individual colonies (i.e. adaptation strains) were picked and grown overnight in 3 mL MRM3G5-Spec250 on a 30°C 150 rpm shaker. They were subjected to the 72-hour growth assay in MRM3A5-Spec250, as described in Example 1. ZW705-ara354 strain was used as a control in the assay. Analysis data for 5 adaptation strains (ZW705-ara354A4 to A8) are presented in Table 3, showing that all adaptation strains performed better than ZW705-ara354. ZW705-ara354A7 was the best strain in terms of growth, ethanol production, and arabinose utilization.

**Table 3. 72-hour growth assay for adaptation strains of ZW705-ara354 in MRM3A5**

| Strain | Growth (OD₆₀₀) | Ethanol (g/L) | Arabinoase (g/L) |
|---|---|---|---|
| ZW705-ara354 | 1.03 | 9.10 | 32.71 |
| ZW705-ara354A4 | 3.29 | 19.03 | 10.31 |
| ZW705-ara354A5 | 3.71 | 18.56 | 10.07 |
| ZW705-ara354A6 | 3.61 | 18.47 | 9.23 |
| ZW705-ara354A7 | 4.04 | 19.73 | 7.36 |
| ZW705-ara354A8 | 2.96 | 17.37 | 12.18 |

### 3. Characterizing growth and metabolite profiles of the P_{gap}-araBAD integration strains, with and without adaptation.

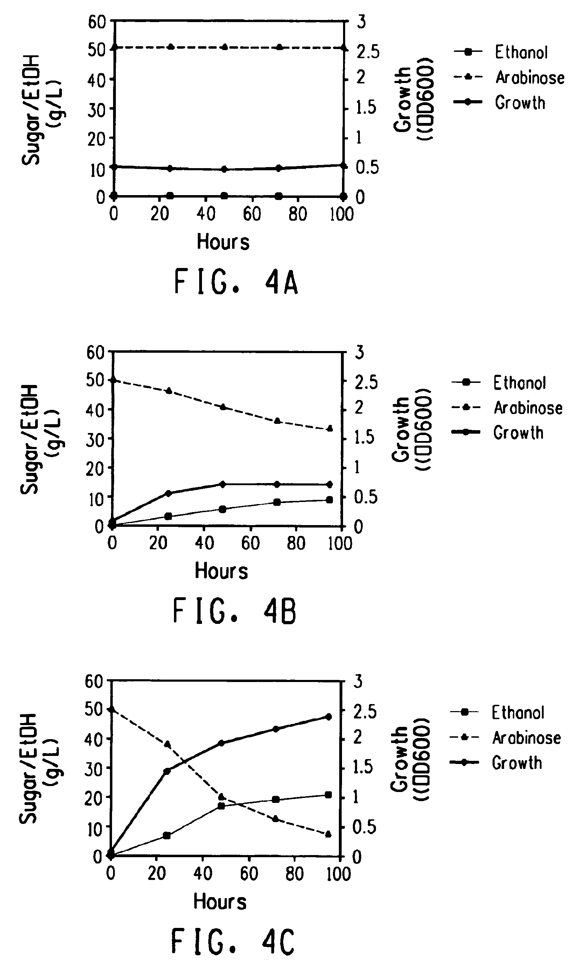

The P_{gap}-araBAD integration strains were further characterized for their ability to utilize arabinose to support cell growth and ethanol production in media containing arabinose as the sole carbon source and in media containing mixed sugars. To characterize these strains in medium containing arabinose as the sole carbon source, first ZW705-ara354 and ZW705-ara354A7 cells were grown overnight in 2 mL MRM3G5-Spec250 in a 30°C 150 rpm shaker. Cells were harvested, washed with MRM3A5, and resuspended in MRM3A5-Spec250 at a starting OD₆₀₀ of 0.1. Twenty mL of the suspension were placed in a 50 mL screw capped VWR centrifuge tube and grown at 30°C with 150 rpm shaking for a 96-hour time course. During the time course, OD₆₀₀ was measured at 0-, 24-, 48-, 72-, and 96-hour, respectively. At each time point, 1 mL of culture was removed and centrifuged at 10,000x g to remove cells. The supernatant was filtered through a 0.22 µm Costar Spin-X Centrifuge Tube Filter and analyzed for ethanol and sugar concentrations by running through a BioRad Aminex HPX-A7H ion exclusion column with 0.01 N H₂SO₄ using a speed of 0.6 mL/min at 55°C on an Agilent 1100 HPLC system. In parallel, ZW705 was grown in media without antibiotics and analyzed as a control. The results are given in Figure 4. These results indicate that, without P_{gap}-araBAD, ZW705 could not metabolize arabinose and could not grow when arabinose was the sole carbon source (Figure 4A). After integration of P_{gap}-araBAD, ZW705-ara354 was able to utilize arabinose to support growth and produce ethanol (Figure 4B). The maximum rate of arabinose consumption was 0.2 g/L/hr. At the end of the time course, arabinose concentration in the medium was reduced by 32.8%, to 34 g/L. Adaptation greatly improved arabinose utilization, cell growth and ethanol production in ZW705-ara354A7. The maximum rate of arabinose consumption was 0.73 g/L/hr. At the end of time the course, arabinose concentration in the medium was reduced by 83.4%, to 8.4 g/L.

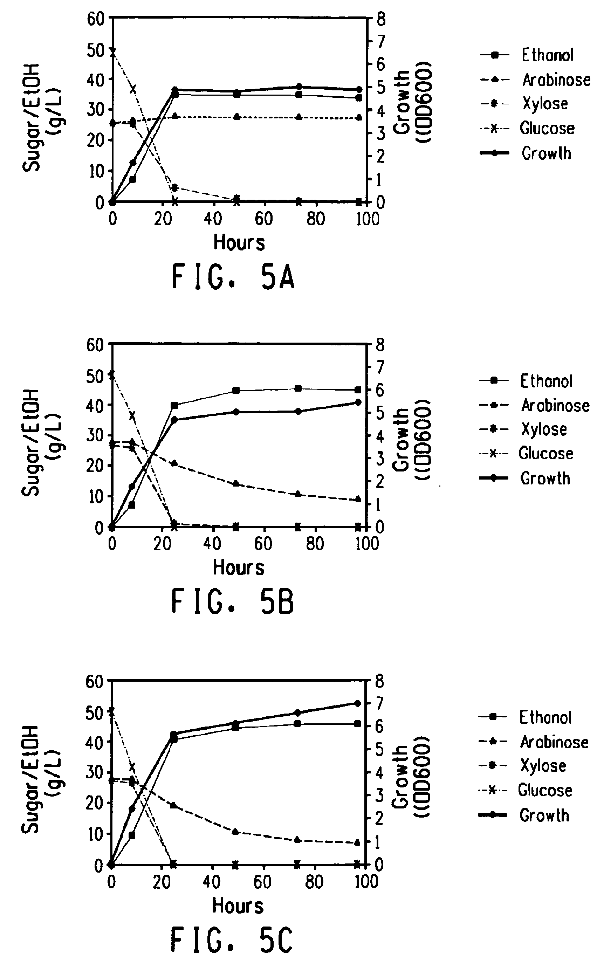

To characterize the strains in a medium containing mixed sugars, ZW705, ZW705-ara354, and ZW705-ara354A7 were grown and analyzed as described above, but the MRM3A5 media used in the previous experiment was replaced by MRM3A2.5X2.5G5 media(MRM3 with 25 g/L arabinose, 25 g/L xylose, and 50 g/L glucose). Due to fast growth in MRM3A2.5X2.5G5, a time point at 10 hour was added. Analysis was as described above for the experiment using arabinose medium..The results are given in Figure 5. These results show that ZW705 efficiently utilized glucose and xylose to support strong cell growth and ethanol production, but it could not metabolize arabinose (Figure 5A). After integration of P_{gap}-araBAD, ZW705-ara354 was able to utilize arabinose to enhance cell growth and ethanol production (Figure 5B). The maximum rate of arabinose consumption was 0.3 g/L/hr. At the end of the time course, arabinose concentration in the medium was reduced by 67.9%, to 8.8 g/L. In the adapted strain ZW705-ara354A7 there was some improvement over the ZW705-ara354 strain in arabinose utilization, which supported better growth and ethanol production. The maximum speed of arabinose consumption was 0.36 g/L/hr. At the end of the time course, arabinose concentration in the medium was reduced by 74.1%, to 7.1 g/L.

### Example 3

### Constructs for Expression of Two Arabinose Transport Systems from E. coli in Zvmomonas

Each of the two arabinose transport systems that are present in *E. coli*, encoded by araE or by araFGH, was expressed in Zymomonas and arabinose utilization analyzed. araE encodes an arabinose-proton symporter while araFGH encodes three proteins that form an ABC transporter.

### 1. Construction of chimeric araE gene and araFGH operon for expression in Zymomonas

*E. coli araE* and araFGH coding sequence DNA fragments were prepared by standard 30-cycle PCR, as described in Example 1, using *E. coli* MG1655 (a K12 strain: ATCC #700926) DNA as template. Each cycle included 45 sec denaturing at 94°C, 45 sec annealing at 60°C, and 4 min extension at 72°C. A forward primer ara135 (SEQ ID NO :54) and a reverse primer ara136 (SEQ ID NO :55) were used in PCR to synthesize a 1,550-bp araE fragment, including the araE coding sequence (1,419 bp) and its 3'UTR (121 bp), adding an Ncol site at the 5' end and an EcoRI site at the 3' end (SEQ ID NO :56). A forward primer ara137 (SEQ ID NO :57) and a reverse primer ara138 (SEQ ID NO :58) were used in PCR to synthesize a 3,744-bp araFGH fragment (SEQ ID NO :59). This fragment was identical to the *E*. *coli* araFGH operon but lacking the promoter. It included the araF coding sequence, araG coding sequence, araH coding sequence, araH 3'UTR, and intact intergenic regions. The primers added a 5' Ncol site and a 3' EcoRI site.

The *Actinoplanes missouriensis* GI promoter (P_{gi}) was chosen to direct the expression of araE and araFGH. It is the promoter of the xylose isomerase gene and has been demonstrated to function in *Z*. *mobilis* as a weak constitutive promoter. To clone *A. missouriensis* P_{gi}, a pair of oligonucleotide primers was designed. Primer ara12 (SEQ ID NO :60) was the forward primer for PCR of P_{gi} which added a SacI and an Spel site at the 5' end of the promoter. Primer ara13 (SEQ ID NO :61) was the reverse primer for PCR of P_{gi} which added an Ncol site at the 3' end of the promoter. These two primers were used in a standard PCR reaction and a plasmid containing the *Actinoplanes missouriensis* GI promoter (SEQ ID NO:62) was used as template DNA. The PCR reaction produced a 201-bp P_{gi} DNA fragment (SEQ ID NO:63) with the 5' Sacl and Spel sites and a 3' NcoI site that was cloned into TOPO Blunt Zero Vector (Invitrogen, Calsbad, CA) by following the manufacturer's instructions. The resulting plasmid pTP-P_{gi} was propagated in E. coli DH5a and plasmid DNA prepared using a Qiagen DNA Miniprep Kit.

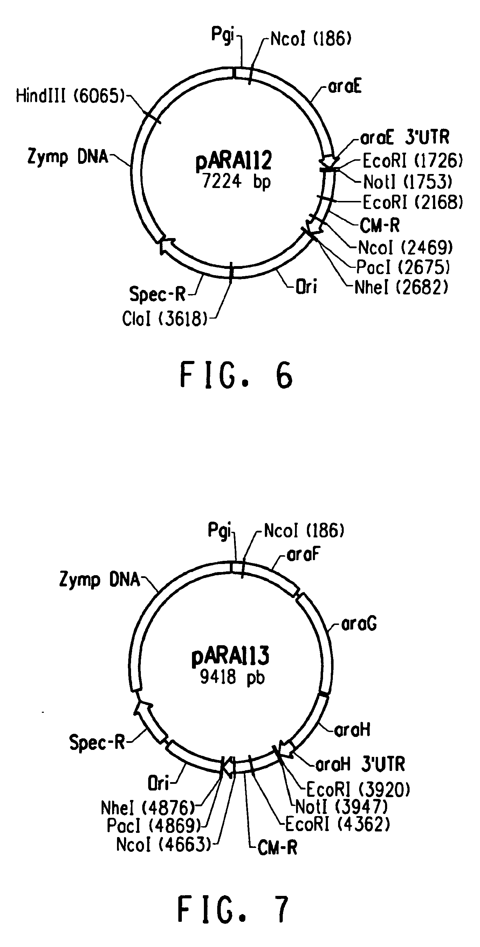

The Spel - Nool P_{gi} fragment from pTP-P_{gi} and the Ncol - EcoRI araE PCR fragment were combined in a pZB188/aada vector along with a chloramphenicol resistance marker (CM-R; SEQ ID NO:64) creating pARA112 (Figure 6; SEQ ID NO:65). pARA112 contains a P_{gi}-araE chimeric gene in the pZB188 derived *E. coli*/*Zymomonas* shuttle vector. The SpeI - Ncol P_{gi} fragment from pTP-P_{gi} and the NcoI - EcoRI araFGH PCR fragment were combined in a pZB188/aada vector along with a chloramphenicol resistance marker creating pARA113 (Figure 7; SEQ ID NO:66). The resulting shuttle vectors were propagated in *E*. *coli* DH5a and plasmid DNA was prepared using a Qiagen DNA Miniprep Kit. The P_{gi}-araE gene and P_{gi}-araFGH operon were confirmed by sequencing.

### Example 4

### Expression of E. coli Arabinose Transport Systems in Zymomonas ZW705-ara354A7

Effects of the two arabinose transport systems of E. coli on arabinose utilizing Zymomonas cells were tested by expressing the constructed P_{gi}-araE gene and P_{gi}-araFGH operon.

### 1. Transforming ZW705-ara354A with pARA112 and pARA113.

pARA112 containing the P_{gi}-araE gene and pARA113 containing the P_{gi}-araFGH operon, both prepared in Example 3, were transformed into cells of ZW705-ara354A7 (prepared in Examples 1 and 2). Competent cells of the ZW705-ara354A7 strain were prepared as described in Example 1. Since tranformation of *Z. mobilis* requires non-methylated DNA, pARA112 and pARA113 were each transformed into *E. coli* SCS110 competant cells and non-methylated plasmid DNA was prepared from a 10 mL-culture of a single colony using a Qiagen DNA Miniprep Kit. Approximately 500 ng of each plasmid DNA was separately mixed with 50 µL ZW705-ara354A7 competant cells in a 1 MM VWR Electroporation Cuvette and electroporated into the cells at 2.0 KV using a BT720 Transporater Plus.

The pARA112 or pARA113 transformed cells (ZW705-ara354A7-ara112 and ZW705-ara354A7-ara113) were recovered in 1 mL MMG5 medium for 4 hours at 30°C and then grown on MMG5-CM120 plates (MMG5 with 120 mg/L chloramphenicol and 15 g/L agar) for 2 days at 30°C inside an anaerobic jar with an AnaeroPack. Individual colonies were streaked onto a new MMG5-CM120 plate and allowed to grow under the same conditions as in the last step. The streaks grew well on the chloramphenicol-containing plates, indicating successful transformation. 2. Expressing P_{gi}-araE and P_{gi}-araFGH in the transformed strains.

Several streaks of the transformed strains were selected from the MMG5-CM120 plates to represent ZW705-ara354A7-ara112 and ZW705-ara354A7-ara113. Expression of P_{gi}-araE or P_{gi}-araFGH was inspected by the 72-hour growth assay described in Example 1. In this assay, cells from each streak were grown overnight in 2 mL MRM3G5-CM120 (MRM3G5 with 120 mg/L chloramphenicol) at 30°C with 150 rpm shaking. Cells were harvested, washed with MRM3A5, and resuspended in MRM3A5-CM120 (MRM3A5 containing 120 mg/L chloramphenicol) at a starting OD₆₀₀ of 0.1. Four mL of the suspension were grown for 72 hours at 30°C with 150 rpm shaking. At the end of growth, OD₆₀₀ was measured and metabolite profiles were analyzed by using a BioRad Aminex HPX-A7H ion exclusion column on an Agilent 1100 HPLC system as described in Example 1. As a control, ZW705-ara354A7 strain was grown and analyzed in parallel with Spec250 replacing CM120. Results for 3 strains in each transformation are given in Table 4.

**Table 4. 72-hour growth assay for ZW705-ara354A7-ara112 and ZW705-ara354A7-ara113 in MRM3A5.**

| Strain | Growth (OD600) | Ethanol (g/L) | Arabinose (g/L) |
|---|---|---|---|
| ZW705-ara354A7 | 3.01 | 18.57 | 5.98 |
| ZW705-ara354A7-ara112-1 | 3.28 | 19.22 | 0.43 |
| ZW705-ara354A7-ara112-2 | 3.33 | 21.38 | 0.34 |
| ZW705-ara354A7-ara112-3 | 3.20 | 19.65 | 0.40 |
| ZW705-ara354A7-ara113-5 | 2.51 | 16.64 | 11.95 |
| ZW705-ara354A7-ara113-6 | 2.12 | 15.65 | 15.97 |
| ZW705-ara354A7-ara113-7 | 2.17 | 15.32 | 13.91 |

Comparing to their parent, all ZW705-ara354A7-ara112 strains utilized more arabinose during 72 hours growth, which supported a higher level of growth and ethanol production. In fact, these ZW705-ara354A7-ara112 strains had consumed almost all available arabinose in the medium. This indicates that araE facilitated arabinose utilization in the engineered strains. On the other hand, expression of araFGH appeared to have a negative impact. It resulted in less arabinose utilization, a lower level of growth and lower ethanol production in ZW705-ara354A7-ara113 strains during 72 hour growth.

### 3. Characterizing growth and metabolite profiles of ZW705-ara354A7-ara112 strain.

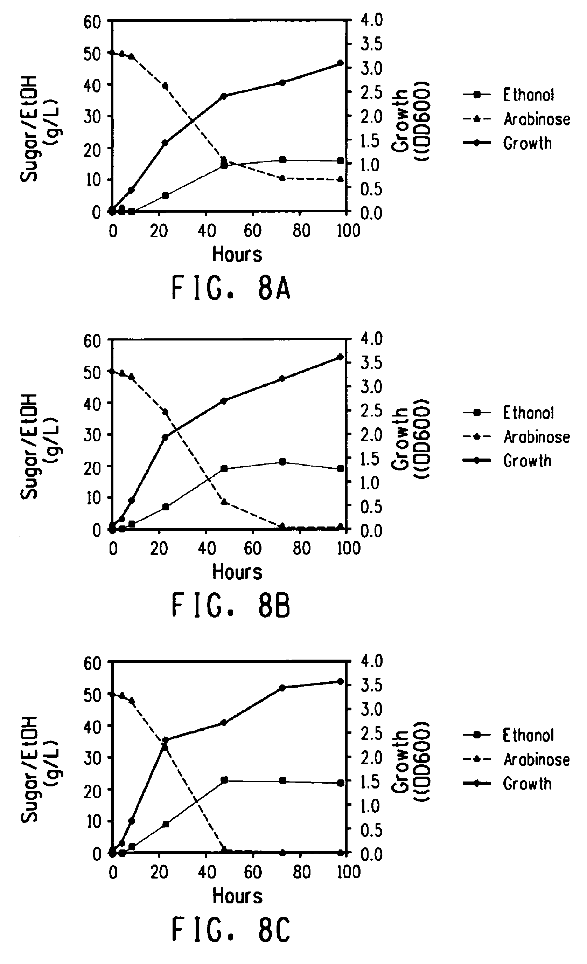

Since ZW705-ara354A7-ara112 strains showed facilitated arabinose metabolism, these strains were analyzed further. Characterization was preformed by following the procedure described in Example 2.3. Because araE was expressed from a shuttle vector, the expression level could vary between different strains. Therefore, two strains (ZW705-ara354A7-ara112-2 and ZW705-ara354A7-ara112-3) were examined side by side. To characterize strains in the single sugar (arabinose) medium, overnight grown ZW705-ara354A7-ara112-2 and ZW705-ara354A7-ara112-3 cultures were harvested, washed with MRM3A5, and resuspended in MRM3A5-CM120 to a starting OD₆₀₀ of 0.1. Twenty mL of the suspensions were grown at 30°C with 150 rpm shaking for a 96-hour time course. OD₆₀₀ was measured at 0, 6, 12, 24, 48, 72, and 96 hour. At each time point, metabolite profiles were analyzed by using a BioRad Aminex HPX-A7H ion exclusion column on an Agilent 1100 HPLC system. In parallel, the parent strain ZW705-ara354A7 was grown in 250 mg/L spectinomycin instead 120 mg/L chloramphenicol and analyzed as a control. The results are given in Figure 8. These results indicate that, without P_{gi}-araE, ZW705-ara354A7 utilized arabinose with a maximum speed of 0.93 g/L/hr. At the end of the time course, arabinose concentration in the medium was reduced by 80.4%, to 9.81 g/L. With expression of araE, ZW705-ara354A7-ara112-2 and ZW705-ara354A7-ara112-3 utilized arabinose more efficiently, which supported higher levels of growth and ethanol production. The maximum speeds of arabinose consumption increased to 1.18 g/Uhr and 1.28 g/Uhr in the 112-2 and 112-3 strains, respectively. At the end of the time course, arabinose concentration in the medium was reduced by 98%, to 1.02 g/L for ZW705-ara354A7-ara112-2 and by 99.2%, to 0.41 g/L for ZW705-ara354A7-ara112-3. In fact, ZW705-ara354A7-ara112-2 and ZW705-ara354A7-ara112-3 had almost exhausted all available arabinose after 72 hour and 48 hour culture, respectively.

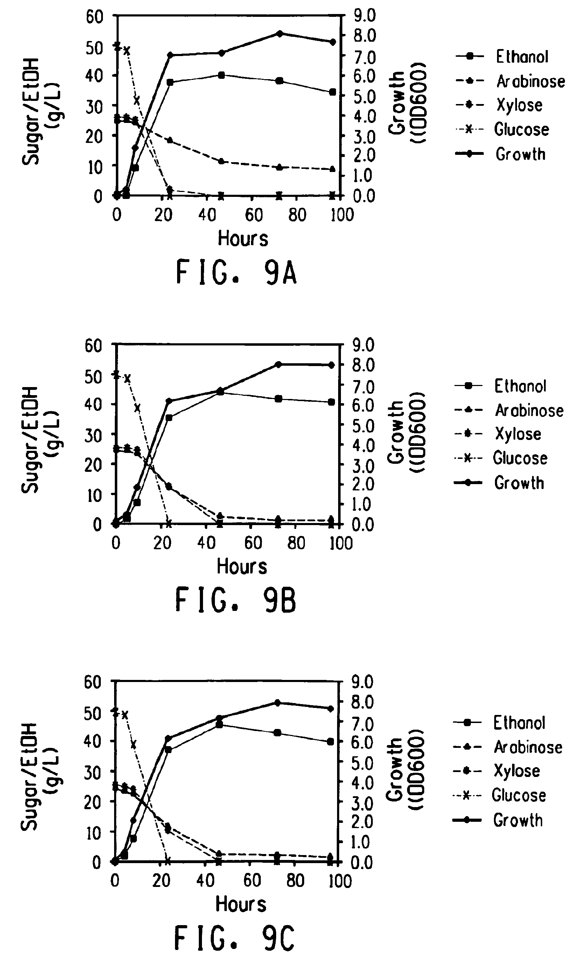

To characterize the strains in a medium containing mixed sugars, ZW705- ara354A7, ZW705-ara354A7-ara112-2, and ZW705-ara354A7-ara112-3 were grown and analyzed as described above but using MRM3A2.5X2.5G5 media. Results are given in Figure 9. These results show that ZW705-ara354A7 efficiently exhausted all glucose and xylose within 24 hours to support strong growth and ethanol production. Its arabinose metabolism was relatively slower and incomplete. The maximum speed of arabinose consumption was 0.43 g/L/hr. At the end of time the course, arabinose concentration in the medium was reduced by 62.4%, to 9 g/L. However, ZW705-ara354A7-ara112-2 and ZW705-ara354A7-ara112-3 utilized arabinose much more efficiently. The maximum speeds of arabinose consumption increased to 0.73 g/L/hr and 0.78 g/L/hr, respectively. At the end of the time course, arabinose concentration in the medium was reduced by 90.3%, to 2.33 g/L for ZW705-ara354A7-ara112-2 and by 90.1 %, to 2.38 g/L for ZW705-ara354A7-ara112-3. It had actually been reduced to near this level within 48 hours in both strains. Therefore, expression of araE had also facilitated arabinose utilization in the mixed sugar medium, which contributed to ethanol production as shown in Figure 9. The expression had no significant effect on glucose metabolism, but it slowed down xylose metabolism so that both ZW705-ara354A7-ara112 strains took 48 hours to exhaust all xylose in the medium while the ZW705-ara354A7 strain took only 24 hours.

### Example 5

### Expression of araE in Zymomonas ZW705-ara354 and ZW801-ara354

In this example, effects of araE expression in non-adapted arabinose utilizing Z. mobilis strains ZW705-ara354 and ZW801-ara354 are analyzed.

### 1. Transforming ZW705-ara354 and ZW801-ara354 with pARA112.

As described in Example 2, ZW705-ara354 and ZW801-ara354 are engineered Z. mobilis strains developed from ZW705 and ZW801-4 by introducing P_{gap}-araBAD into the IdhA locus. ZW705-ara354 is the parental strain of ZW705-ara354A7 that was not adaptated in MRM3A5. Competent cells of both strains were prepared. Non-methylated DNA of pARA112 was electroporated into the competent cells as described in the previous examples.

The pARA112-transformed ZW705-ara354 (ZW705-ara354-ara112) and ZW801-ara354 ((ZW801-ara354-ara112) were recovered in 1 mL MMG5 medium for 4 hours at 30°C and then grown on MMG5-CM120 plates for 2 days at 30°C inside an anaerobic jar with an AnaeroPack. Individual colonies were streaked onto a new MMG5-CM120 plate and grown under the same conditions as in the last step. The streaks grew well on the chloramphenicol-containing plates, indicating successful transformation.

### 2. Expressing P_{gi}-araE in the transformed strains.

Several streaks of the transformed strains were selected from the MMG5-CM120 plates to represent ZW705-ara354-ara112 and ZW801-ara354-ara112, respectively. Expression of P_{gi}-araE was inspected by the 72-hour growth assay in MRM3A5. The details of assay were the same as in previous examples. As controls, ZW705-ara354 and ZW801-ara354 strains were grown and analyzed in parallel with 250 mg/L spectrinomycin replacing 120 mg/L chloramphenicol in the growth medium. The results for 3 strains from each transformation are given in Table 5. Compared to their parental strains, all ZW705-ara354-ara112 and ZW801-ara354-ara112 strains utilized significantly more arabinose during 72 hours growth, which supported a higher level of growth and ethanol production. Therefore, araE also facilitated arabinose utilization in the both ZW705-ara354-ara112 and ZW801-ara354-ara112 strains.

**Table 5. 72-hour growth assay for ZW705-ara354-ara112 and ZW801-ara354-ara112 in MRM3A5**

| Strain | Growth (OD600) | Ethanol (g/L) | Arabinose (g/L) |
|---|---|---|---|
| ZW705-ara354 | 1.15 | 9.56 | 27.88 |
| ZW705-ara354-ara112-1 | 1.56 | 14.18 | 17.24 |
| ZW705-ara354-ara112-2 | 1.67 | 16.71 | 10.93 |
| ZW705-ara354-ara112-3 | 1.47 | 13.76 | 19.06 |
| ZW801-ara354 | 1.39 | 9.65 | 27.08 |
| ZW801-ara354-ara112-4 | 1.95 | 15.01 | 15.12 |
| ZW801-ara354-ara112-5 | 2.07 | 15.51 | 12.94 |
| ZW801-ara354-ara112-5 | 2.29 | 15.79 | 13.05 |

### 3. Characterizing growth and metabolite profiles of ZW705-ara354-ara112 and ZW801-ara354-ara112 strains.

ZW705-ara354-ara112 and ZW801-ara354-ara112 strains were further characterized for their growth and metabolite profiles during a 96-hour time course. Characterization was performed by following the same procedure described in Example 4.3. ZW705-ara354-ara112-1 and ZW705-ara354-ara112-2 were examined and compared to their parent ZW705-ara354, while ZW801-ara354-ara112-5 and ZW801-ara354-ara112-6 were examined and compared to their parent ZW801-ara354. Measurement and analysis were done at 0, 6, 12, 24, 48, 72, and 96 hour time points.

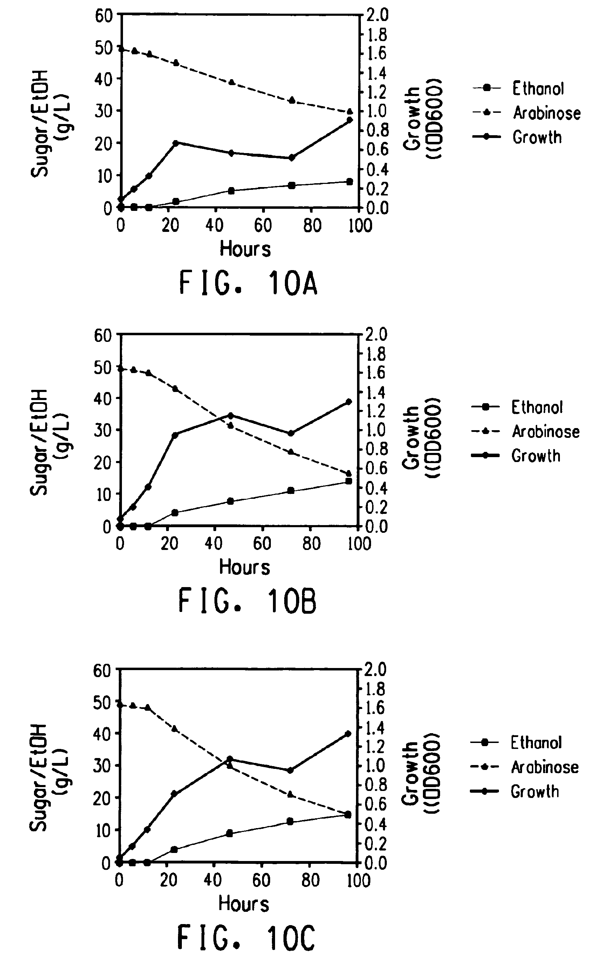

Figure 10 shows the results obtained from ZW705-ara354 and ZW705-ara354-ara112 strains grown in MRM3A5. The results show that, without P_{gi}-araE, ZW705-ara354 utilized arabinose poorly, with a maximum rate of 0.25 g/L/hr. At the end of the time course, arabinose concentration in the medium was reduced by only 38.19%, to 30.22 g/L. With expression of araE, ZW705-ara354-ara112-1 and ZW705-ara354-ara112-2 utilized arabinose more efficiently, which supported higher levels of growth and ethanol production. The maximum rate of arabinose consumption increased to 0.46 g/L/hr and 0.48 g/L/hr, respectively. At the end of the time course, arabinose concentration in the medium was reduced by 65.8%, to 16.73 g/L for ZW705-ara354-ara112-1 and by 69.61 %, to 14.86 g/L for ZW705-ara354-ara112-2.

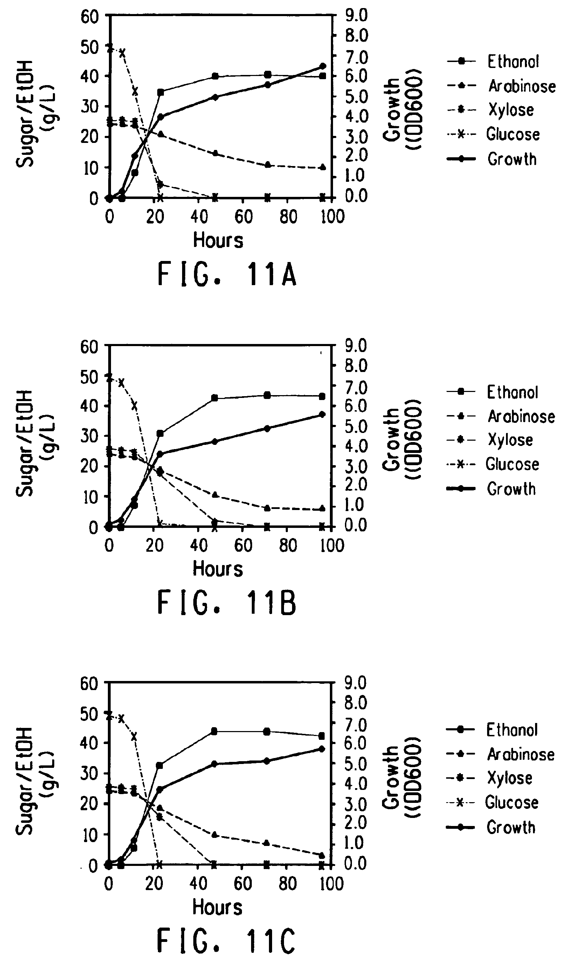

Figure 11 shows the results obtained from ZW705-ara354 and ZW705-ara354-ara112 strains grown in the mixed sugars medium MRM3A2.5X2.5G5. The results show that ZW705-ara354 efficiently used glucose and xylose to support strong growth and ethanol production. Its arabinose metabolism was slow and incomplete. The maximum rate of arabinose consumption was 0.29 g/L/hr. At the end of the time course, arabinose concentration in the medium was reduced by 57.32%, to 10.21 g/L. However, ZW705-ara354-ara112-1 and ZW705-ara354-ara112-2 utilized arabinose more efficiently. The maximum rate of arabinose consumption increased to 0.32 g/L/hr and 0.35 g/L/hr, respectively. At the end of the time course, arabinose concentration in the medium was reduced by 86.33%, to 3.27 g/L for ZW705-ara354-ara112-1 and by 85.2%, to 3.54 g/L for ZW705-ara354-ara112-2. These results demonstrated that expression of araE facilitated arabinose utilization in ZW705-ara354-ara112 strains in both single sugar medium (arabinose) and mixed sugar medium. Therefore, the araE effect did not require a genetic background acquired during the adaptation of ZW705-ara354A7. Similar to results in ZW705-ara354A7-ara112, the expression of araE slightly slowed down xylose metabolism in ZW705-ara354-ara112 grown in the mixed sugar medium.

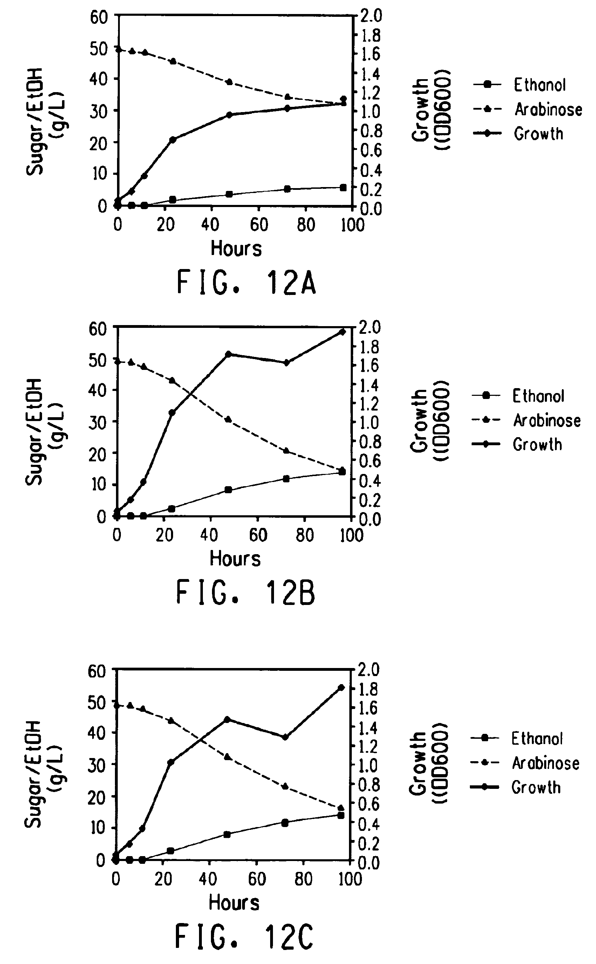

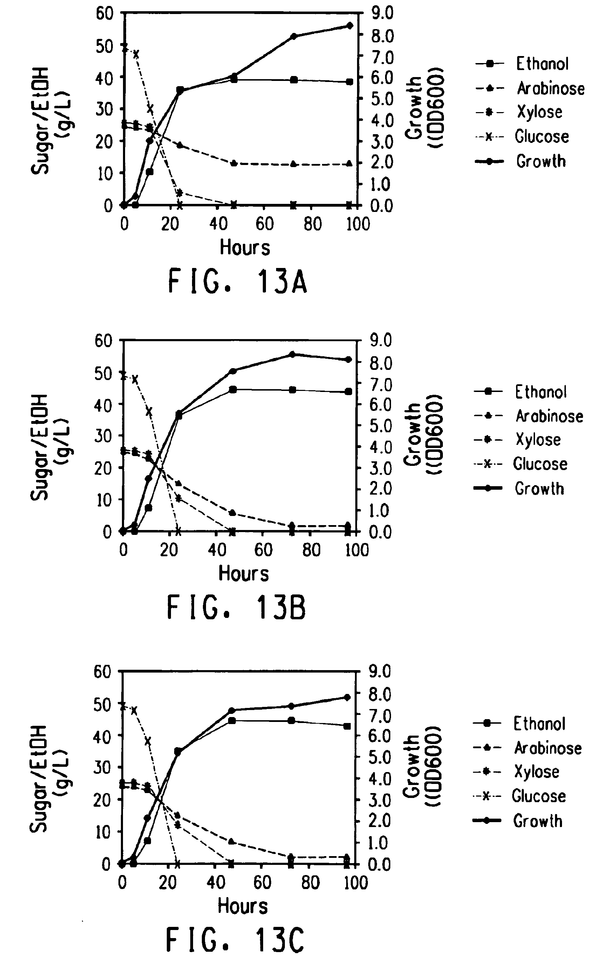

Figure 12 shows the results obtained from ZW801-ara354 and ZW801-ara354-ara112 strains growing in MRM3A5. The results indicate that, without P_{gi}-araE, ZW801-ara354 utilized arabinose poorly, with a maximum rate of 0.25 g/L/hr. At the end of the time course, arabinose concentration in the medium was reduced by only 32.99%, to 32.76 g/L. With expression of araE, ZW801-ara354-ara112-5 and ZW801-ara354-ara112-6 utilized arabinose more efficiently, which supported higher levels of growth and ethanol production. The maximum rate of arabinose consumption increased to 0.49 g/Uhr and 0.47 g/L/hr, respectively. At the end of the time course, arabinose concentration in the medium was reduced by 69.52%, to 14.90 g/L for ZW801-ara354-ara112-5 and by 65.92%, to 16.66 g/L for ZW801-ara354-ara112-6. Figure 13 shows the results obtained from ZW801-ara354 and ZW801-ara354-ara112 strains grown in mixed sugar medium MRM3A2.5X2.5G5. It shows that ZW801-ara354 efficiently used glucose and xylose to support strong growth and ethanol production. Its arabinose metabolism was slow and incomplete. The maximum rate of arabinose consumption was 0.22 g/Uhr. At the end of the time course, arabinose concentration in the medium was reduced by 45.48%, to 13.04 g/L. However, ZW801-ara354-ara112-5 and ZW801-ara354-ara112-6 utilized arabinose more efficiently. The maximum rate of arabinose consumption increased to 0.35 g/L/hr and 0.36 g/L/hr, respectively. At the end of the time course, arabinose concentration in the medium was reduced by 89.92%, to 2.41 g/L for ZW801-ara354-ara112-5 and by 88.38%, to 2.78 g/L for ZW801-ara354-ara112-6. These results further demonstrated that expression of araE facilitated arabinose utilization in ZW801-ara354-ara112 strains in both single sugar medium and mixed sugar medium. Therefore, the araE effect was not limited to ZW705-ara354 and the derived strains. Similar to that in ZW705-ara354A7-ara112 and ZW705-ara354-ara112, the expression of araE slightly slowed down xylose metabolism in ZW801-ara354-ara112 grown in the mixed sugar medium.

## Claims

1. A recombinant microorganism of the genus *Zymomonas* or *Zymobacter* that utilizes arabinose to produce ethanol, said microorganism comprising at least one heterologous gene encoding an arabinose-proton symporter, wherein said symporter is expressed by said microorganism.

2. The recombinant microorganism of claim 1 wherein the arabinose-proton symporter is encoded by the coding region of an araE gene.

3. The recombinant microorganism of claim 1 wherein arabinose utilization is improved by at least about 10% as compared to a parental microorganism wherein said parental microorganism is lacking the at least one heterologous gene encoding an arabinose-proton symporter.

4. The recombinant microorganism of claim 1 wherein the strain additionally utilizes xylose to produce ethanol.

5. A process for generating a recombinant microorganism of the genus *Zymomonas* or *Zymobacter* that has increased arabinose utilization comprising:
a) providing a recombinant *Zymomonas* or *Zymobacter* strain that utilizes arabinose to produce ethanol under suitable conditions; and
b) introducing at least one heterologous gene encoding an arabinose-proton symporter to the strain of (a) for expression of said symporter.

6. The process according to claim 5, further comprising adapting the strain either before or after step (b), or both before and after step (b), by serial growth in media containing arabinose as the sole carbon source whereby an adapted strain is produced and wherein said strain has further improved arabinose utilization as compared to the strain with no adaptation.

7. The process according to claim 6, wherein the adapted strain additionally utilizes xylose and glucose for ethanol production in mixed sugars media comprising arabinose, xylose, and glucose.

8. A process for producing ethanol comprising:
a) providing a recombinant *Zymomonas* or *Zymobacter* strain that utilizes arabinose to produce ethanol, said strain comprising at least one heterologous gene encoding an arabinose-proton symporter, wherein said symporter is expressed by said strain; and
b) culturing the strain of (a) in a medium comprising arabinose whereby arabinose is converted to ethanol.

9. The process according to claim 8 wherein the arabinose-proton symporter is encoded by the coding region of an araE gene.

10. The process according to claim 8 wherein arabinose utilization is improved by at least about 10% as compared to a parental microorganism wherein said parental microorganism lacks a heterologous gene encoding an arabinose-proton symporter.

11. The process according to claim 8 wherein the strain of (a) is further capable of utilizing xylose and glucose to produce ethanol.

12. The process according to claim 8 wherein the strain of (a) has been adapted by serial growth in media containing arabinose as the sole carbon source whereby an arabinose-adapted strain is produced wherein said arabinose-adapted strain has increased ethanol production as compared to the strain of (a) that has not been adapted.

13. The process according to claim 8 wherein conversion of arabinose to ethanol is increased relative to conversion of arabinose to ethanol by a recombinant parental strain without at least one heterologous gene encoding an arabinose-proton symporter.

14. The process of claim 8 wherein the medium comprises either a mixture of sugars comprising arabinose or arabinose as a sole sugar.

15. A method for improving arabinose utilization by an arabinose-utilizing microorganism comprising:
(a) providing an arabinose-utlizing microorganism wherein said microorganism is selected from the group consisting of a recombinant *Zymomonas* or *Zymobacter* strain that utilizes arabinose to produce ethanol;
(b) introducing into the genome of said microorganism at least one heterologous gene encoding an arabinose-proton symporter wherein said symporter is expressed by said microorganism; and
(c) contacting the microorganism of (b) with a medium comprising arabinose, wherein said microorganism metabolizes said arabinose at an increased rate as compared to said microorganism that is lacking the arabinose-proton symporter.

## Patentansprüche

1. Rekombinanter Mikroorganismus des Genus *Zymomonas* oder *Zymobacter*, der Arabinose verwertet, um Ethanol zu produzieren, wobei der Mikroorganismus zumindest ein heterologes Gen umfasst, das für einen Arabinose-Protonen-Symporter codiert, wobei der Symporter durch den Mikroorganismus exprimiert wird.

2. Rekombinanter Mikroorganismus nach Anspruch 1, wobei der Arabinose-Protonen-Symporter durch die codierende Region eines araE Gens codiert ist.

3. Rekombinanter Mikroorganismus nach Anspruch 1, wobei die Arabinoseverwertung um zumindest etwa 10% im Vergleich zu einem parentalen Mikroorganismus verbessert ist, wobei dem parentalen Mikroorganismus das zumindest eine heterologe Gen fehlt, das für einen Arabinose-Protonen-Symporter codiert.

4. Rekombinanter Mikroorganismus nach Anspruch 1, wobei der Stamm zusätzlich Xylose verwertet, um Ethanol zu produzieren.

5. Verfahren zur Erzeugung eines rekombinanten Mikroorganismus des Genus *Zymomonas* oder *Zymobacter*, der gesteigerte Arabinoseverwertung aufweist, umfassend:
a) Bereitstellen eines rekombinanten *Zymomonas*- oder *Zymobacter-Stamms,* der Arabinose verwertet, um Ethanol zu produzieren, unter geeigneten Bedingungen; und
b) Einbringen zumindest eines heterologen Gens, das für einen Arabinose-Protonen-Symporter codiert, in den Stamm aus (a) zwecks Expression des Symporters.

6. Verfahren nach Anspruch 5, weiterhin umfassend Anpassen des Stamms entweder vor oder nach Schritt (b), oder sowohl vor als auch nach Schritt (b), durch serielle Züchtung in Medien, die Arabinose als die alleinige Kohlenstoffquelle enthalten, wodurch ein angepasster Stamm produziert wird und wobei der Stamm eine weiter verbesserte Arabinoseverwertung im Vergleich zum Stamm ohne Anpassung aufweist.

7. Verfahren nach Anspruch 6, wobei der angepasste Stamm zusätzlich Xylose und Glukose zur Ethanolproduktion in Arabinose, Xylose und Glukose umfassenden Zuckergemischmedien verwertet.

8. Verfahren zur Produktion von Ethanol, umfassend:
a) Bereitstellen eines rekombinanten *Zymomonas-* oder *Zymobacter-Stamms,* der Arabinose verwertet, um Ethanol zu produzieren, wobei der Stamm zumindest ein heterologes Gen umfasst, das für einen Arabinose-Protonen-Symporter codiert, wobei der Symporter durch den Stamm exprimiert wird; und
b) Kultivieren des Stamms aus (a) in einem Arabinose umfassenden Medium, wodurch Arabinose zu Ethanol umgewandelt wird.

9. Verfahren nach Anspruch 8, wobei der Arabinose-Protonen-Symporter durch die codierende Region eines araE Gens codiert ist.

10. Verfahren nach Anspruch 8, wobei die Arabinoseverwertung um zumindest etwa 10% im Vergleich zu einem parentalen Mikroorganismus verbessert wird, wobei dem parentalen Mikroorganismus ein heterologes Gen fehlt, das für einen Arabinose-Protonen-Symporter codiert.

11. Verfahren nach Anspruch 8, wobei der Stamm aus (a) weiterhin in der Lage ist, Xylose und Glukose zu verwerten, um Ethanol zu produzieren.

12. Verfahren nach Anspruch 8, wobei der Stamm aus (a) durch serielle Züchtung in Medien, die Arabinose als die alleinige Kohlenstoffquelle enthalten, angepasst worden ist, wodurch ein Arabinose-angepasster Stamm produziert wird, wobei der Arabinose-angepasste Stamm eine gesteigerte Ethanolproduktion im Vergleich zu dem Stamm aus (a) aufweist, der nicht angepasst worden ist.

13. Verfahren nach Anspruch 8, wobei Umwandlung von Arabinose zu Ethanol gesteigert wird in Bezug auf Umwandlung von Arabinose zu Ethanol durch einen rekombinanten parentalen Stamm ohne zumindest ein heterologes Gen, das für einen Arabinose-Protonen-Symporter codiert.

14. Verfahren nach Anspruch 8, wobei das Medium entweder ein Gemisch von Zuckern, umfassend Arabinose, oder Arabinose als alleinigen Zucker umfasst.

15. Verfahren zur Verbesserung der Arabinoseverwertung durch einen Arabinose verwertenden Mikroorganismus, umfassend:
(a) Bereitstellen eines Arabinose verwertenden Mikroorganismus, wobei der Mikroorganismus aus der Gruppe ausgewählt ist, die aus einem rekombinanten *Zymomonas*- oder *Zymobacter*-Stamm besteht, der Arabinose verwertet, um Ethanol zu produzieren;
(b) Einbringen, in das Genom des Mikroorganismus, zumindest eines heterologen Gens, das für einen Arabinose-Protonen-Symporter codiert, wobei der Symporter durch den Mikroorganismus exprimiert wird; und
(c) In-Kontakt-Bringen des Mikroorganismus aus (b) mit einem Arabinose umfassenden Medium, wobei der Mikroorganismus die Arabinose mit einer gesteigerten Rate verstoffwechselt im Vergleich zu dem Mikroorganismus, dem der Arabinose-Protonen-Symporter fehlt.

## Revendications

1. Microorganisme recombinant du genre *Zymomonas* ou *Zymobacter* qui utilise l'arabinose pour produire de l'éthanol, ledit microorganisme comprenant au moins un gène hétérologue codant pour un symporteur d'arabinose-proton, dans lequel ledit symporteur est exprimé par ledit microorganisme.

2. Microorganisme recombinant selon la revendication 1, dans lequel le symporteur d'arabinose-proton est codé par la région de codage d'un gène araE.

3. Microorganisme recombinant selon la revendication 1, dans lequel l'utilisation d'arabinose est améliorée d'au moins environ 10% comparé à un microorganisme parental où ledit microorganisme parental est dénué du au moins un gène hétérologue codant pour un symporteur d'arabinose-proton.

4. Microorganisme recombinant selon la revendication 1, dans lequel la souche utilise en outre le xylose pour produire de l'éthanol.

5. Procédé pour la génération d'un microorganisme recombinant du genre *Zymomonas* ou *Zymobacter* qui a une utilisation d'arabinose améliorée comprenant:
a) la fourniture d'une souche de *Zymomonas* ou de *Zymobacter* recombinante qui utilise l'arabinose pour produire de l'éthanol dans des conditions appropriées; et
b) l'introduction d'au moins un gène hétérologue codant pour un symporteur d'arabinose-proton dans la souche de (a) pour une expression dudit symporteur.

6. Procédé selon la revendication 5, comprenant en outre l'adaptation de la souche soit avant, soit après l'étape (b), ou à la fois avant et après l'étape (b), par une croissance en série dans un milieu contenant de l'arabinose en tant que seule source de carbone en conséquence de quoi une souche adaptée est produite et dans lequel ladite souche a en outre une utilisation d'arabinose améliorée comparé à la souche sans adaptation.

7. Procédé selon la revendication 6, dans lequel la souche adaptée utilise en outre le xylose et le glucose pour une production d'éthanol dans un milieu de sucres mélangés comprenant de l'arabinose, du xylose et du glucose.

8. Procédé pour la production d'éthanol comprenant:
a) la fourniture d'une souche de *Zymomonas* ou de *Zymobacter* recombinante qui utilise l'arabinose pour produire de l'éthanol, ladite souche comprenant au moins un gène hétérologue codant pour un symporteur d'arabinose-proton, dans lequel ledit symporteur est exprimé par ladite souche; et
b) la culture de la souche de (a) dans un milieu comprenant de l'arabinose en conséquence de quoi l'arabinose est converti en éthanol.

9. Procédé selon la revendication 8, dans lequel le symporteur d'arabinose-proton est codé par la région de codage d'un gène araE.

10. Procédé selon la revendication 8, dans lequel l'utilisation d'arabinose est améliorée d'au moins environ 10% comparé à un microorganisme parental où ledit microorganisme parental est dénué du gène hétérologue codant pour un symporteur d'arabinose-proton.

11. Procédé selon la revendication 8, dans lequel la souche de (a) est capable en outre d'utiliser le xylose et le glucose pour produire de l'éthanol.

12. Procédé selon la revendication 8, dans lequel la souche de (a) a été adaptée par une croissance en série dans un milieu contenant de l'arabinose en tant que seule source de carbone en conséquence de quoi une souche adaptée pour l'arabinose est produite où ladite souche adaptée pour l'arabinose a une production d'éthanol augmentée comparé à la souche de (a) qui n'a pas été adaptée.

13. Procédé selon la revendication 8, dans lequel la conversion d'arabinose en éthanol est augmentée relativement à la conversion d'arabinose en éthanol par une souche parentale recombinante sans au moins un gène hétérologue codant pour un symporteur d'arabinose-proton.

14. Procédé selon la revendication 8, dans lequel le milieu comprend soit un mélange de sucres comprenant de l'arabinose, soit de l'arabinose en tant que seul sucre.

15. Méthode pour l'amélioration de l'utilisation d'arabinose par un microorganisme utilisant l'arabinose comprenant:
(a) la fourniture d'un microorganisme utilisant l'arabinose, dans laquelle ledit microorganisme est choisi dans le groupe constitué d'une souche de *Zymomonas* ou de *Zymobacter* recombinante qui utilise l'arabinose pour produire de l'éthanol;
(b) l'introduction dans le génome dudit microorganisme d'au moins un gène hétérologue codant pour un symporteur d'arabinose-proton, dans laquelle ledit symporteur est exprimé par ledit microorganisme; et
(c) la mise en contact du microorganisme de (b) avec un milieu comprenant de l'arabinose, dans laquelle ledit microorganisme métabolise ledit arabinose à un taux accru comparé audit microorganisme qui est dénué du symporteur d'arabinose-proton.


### SEQUENCE LISTING

<110> E. I. du Pont de Nemours and Company
<120> Zymomonas wtih improved arabinose utilization
<130> CL4545PCT
<160> 66
<170> PatentIn version 3.4
<210> 1
   <211> 1416
   <212> DNA
   <213> Escherichia coli
<400> 1
   atggttacta tcaatacgga atctgcttta acgccacgtt ctttgcggga tacgcggcgt      60
   atgaatatgt ttgtttcggt agctgctgcg gtcgcaggat tgttatttgg tcttgatatc     120
   ggcgtaatcg ccggagcgtt gccgttcatt accgatcact ttgtgctgac cagtcgtttg     180
   caggaatggg tggttagtag catgatgctc ggtgcagcaa ttggtgcgct gtttaatggt     240
   tggctgtcgt tccgcctggg gcgtaaatac agcctgatgg cgggggccat cctgtttgta     300
   ctcggttcta tagggtccgc ttttgcgacc agcgtagaga tgttaatcgc cgctcgtgtg     360
   gtgctgggca ttgctgtcgg gatcgcgtct tacaccgctc ctctgtatct ttctgaaatg     420
   gcaagtgaaa acgttcgcgg taagatgatc agtatgtacc agttgatggt cacactcggc     480
   atcgtgctgg cgtttttatc cgatacagcg ttcagttata gcggtaactg gcgcgcaatg     540
   ttgggggttc ttgctttacc agcagttctg ctgattattc tggtagtctt cctgccaaat     600
   agcccgcgct ggctggcgga aaaggggcgt catattgagg cggaagaagt attgcgtatg     660
   ctgcgcgata cgtcggaaaa agcgcgagaa gaactcaacg aaattcgtga aagcctgaag     720
   ttaaaacagg gcggttgggc actgtttaag atcaaccgta acgtccgtcg tgctgtgttt     780
   ctcggtatgt tgttgcaggc gatgcagcag tttaccggta tgaacatcat catgtactac     840
   gcgccgcgta tcttcaaaat ggcgggcttt acgaccacag aacaacagat gattgcgact     900
   ctggtcgtag ggctgacctt tatgttcgcc acctttattg cggtgtttac ggtagataaa     960
   gcagggcgta aaccggctct gaaaattggt ttcagcgtga tggcgttagg cactctggtg    1020
   ctgggctatt gcctgatgca gtttgataac ggtacggctt ccagtggctt gtcctggctc    1080
   tctgttggca tgacgatgat gtgtattgcc ggttatgcga tgagcgccgc gccagtggtg    1140
   tggatcctgt gctctgaaat tcagccgctg aaatgccgcg atttcggtat tacctgttcg    1200
   accaccacga actgggtgtc gaatatgatt atcggcgcga ccttcctgac actgcttgat    1260
   agcattggcg ctgccggtac gttctggctc tacactgcgc tgaacattgc gtttgtgggc    1320
   attactttct ggctcattcc ggaaaccaaa aatgtcacgc tggaacatat cgaacgcaaa    1380
   ctgatggcag gcgagaagtt gagaaatatc ggcgtc                              1416
<210> 2
   <211> 472
   <212> PRT
   <213> Escherichia coli
<400> 2
   Met Val Thr Ile Asn Thr Glu Ser Ala Leu Thr Pro Arg Ser Leu Arg
   1               5                   10                  15
   Asp Thr Arg Arg Met Asn Met Phe Val Ser Val Ala Ala Ala Val Ala
               20                  25                  30
   Gly Leu Leu Phe Gly Leu Asp Ile Gly Val Ile Ala Gly Ala Leu Pro
           35                  40                  45
   Phe Ile Thr Asp His Phe Val Leu Thr Ser Arg Leu Gln Glu Trp Val
       50                  55                  60
   Val Ser Ser Met Met Leu Gly Ala Ala Ile Gly Ala Leu Phe Asn Gly
   65                  70                  75                  80
   Trp Leu Ser Phe Arg Leu Gly Arg Lys Tyr Ser Leu Met Ala Gly Ala
                   85                  90                  95
   Ile Leu Phe Val Leu Gly Ser Ile Gly Ser Ala Phe Ala Thr Ser Val
               100                 105                 110
   Glu Met Leu Ile Ala Ala Arg Val Val Leu Gly Ile Ala Val Gly Ile
           115                 120                 125
   Ala Ser Tyr Thr Ala Pro Leu Tyr Leu Ser Glu Met Ala Ser Glu Asn
       130                 135                 140
   Val Arg Gly Lys Met Ile Ser Met Tyr Gln Leu Met Val Thr Leu Gly
   145                 150                 155                 160
   Ile Val Leu Ala Phe Leu Ser Asp Thr Ala Phe Ser Tyr Ser Gly Asn
                   165                 170                 175
   Trp Arg Ala Met Leu Gly Val Leu Ala Leu Pro Ala Val Leu Leu Ile
               180                 185                 190
   Ile Leu Val Val Phe Leu Pro Asn Ser Pro Arg Trp Leu Ala Glu Lys
           195                 200                 205
   Gly Arg His Ile Glu Ala Glu Glu Val Leu Arg Met Leu Arg Asp Thr
       210                 215                 220
   Ser Glu Lys Ala Arg Glu Glu Leu Asn Glu Ile Arg Glu Ser Leu Lys
   225 230 235 240
   Leu Lys Gln Gly Gly Trp Ala Leu Phe Lys Ile Asn Arg Asn Val Arg
   245 250 255
   Arg Ala Val Phe Leu Gly Met Leu Leu Gln Ala Met Gln Gln Phe Thr
   260 265 270
   Gly Met Asn Ile Ile Met Tyr Tyr Ala Pro Arg Ile Phe Lys Met Ala
   275 280 285
   Gly Phe Thr Thr Thr Glu Gln Gln Met Ile Ala Thr Leu Val Val Gly
   290 295 300
   Leu Thr Phe Met Phe Ala Thr Phe Ile Ala Val Phe Thr Val Asp Lys
   305 310 315 320
   Ala Gly Arg Lys Pro Ala Leu Lys Ile Gly Phe Ser Val Met Ala Leu
   325 330 335
   Gly Thr Leu Val Leu Gly Tyr Cys Leu Met Gln Phe Asp Asn Gly Thr
   340 345 350
   Ala Ser Ser Gly Leu Ser Trp Leu Ser Val Gly Met Thr Met Met Cys
   355 360 365
   Ile Ala Gly Tyr Ala Met Ser Ala Ala Pro Val Val Trp Ile Leu Cys
   370 375 380
   Ser Glu Ile Gln Pro Leu Lys Cys Arg Asp Phe Gly Ile Thr Cys Ser
   385 390 395 400
   Thr Thr Thr Asn Trp Val Ser Asn Met Ile Ile Gly Ala Thr Phe Leu
   405 410 415
   Thr Leu Leu Asp Ser Ile Gly Ala Ala Gly Thr Phe Trp Leu Tyr Thr
   420 425 430
   Ala Leu Asn Ile Ala Phe Val Gly Ile Thr Phe Trp Leu Ile Pro Glu
   435 440 445
   Thr Lys Asn Val Thr Leu Glu His Ile Glu Arg Lys Leu Met Ala Gly
   450 455 460
   Glu Lys Leu Arg Asn Ile Gly Val
   465 470
<210> 3
   <211> 1416
   <212> **DNA**
   <213> Shigella flexneri
<400> 3
   atggttacta tcaatacgga atctgcttta acgccacgtt ctttgcgtga tacgcggcgt 60
   atgaatatgt ttgtttcggt agctgctgcg gtcgcaggat tgttatttgg tcttgatatc 120
   ggcgtaatcg ccggagcgtt gccgttcatt accgatcact ttgtgctgac cagtcgtttg 180
   caggaatggg tggttagtag catgatgctc ggcgcagcaa ttggtgcgct gtttaatggt 240
   tggctgtcgt tccgcctggg gcgtaaatac agcctgatgg cgggggccat cctgtttgta 300
   ctcggttcta tagggtccgc ttttgcgacc agcgtagaga tgttaatcgc cgctcgtgtg 360
   gtgctgggca ttgctgtcgg gatcgcgtct tacaccgctc ctctgtatct ttctgaaatg 420
   gcaagtgaaa acgttcgcgg taagatgatc agtatgtacc agttgatggt cacactcggc 480
   atcgtgctgg cgtttttatc cgatacagcg ttcagttata gcggtaactg gcgcgcaatg 540
   ttgggggttc ttgctttacc agcagttctg ctgattattc tggtggtctt cctgccaaat 600
   agcccgcgct ggctggcgga aaaggggcgt catattgagg cggaagaagt gttgcgtatg 660
   ctgcgcgata cgtcggaaaa agcgcgagaa gaactcaacg aaattcgtga aagcctgaag 720
   ttaaaacagg gcggttgggc actgtttaag atcaaccgta acgtccgtcg tgctgtgttt 780
   ctcggtatgt tgttgcaggc gatgcagcag tttaccggta tgaacatcat catgtactac 840
   gcgccgcgta tcttcaaaat ggcgggcttt acgaccacag aacaacagat gattgcgact 900
   ctggtcgtgg gactgacctt tatgttcgcg accttcattg cggtctttac ggtagataaa 960
   gcaggtcgta aaccggctct gaaaattggt ttcagcgtga tggcgttagg cactctggtg 1020
   ctgggctatt gcctgatgca gtttgataac ggtacggctt ccagtggctt gtcctggctc 1080
   tctgttggca tgacgatgat gtgtattgcc ggttatgcga tgagcgccgc gccagtggtg 1140
   tggatcctgt gctctgaaat tcagccgctg aaatgccgcg atttcggtat tacctgttcg 1200
   acgacgacaa actgggtgtc gaatatgatt atcggcgcgg ccttcctgac actgcttgat 1260
   agcattggcg ctgccggtac gttctggctc tacactgcgc tgaacattgc gtttgtgggt 1320
   attactttct ggctcattcc ggaaaccaaa aatgtcacgc tggaacatat cgaacgcaaa 1380
   ctgatggcag gcgagaagtt gagaaatatc ggcgtc 1416
<210> 4
   <211> 472
   <212> PRT
   <213> Shigella flexneri
<400> 4
   Met Val Thr Ile Asn Thr Glu Ser Ala Leu Thr Pro Arg Ser Leu Arg
   1 5 10 15
   Asp Thr Arg Arg Met Asn Met Phe Val Ser Val Ala Ala Ala Val Ala
               20                  25                  30
   Gly Leu Leu Phe Gly Leu Asp Ile Gly Val Ile Ala Gly Ala Leu Pro
           35                  40                  45
   Phe Ile Thr Asp His Phe Val Leu Thr Ser Arg Leu Gln Glu Trp Val
       50                  55                  60
   Val Ser Ser Met Met Leu Gly Ala Ala Ile Gly Ala Leu Phe Asn Gly
   65                  70                  75                  80
   Trp Leu Ser Phe Arg Leu Gly Arg Lys Tyr Ser Leu Met Ala Gly Ala
                   85                  90                  95
   Ile Leu Phe Val Leu Gly Ser Ile Gly Ser Ala Phe Ala Thr Ser Val
               100                 105                 110
   Glu Met Leu Ile Ala Ala Arg Val Val Leu Gly Ile Ala Val Gly Ile
           115                 120                 125
   Ala Ser Tyr Thr Ala Pro Leu Tyr Leu Ser Glu Met Ala Ser Glu Asn
       130                 135                 140
   Val Arg Gly Lys Met Ile Ser Met Tyr Gln Leu Met Val Thr Leu Gly
   145                 150                 155                 160
   Ile Val Leu Ala Phe Leu Ser Asp Thr Ala Phe Ser Tyr Ser Gly Asn
                   165                 170                 175
   Trp Arg Ala Met Leu Gly Val Leu Ala Leu Pro Ala Val Leu Leu Ile
               180                 185                 190
   Ile Leu Val Val Phe Leu Pro Asn Ser Pro Arg Trp Leu Ala Glu Lys
           195                 200                 205
   Gly Arg His Ile Glu Ala Glu Glu Val Leu Arg Met Leu Arg Asp Thr
       210                 215                 220
   Ser Glu Lys Ala Arg Glu Glu Leu Asn Glu Ile Arg Glu Ser Leu Lys
   225                 230                 235                 240
   Leu Lys Gln Gly Gly Trp Ala Leu Phe Lys Ile Asn Arg Asn Val Arg
                   245                 250                 255
   Arg Ala Val Phe Leu Gly Met Leu Leu Gln Ala Met Gln Gln Phe Thr
               260                 265                 270
   Gly Met Asn Ile Ile Met Tyr Tyr Ala Pro Arg Ile Phe Lys Met Ala
   275 280 285
   Gly Phe Thr Thr Thr Glu Gln Gln Met Ile Ala Thr Leu Val Val Gly
   290 295 300
   Leu Thr Phe Met Phe Ala Thr Phe Ile Ala Val Phe Thr Val Asp Lys
   305 310 315 320
   Ala Gly Arg Lys Pro Ala Leu Lys Ile Gly Phe Ser Val Met Ala Leu
   325 330 335
   Gly Thr Leu Val Leu Gly Tyr Cys Leu Met Gln Phe Asp Asn Gly Thr
   340 345 350
   Ala Ser Ser Gly Leu Ser Trp Leu Ser Val Gly Met Thr Met Met Cys
   355 360 365
   Ile Ala Gly Tyr Ala Met Ser Ala Ala Pro Val Val Trp Ile Leu Cys
   370 375 380
   Ser Glu Ile Gln Pro Leu Lys Cys Arg Asp Phe Gly Ile Thr Cys Ser
   385 390 395 400
   Thr Thr Thr Asn Trp Val Ser Asn Met Ile Ile Gly Ala Ala Phe Leu
   405 410 415
   Thr Leu Leu Asp Ser Ile Gly Ala Ala Gly Thr Phe Trp Leu Tyr Thr
   420 425 430
   Ala Leu Asn Ile Ala Phe Val Gly Ile Thr Phe Trp Leu Ile Pro Glu
   435 440 445
   Thr Lys Asn Val Thr Leu Glu His Ile Glu Arg Lys Leu Met Ala Gly
   450 455 460
   Glu Lys Leu Arg Asn Ile Gly Val
   465 470
<210> 5
   <211> 1416
   <212> DNA
   <213> Shigella boydii
<400> 5
   atggttacta tcaatacgga atctgcttta acgccacgtt ctttgcggga tacgcggcgt 60
   atgaatatgt ttgtttcggt agctgctgcg gtcgcaggat tgttatttgg tcttgatatc 120
   ggcgtaatcg ccggagcgtt gccgttcatt accgatcact ttgtgctgac cagtcatttg 180
   caggaatggg tggttagtag catgatgctc ggcgcagcaa ttggtgcgct gtttaatggt 240
   tggctgtcgt tccgcctggg gcgtaaatac agcctgatgg cgggggccat cctgtttgta 300
   ctcggttcta tagggtccgc ttttgcgacc agcgtagaga tgttaatcgc cgctcgtgtg 360
   gtgctgggca ttgctgtcgg gatcgcgtct tacaccgctc ctctgtatct ttctgaaatg 420
   gcaagtgaaa acgttcgcgg taagatgatc agtatgtacc agttgatggt cacactcggc 480
   atcgtgctgg cgtttttatc cgatacagcg ttcagttata gcggtaactg gcgcgcaatg 540
   ttgggggttc ttgctttacc agcagttctg ctgattattc tggtggtctt cctgccaaat 600
   agcccgcgct ggttggcgga aaaggggcgt catattgagg cggaagaagt attgcgtatg 660
   ctgcgcgata cgtcggaaaa agcgcgagaa gaactcaacg aaattcgtga aagcctgaag 720
   ttaaaacagg gcggttgggc actgtttaag atcaaccgta acgtccgtcg tgctgtgttt 780
   ctcggtatgt tgttgcaggc gatgcagcag tttaccggta tgaacatcat catgtactac 840
   gcgccgcgta tcttcaaaat ggcgggcttt acgaccacag aacaacagat gattgcgact 900
   ctggtcgtag ggctgacctt tatgttcgcc acctttattg cggtgtttac ggtagataaa 960
   gcagggcgta aaccggctct gaaaattggt ttcagcgtga tggcgttagg cactctggtg 1020
   ctgggctatt gcctgatgca gtttgataac ggtacggctt ccagtggctt gtcctggctc 1080
   tctgttggca tgacgatgat gtgtattgcc ggttatgcga tgagcgccgc gccagtggtg 1140
   tggatcctgt gctctgaaat tcagccgctg aaatgccgcg atttcggtat tacctgttcg 1200
   accaccacga actgggtgtc gaatatgatt atcggcgcga ccttcctgac gctgctcgac 1260
   agcattggcg ctgccggtac gttctggctc tacactgcgc tgaacattgc gtttgtgggc 1320
   atcactttct ggctcattcc ggaaaccaaa aatgtcacgc tggaacatat cgaacgcaaa 1380
   ctgatggcag gcgagaagtt gagaaatatc ggcatc 1416
<210> 6
   <211> 472
   <212> PRT
   <213> Shigella boydii
<400> 6
   Met Val Thr Ile Asn Thr Glu Ser Ala Leu Thr Pro Arg Ser Leu Arg
   1 5 10 15
   Asp Thr Arg Arg Met Asn Met Phe Val Ser Val Ala Ala Ala Val Ala
   20 25 30
   Gly Leu Leu Phe Gly Leu Asp Ile Gly Val Ile Ala Gly Ala Leu Pro
   35 40 45
   Phe Ile Thr Asp His Phe Val Leu Thr Ser His Leu Gln Glu Trp Val
   50 55 60
   Val Ser Ser Met Met Leu Gly Ala Ala Ile Gly Ala Leu Phe Asn Gly
   65 70 75 80
   Trp Leu Ser Phe Arg Leu Gly Arg Lys Tyr Ser Leu Met Ala Gly Ala
   85 90 95
   Ile Leu Phe Val Leu Gly Ser Ile Gly Ser Ala Phe Ala Thr Ser Val
   100 105 110
   Glu Met Leu Ile Ala Ala Arg Val Val Leu Gly Ile Ala Val Gly Ile
   115 120 125
   Ala Ser Tyr Thr Ala Pro Leu Tyr Leu Ser Glu Met Ala Ser Glu Asn
   130 135 140
   Val Arg Gly Lys Met Ile Ser Met Tyr Gln Leu Met Val Thr Leu Gly
   145 150 155 160
   Ile Val Leu Ala Phe Leu Ser Asp Thr Ala Phe Ser Tyr Ser Gly Asn
   165 170 175
   Trp Arg Ala Met Leu Gly Val Leu Ala Leu Pro Ala Val Leu Leu Ile
   180 185 190
   Ile Leu Val Val Phe Leu Pro Asn Ser Pro Arg Trp Leu Ala Glu Lys
   195 200 205
   Gly Arg His Ile Glu Ala Glu Glu Val Leu Arg Met Leu Arg Asp Thr
   210 215 220
   Ser Glu Lys Ala Arg Glu Glu Leu Asn Glu Ile Arg Glu Ser Leu Lys
   225 230 235 240
   Leu Lys Gln Gly Gly Trp Ala Leu Phe Lys Ile Asn Arg Asn Val Arg
   245 250 255
   Arg Ala Val Phe Leu Gly Met Leu Leu Gln Ala Met Gln Gln Phe Thr
   260 265 270
   Gly Met Asn Ile Ile Met Tyr Tyr Ala Pro Arg Ile Phe Lys Met Ala
   275 280 285
   Gly Phe Thr Thr Thr Glu Gln Gln Met Ile Ala Thr Leu Val Val Gly
   290 295 300
   Leu Thr Phe Met Phe Ala Thr Phe Ile Ala Val Phe Thr Val Asp Lys
   305 310 315 320
   Ala Gly Arg Lys Pro Ala Leu Lys Ile Gly Phe Ser Val Met Ala Leu
   325 330 335
   Gly Thr Leu Val Leu Gly Tyr Cys Leu Met Gln Phe Asp Asn Gly Thr
   340 345 350
   Ala Ser Ser Gly Leu Ser Trp Leu Ser Val Gly Met Thr Met Met Cys
   355 360 365
   Ile Ala Gly Tyr Ala Met Ser Ala Ala Pro Val Val Trp Ile Leu Cys
   370 375 380
   Ser Glu Ile Gln Pro Leu Lys Cys Arg Asp Phe Gly Ile Thr Cys Ser
   385 390 395 400
   Thr Thr Thr Asn Trp Val Ser Asn Met Ile Ile Gly Ala Thr Phe Leu
   405 410 415
   Thr Leu Leu Asp Ser Ile Gly Ala Ala Gly Thr Phe Trp Leu Tyr Thr
   420 425 430
   Ala Leu Asn Ile Ala Phe Val Gly Ile Thr Phe Trp Leu Ile Pro Glu
   435 440 445
   Thr Lys Asn Val Thr Leu Glu His Ile Glu Arg Lys Leu Met Ala Gly
   450 455 460
   Glu Lys Leu Arg Asn Ile Gly Ile
   465 470
<210> 7
   <211> 1416
   <212> DNA
   <213> Shigella dysenteriae
<400> 7
   atggttacta tcaatacgga atctgcttta acgccacgtt ctttgcgtga tacgcggcgt      60
   atgaatatgt ttgtttcggt agctgctgcg gtcgcaggat tgttatttgg tcttgatatc     120
   ggcgtaatcg ccggagcgtt gccgttcatt accgatcact ttgtgctgac cagtcgtttg     180
   caggaatggg tggttagtag catgatgctc ggcgcagcaa ttggtgcgct gtttaatggt     240
   tggctgtcgt tccgcctggg gcgtaaatac agcctgatgg cgggggccat cctgtttgta     300
   ctcggttcta tagggtccgc ttttgctacc agcgtagaga tgttaatcgc cgctcgtgtg     360
   gtgctgggca ttgctgtcgg gatcgcgtct tacaccgctc ctctgtatct ttctgaaatg     420
   gcaagtgaaa acgttcgcgg taagatgatc agtatgtacc agttgatggt cacactcggc     480
   atcgtgctgg cgtttttatc cgatacagcg ttcagttata gcggtaactg gcgcgcaatg     540
   ttgggggttc ttgctttacc agcagtcctg ctgattattc tggtggtctt cctgccaaat     600
   agcccgcgct ggctggcgga aaaggggcgt catattgagg cggaagaagt gttgcgtatg     660
   ctgcgcgata cgtcggaaaa agcgcgagaa gaactcaacg aaattcgtga aagcctgaag     720
   ttaaaacaag gcggttgggc actgtttaag atcaaccgta acgtccgtcg tgctgtgttt     780
   ctcggtatgt tgttgcaggc gatgcagcag tttaccggta tgaacatcat catgtactat     840
   gcgccgcgta tcttcaaaat ggcgggcttt acgaccacag aacaacagat gattgcgact     900
   ctggtcgtgg gactgacctt tatgttcgcg accttcattg cggtctttac ggtagataaa     960
   gcaggtcgta aaccggctct gaaaattggt ttcagcgtga tggcgttagg cactctggtg    1020
   ctgggctatt gcctgatgca gtttgataac ggtacggctt ccagtggctt gtcctggctc    1080
   tctgttggca tgacgatgat gtgtattgcc ggttatgcga tgagcgccgc gccagtggtg    1140
   tggatcctgt gctctgaaat tcagccgctg aaatgccacg atttcggtat tacctgttcg    1200
   acgacgacaa actgggtgtc gaatatgatt atcggcgcga ccttcctgac actgcttgat    1260
   agcattggcg ctgccggtac gttctggctc tacactgcgc tgaacattgc gtttgtgggc    1320
   atcactttct ggctcattcc ggaaaccaaa aatgtcacgc tggaacatat cgaacgcaaa    1380
   ctgatggcag gcgagaagtt gagaaatatc ggcgtc                              1416
<210> 8
   <211> 472
   <212> PRT
   <213> Shigella dysenteriae
<400> 8
   Met Val Thr Ile Asn Thr Glu Ser Ala Leu Thr Pro Arg Ser Leu Arg
   1               5                   10                  15
   Asp Thr Arg Arg Met Asn Met Phe Val Ser Val Ala Ala Ala Val Ala
               20                  25                  30
   Gly Leu Leu Phe Gly Leu Asp Ile Gly Val Ile Ala Gly Ala Leu Pro
           35                  40                  45
   Phe Ile Thr Asp His Phe Val Leu Thr Ser Arg Leu Gln Glu Trp Val
       50                  55                  60
   Val Ser Ser Met Met Leu Gly Ala Ala Ile Gly Ala Leu Phe Asn Gly
   65                  70                  75                  80
   Trp Leu Ser Phe Arg Leu Gly Arg Lys Tyr Ser Leu Met Ala Gly Ala
                   85                  90                  95
   Ile Leu Phe Val Leu Gly Ser Ile Gly Ser Ala Phe Ala Thr Ser Val
               100                 105                 110
   Glu Met Leu Ile Ala Ala Arg Val Val Leu Gly Ile Ala Val Gly Ile
           115                 120                 125
   Ala Ser Tyr Thr Ala Pro Leu Tyr Leu Ser Glu Met Ala Ser Glu Asn
       130                 135                 140
   Val Arg Gly Lys Met Ile Ser Met Tyr Gln Leu Met Val Thr Leu Gly
   145                 150                 155                 160
   Ile Val Leu Ala Phe Leu Ser Asp Thr Ala Phe Ser Tyr Ser Gly Asn
                   165                 170                 175
   Trp Arg Ala Met Leu Gly Val Leu Ala Leu Pro Ala Val Leu Leu Ile
               180                 185                 190
   Ile Leu Val Val Phe Leu Pro Asn Ser Pro Arg Trp Leu Ala Glu Lys
           195                 200                 205
   Gly Arg His Ile Glu Ala Glu Glu Val Leu Arg Met Leu Arg Asp Thr
       210                 215                 220
   Ser Glu Lys Ala Arg Glu Glu Leu Asn Glu Ile Arg Glu Ser Leu Lys
   225                 230                 235                 240
   Leu Lys Gln Gly Gly Trp Ala Leu Phe Lys Ile Asn Arg Asn Val Arg
                   245                 250                 255
   Arg Ala Val Phe Leu Gly Met Leu Leu Gln Ala Met Gln Gln Phe Thr
               260                 265                 270
   Gly Met Asn Ile Ile Met Tyr Tyr Ala Pro Arg Ile Phe Lys Met Ala
           275                 280                 285
   Gly Phe Thr Thr Thr Glu Gln Gln Met Ile Ala Thr Leu Val Val Gly
       290                 295                 300
   Leu Thr Phe Met Phe Ala Thr Phe Ile Ala Val Phe Thr Val Asp Lys
   305                 310                 315                 320
   Ala Gly Arg Lys Pro Ala Leu Lys Ile Gly Phe Ser Val Met Ala Leu
                   325                 330                 335
   Gly Thr Leu Val Leu Gly Tyr Cys Leu Met Gln Phe Asp Asn Gly Thr
               340                 345                 350
   Ala Ser Ser Gly Leu Ser Trp Leu Ser Val Gly Met Thr Met Met Cys
           355                 360                 365
   Ile Ala Gly Tyr Ala Met Ser Ala Ala Pro Val Val Trp Ile Leu Cys
       370                 375                 380
   Ser Glu Ile Gln Pro Leu Lys Cys His Asp Phe Gly Ile Thr Cys Ser
   385 390 395 400
   Thr Thr Thr Asn Trp Val Ser Asn Met Ile Ile Gly Ala Thr Phe Leu
   405 410 415
   Thr Leu Leu Asp Ser Ile Gly Ala Ala Gly Thr Phe Trp Leu Tyr Thr
   420 425 430
   Ala Leu Asn Ile Ala Phe Val Gly Ile Thr Phe Trp Leu Ile Pro Glu
   435 440 445
   Thr Lys Asn Val Thr Leu Glu His Ile Glu Arg Lys Leu Met Ala Gly
   450 455 460
   Glu Lys Leu Arg Asn Ile Gly Val
   465 470
<210> 9
   <211> 1416
   <212> DNA
   <213> Salmonella typhimurium
<400> 9
   atggtctcta ttaatcatga ctctgcttta acgccgcgtt cgcttcgcga cacacgacgt 60
   atgaatatgt ttgtttcggt ttctgcagcg gtagcgggac tgttatttgg tctggatatc 120
   ggcgttatcg ccggggcgct gccttttatt accgaccatt tcgtactgac cagccggctg 180
   caggaatggg tcgtcagcag catgatgctt ggcgcggcaa ttggcgcatt atttaacggc 240
   tggctttcat tccggctggg gcgtaagtat agcctgatgg ctggcgcgat tttgttcgtg 300
   ctcggctcgc tggggtcggc gtttgcttcc agcgtggaag tattgattgg cgcccgcgtg 360
   atactgggcg tagcagtagg gattgcctcc tataccgcgc cgctttatct ctctgaaatg 420
   gcaagtgaaa atgttcgcgg caaaatgatc agtatgtatc aactgatggt gacgttaggc 480
   attgtgctgg cttttttatc cgatacggca ttcagctaca gcggcaactg gcgcgcgatg 540
   ttgggcgtgc tggcgctgcc tgcggtgttg ctcattattc tggtggtatt cctgccgaat 600
   agtccgcgtt ggctggcgca aaaaggtcgc catattgaag cggaagaggt gctgcgtatg 660
   ctgcgcgata cctcggaaaa agcccgtgat gaactgaatg agattcggga aagcctcaaa 720
   ctcaagcagg gagggtgggc attatttaaa gctaaccgca atgttcgccg cgccgtgttc 780
   ctcggtatgc tgctacaggc aatgcagcag ttcaccggca tgaacatcat tatgtactat 840
   gcgccgcgca tttttaaaat ggccggcttt accaccacgg aacagcaaat gatcgccacg 900
   ctggtggtcg gactgacttt tatgttcgcg acgtttatcg ccgtctttac ggtcgataag 960
   gccgggcgta aaccggcgtt aaaaatcggt ttcagcgtaa tggcgttagg gacattggtg 1020
   ttgggctact gcctgatgca gtttgataac ggtacggcat caagcggtct ctcctggctt 1080
   tccgttggga tgacgatgat gtgtatcgcc ggttacgcga tgagcgccgc tccggtggtg 1140
   tggatactgt gttcggaaat ccagccgctg aaatgccgtg attttggcat tacctgttca 1200
   accacgacaa actgggtatc gaacatgatc atcggcgcga cattcctgac actgttggac 1260
   agcattggcg cggcaggtac attctggctc tacaccgcgc tgaatatcgc ttttatcggc 1320
   atcactttct ggctgattcc ggaaaccaaa aatgtcaccc tggagcacat cgaacgcaag 1380
   ctgatggcgg gcgagaagct aagaaatatt ggcgtg 1416
<210> 10
   <211> 472
   <212> PRT
   <213> Salmonella typhimurium
<400> 10
   Met Val Ser Ile Asn His Asp Ser Ala Leu Thr Pro Arg Ser Leu Arg
   1               5                   10                  15
   Asp Thr Arg Arg Met Asn Met Phe Val Ser Val Ser Ala Ala Val Ala
               20                  25                  30
   Gly Leu Leu Phe Gly Leu Asp Ile Gly Val Ile Ala Gly Ala Leu Pro
           35                  40                  45
   Phe Ile Thr Asp His Phe Val Leu Thr Ser Arg Leu Gln Glu Trp Val
       50                  55                  60
   Val Ser Ser Met Met Leu Gly Ala Ala Ile Gly Ala Leu Phe Asn Gly
   65                  70                  75                  80
   Trp Leu Ser Phe Arg Leu Gly Arg Lys Tyr Ser Leu Met Ala Gly Ala
                   85                  90                  95
   Ile Leu Phe Val Leu Gly Ser Leu Gly Ser Ala Phe Ala Ser Ser Val
               100                 105                 110
   Glu Val Leu Ile Gly Ala Arg Val Ile Leu Gly Val Ala Val Gly Ile
           115                 120                 125
   Ala Ser Tyr Thr Ala Pro Leu Tyr Leu Ser Glu Met Ala Ser Glu Asn
       130                 135                 140
   Val Arg Gly Lys Met Ile Ser Met Tyr Gln Leu Met Val Thr Leu Gly
   145                 150                 155                 160
   Ile Val Leu Ala Phe Leu Ser Asp Thr Ala Phe Ser Tyr Ser Gly Asn
                   165                 170                 175
   Trp Arg Ala Met Leu Gly Val Leu Ala Leu Pro Ala Val Leu Leu Ile
   180 185 190
   Ile Leu Val Val Phe Leu Pro Asn Ser Pro Arg Trp Leu Ala Gln Lys
   195 200 205
   Gly Arg His Ile Glu Ala Glu Glu Val Leu Arg Met Leu Arg Asp Thr
   210 215 220
   Ser Glu Lys Ala Arg Asp Glu Leu Asn Glu Ile Arg Glu Ser Leu Lys
   225 230 235 240
   Leu Lys Gln Gly Gly Trp Ala Leu Phe Lys Ala Asn Arg Asn Val Arg
   245 250 255
   Arg Ala Val Phe Leu Gly Met Leu Leu Gln Ala Met Gln Gln Phe Thr
   260 265 270
   Gly Met Asn Ile Ile Met Tyr Tyr Ala Pro Arg Ile Phe Lys Met Ala
   275 280 285
   Gly Phe Thr Thr Thr Glu Gln Gln Met Ile Ala Thr Leu Val Val Gly
   290 295 300
   Leu Thr Phe Met Phe Ala Thr Phe Ile Ala Val Phe Thr Val Asp Lys
   305 310 315 320
   Ala Gly Arg Lys Pro Ala Leu Lys Ile Gly Phe Ser Val Met Ala Leu
   325 330 335
   Gly Thr Leu Val Leu Gly Tyr Cys Leu Met Gln Phe Asp Asn Gly Thr
   340 345 350
   Ala Ser Ser Gly Leu Ser Trp Leu Ser Val Gly Met Thr Met Met Cys
   355 360 365
   Ile Ala Gly Tyr Ala Met Ser Ala Ala Pro Val Val Trp Ile Leu Cys
   370 375 380
   Ser Glu Ile Gln Pro Leu Lys Cys Arg Asp Phe Gly Ile Thr Cys Ser
   385 390 395 400
   Thr Thr Thr Asn Trp Val Ser Asn Met Ile Ile Gly Ala Thr Phe Leu
   405 410 415
   Thr Leu Leu Asp Ser Ile Gly Ala Ala Gly Thr Phe Trp Leu Tyr Thr
   420 425 430
   Ala Leu Asn Ile Ala Phe Ile Gly Ile Thr Phe Trp Leu Ile Pro Glu
   435 440 445
   Thr Lys Asn Val Thr Leu Glu His Ile Glu Arg Lys Leu Met Ala Gly
   450 455 460
   Glu Lys Leu Arg Asn Ile Gly Val
   465 470
<210> 11
   <211> 1431
   <212> DNA
   <213> Salmonella enterica
<400> 11
   ttgtggcagg aaaatatggt ctctattaat catgactctg ctttaacgcc gcgttcgctt      60
   cgcgacacac gacgtatgaa tatgtttgtt tcggtttctg cagcggtagc gggactgtta     120
   tttggtctgg atatcggcgt tatcgccggg gcgctgcctt ttattaccga ccatttcgta     180
   ctgaccagcc ggctgcagga atgggtcgtc agcagtatga tgcttggcgc ggcaattggc     240
   gcattattta acggctggct ttcattccgg ctggggcgta agtatagcct gatggctggc     300
   gcgattttgt tcgtgctcgg ctcgctgggg tcggcgtttg cttccagcgt ggaagtattg     360
   attggcgccc gcgtgatact gggcgtagca gtagggattg cgtcctatac cgcgccgctt     420
   tatctctctg aaatggcaag tgaaaatgtt cgcggcaaaa tgatcagtat gtatcaactg     480
   atggtgacgt taggcattgt gctggctttt ttatccgata cggcattcag ctacagcggc     540
   aactggcgcg cgatgttggg cgtgctggcg ctgcctgcgg tgttgctcat tattctcgtg     600
   gtattcctgc cgaatagtcc gcgttggctg gcgcaaaaag gtcgccatat tgaagcggaa     660
   gaggtgctgc gtatgctgcg cgatacctcg gaaaaagccc gtgatgaact gaatgagatt     720
   cgggaaagcc tcaaactcaa gcagggcggg tgggcattat ttaaagctaa ccgcaatgtt     780
   cgccgcgccg tgttcctcgg tatgctgcta caggcaatgc agcagttcac cggcatgaac     840
   atcattatgt actatgcgcc gcgcattttt aaaatggccg gctttaccac cacggaacag     900
   caaatgatcg ccacgctggt ggtcggactg acctttatgt tcgcgacgtt tatcgccgtc     960
   tttacggtcg ataaggccgg gcgtaaaccg gcgttaaaaa tcggtttcag cgtaatggcg    1020
   ttagggacat tggtgttggg ctactgcctg atgcagtttg ataacggtac ggcatcaagc    1080
   ggtctctcct ggctttccgt tgggatgacg atgatgtgta tcgccggtta cgcgatgagc    1140
   gccgctccgg tggtgtggat actgtgttcg gaaatccagc cgctgaaatg ccgtgatttt    1200
   ggcattacct gttcaaccac gacaaactgg gtatcgaaca tgatcatcgg cgcgacattc    1260
   ctgacactgt tggacagtat tggcgcggca ggtacattct ggctctacac cgcgctgaat    1320
   atcgctttta tcggcatcac tttctggctg attccggaaa ccaaaaatgt caccctggag    1380
   catatcgaac gcaagctaat ggcgggcgag aagctaagaa atattggcgt g             1431
<210> 12
   <211> 477
   <212> PRT
   <213> Salmonella enterica
<400> 12
   Met Trp Gln Glu Asn Met Val Ser Ile Asn His Asp Ser Ala Leu Thr
   1               5                   10                  15
   Pro Arg Ser Leu Arg Asp Thr Arg Arg Met Asn Met Phe Val Ser Val
               20                  25                  30
   Ser Ala Ala Val Ala Gly Leu Leu Phe Gly Leu Asp Ile Gly Val Ile
           35                  40                  45
   Ala Gly Ala Leu Pro Phe Ile Thr Asp His Phe Val Leu Thr Ser Arg
       50                  55                  60
   Leu Gln Glu Trp Val Val Ser Ser Met Met Leu Gly Ala Ala Ile Gly
   65                  70                  75                  80
   Ala Leu Phe Asn Gly Trp Leu Ser Phe Arg Leu Gly Arg Lys Tyr Ser
                   85                  90                  95
   Leu Met Ala Gly Ala Ile Leu Phe Val Leu Gly Ser Leu Gly Ser Ala
               100                 105                 110
   Phe Ala Ser Ser Val Glu Val Leu Ile Gly Ala Arg Val Ile Leu Gly
           115                 120                 125
   Val Ala Val Gly Ile Ala Ser Tyr Thr Ala Pro Leu Tyr Leu Ser Glu
       130                 135                 140
   Met Ala Ser Glu Asn Val Arg Gly Lys Met Ile Ser Met Tyr Gln Leu
   145                 150                 155                 160
   Met Val Thr Leu Gly Ile Val Leu Ala Phe Leu Ser Asp Thr Ala Phe
                   165                 170                 175
   Ser Tyr Ser Gly Asn Trp Arg Ala Met Leu Gly Val Leu Ala Leu Pro
               180                 185                 190
   Ala Val Leu Leu Ile Ile Leu Val Val Phe Leu Pro Asn Ser Pro Arg
           195                 200                 205
   Trp Leu Ala Gln Lys Gly Arg His Ile Glu Ala Glu Glu Val Leu Arg
       210                 215                 220
   Met Leu Arg Asp Thr Ser Glu Lys Ala Arg Asp Glu Leu Asn Glu Ile
   225 230 235 240
   Arg Glu Ser Leu Lys Leu Lys Gln Gly Gly Trp Ala Leu Phe Lys Ala
   245 250 255
   Asn Arg Asn Val Arg Arg Ala Val Phe Leu Gly Met Leu Leu Gln Ala
   260 265 270
   Met Gln Gln Phe Thr Gly Met Asn Ile Ile Met Tyr Tyr Ala Pro Arg
   275 280 285
   Ile Phe Lys Met Ala Gly Phe Thr Thr Thr Glu Gln Gln Met Ile Ala
   290 295 300
   Thr Leu Val Val Gly Leu Thr Phe Met Phe Ala Thr Phe Ile Ala Val
   305 310 315 320
   Phe Thr Val Asp Lys Ala Gly Arg Lys Pro Ala Leu Lys Ile Gly Phe
   325 330 335
   Ser Val Met Ala Leu Gly Thr Leu Val Leu Gly Tyr Cys Leu Met Gln
   340 345 350
   Phe Asp Asn Gly Thr Ala Ser Ser Gly Leu Ser Trp Leu Ser Val Gly
   355 360 365
   Met Thr Met Met Cys Ile Ala Gly Tyr Ala Met Ser Ala Ala Pro Val
   370 375 380
   Val Trp Ile Leu Cys Ser Glu Ile Gln Pro Leu Lys Cys Arg Asp Phe
   385 390 395 400
   Gly Ile Thr Cys Ser Thr Thr Thr Asn Trp Val Ser Asn Met Ile Ile
   405 410 415
   Gly Ala Thr Phe Leu Thr Leu Leu Asp Ser Ile Gly Ala Ala Gly Thr
   420 425 430
   Phe Trp Leu Tyr Thr Ala Leu Asn Ile Ala Phe Ile Gly Ile Thr Phe
   435 440 445
   Trp Leu Ile Pro Glu Thr Lys Asn Val Thr Leu Glu His Ile Glu Arg
   450 455 460
   Lys Leu Met Ala Gly Glu Lys Leu Arg Asn Ile Gly Val
   465 470 475
<210> 13
   <211> 1419
   <212> DNA
   <213> Klebsiella pneumoniae
<400> 13
   atgacttcaa tcagtaacga ctctgcatta acgccgcgga cacaacgtga cacccggcgg      60
   atgaactggt ttgtttctat cgctgcggcg gtagcggggt tgctctttgg cctggatatc     120
   ggcgtgatat ccggggcgct gccctttatt accgaccact tcaccttatc cagccagctt    180
   caggagtggg tggtcagcag tatgatgttg ggggcggcga tcggtgcgct gtttaacggc      240
   tggctgtcgt tccgcctcgg ccgtaaatac agcctgatgg cgggggctgt gctctttgtt     300
   gccggctcta tcggctccgc ttttgccgcc agcgtggagg tgctgctgat agcccgcgtg     360
   gtgttggggg tggccgtcgg gatcgcttcc tataccgcgc cgttgtacct ctccgagatg     420
   gccagtgaga acgtgcgcgg gaaaatgatc agtatgtacc agctgatggt gaccctcggc     480
   attgtgctgg cgtttctttc cgatactgcc tttagctaca gcggtaactg gcgcgccatg     540
   ttaggcgtgc tggcactgcc ggcggtgatc ctgattattc tggtcgtctt tttgccgaac     600
   agcccgcgct ggctggcgga gaaaggacgc catatcgaag cggaagaggt gctgcggatg     660
   ctgcgcgata cctcggaaaa ggcgcgcgac gagcttaacg agatccgtga gagcctgaag     720
   ctgaagcagg gcggctgggc gttgtttaag gtcaatcgta acgtgcgccg ggcggtgttc     780
   cttggcatgc tgctgcaggc gatgcagcag ttcaccggca tgaacatcat catgtactac     840
   gcgccgcgta tctttaaaat ggcgggcttt accactaccg aacagcagat gatcgccacc     900
   ctggtggtgg gcctgacctt tatgtttgcc acctttattg cggtgttcac ggtggataaa     960
   gcgggtcgta agccggcgct aaaaatcggc tttagcgtga tggcgctggg caccctggtg    1020
   ctgggctact gcctgatgca gttcgacaat ggcaccgcct ccagcggtct ctcctggctt    1080
   tccgtcggca tgaccatgat gtgtattgcc gggtatgcga tgagcgcggc gccggtggtg    1140
   tggatcctct gctccgagat ccagccgctg aaatgccgcg acttcggtat cacctgctcg    1200
   accaccacca actgggtgtc gaacatgatc atcggcgcca ccttcctgac gctgcttgac    1260
   gcgattggcg ccgccggcac cttctggctc tacacggtgc tcaacgtggc ctttatcggc    1320
   gtcaccttct ggctgatccc ggaaaccaag aatgtcaccc tcgagcacat tgagcgcaac    1380
   ctgatggcgg gcgagaagct gcgcaacatc ggtaaccgt                           1419
<210> 14
   <211> 473
   <212> PRT
   <213> Klebsiella pneumoniae
<400> 14
   Met Thr Ser Ile Ser Asn Asp Ser Ala Leu Thr Pro Arg Thr Gln Arg
   1               5                   10                  15
   Asp Thr Arg Arg Met Asn Trp Phe Val Ser Ile Ala Ala Ala Val Ala
   20 25 30
   Gly Leu Leu Phe Gly Leu Asp Ile Gly Val Ile Ser Gly Ala Leu Pro
   35 40 45
   Phe Ile Thr Asp His Phe Thr Leu Ser Ser Gln Leu Gln Glu Trp Val
   50 55 60
   Val Ser Ser Met Met Leu Gly Ala Ala Ile Gly Ala Leu Phe Asn Gly
   65 70 75 80
   Trp Leu Ser Phe Arg Leu Gly Arg Lys Tyr Ser Leu Met Ala Gly Ala
   85 90 95
   Val Leu Phe Val Ala Gly Ser Ile Gly Ser Ala Phe Ala Ala Ser Val
   100 105 110
   Glu Val Leu Leu Ile Ala Arg Val Val Leu Gly Val Ala Val Gly Ile
   115 120 125
   Ala Ser Tyr Thr Ala Pro Leu Tyr Leu Ser Glu Met Ala Ser Glu Asn
   130 135 140
   Val Arg Gly Lys Met Ile Ser Met Tyr Gln Leu Met Val Thr Leu Gly
   145 150 155 160
   Ile Val Leu Ala Phe Leu Ser Asp Thr Ala Phe Ser Tyr Ser Gly Asn
   165 170 175
   Trp Arg Ala Met Leu Gly Val Leu Ala Leu Pro Ala Val Ile Leu Ile
   180 185 190
   Ile Leu Val Val Phe Leu Pro Asn Ser Pro Arg Trp Leu Ala Glu Lys
   195 200 205
   Gly Arg His Ile Glu Ala Glu Glu Val Leu Arg Met Leu Arg Asp Thr
   210 215 220
   Ser Glu Lys Ala Arg Asp Glu Leu Asn Glu Ile Arg Glu Ser Leu Lys
   225 230 235 240
   Leu Lys Gln Gly Gly Trp Ala Leu Phe Lys Val Asn Arg Asn Val Arg
   245 250 255
   Arg Ala Val Phe Leu Gly Met Leu Leu Gln Ala Met Gln Gln Phe Thr
   260 265 270
   Gly Met Asn Ile Ile Met Tyr Tyr Ala Pro Arg Ile Phe Lys Met Ala
   275 280 285
   Gly Phe Thr Thr Thr Glu Gln Gln Met Ile Ala Thr Leu Val Val Gly
   290 295 300
   Leu Thr Phe Met Phe Ala Thr Phe Ile Ala Val Phe Thr Val Asp Lys
   305 310 315 320
   Ala Gly Arg Lys Pro Ala Leu Lys Ile Gly Phe Ser Val Met Ala Leu
   325 330 335
   Gly Thr Leu Val Leu Gly Tyr Cys Leu Met Gln Phe Asp Asn Gly Thr
   340 345 350
   Ala Ser Ser Gly Leu Ser Trp Leu Ser Val Gly Met Thr Met Met Cys
   355 360 365
   Ile Ala Gly Tyr Ala Met Ser Ala Ala Pro Val Val Trp Ile Leu Cys
   370 375 380
   Ser Glu Ile Gln Pro Leu Lys Cys Arg Asp Phe Gly Ile Thr Cys Ser
   385 390 395 400
   Thr Thr Thr Asn Trp Val Ser Asn Met Ile Ile Gly Ala Thr Phe Leu
   405 410 415
   Thr Leu Leu Asp Ala Ile Gly Ala Ala Gly Thr Phe Trp Leu Tyr Thr
   420 425 430
   Val Leu Asn Val Ala Phe Ile Gly Val Thr Phe Trp Leu Ile Pro Glu
   435 440 445
   Thr Lys Asn Val Thr Leu Glu His Ile Glu Arg Asn Leu Met Ala Gly
   450 455 460
   Glu Lys Leu Arg Asn Ile Gly Asn Arg
   465 470
<210> 15
   <211> 1416
   <212> DNA
   <213> Klebsiella oxytoca
<400> 15
   atgaccactc tcagtcacga ctctacaacc atgccgcgta cgcagcgcga tacccggcgc      60
   atgaatcagt ttgtctccat tgccgccgcg gtggcagggt tgctgtttgg cctcgatatc     120
   ggggtgattg ccggggcgct gccctttatt accgaccatt ttgttttatc cagccgcctg     180
   caggagtggg tggtgagcag catgatgctg ggagccgcca tcggcgcgtt atttaacggc     240
   tggctctctt tccgcctcgg gcgcaaatac agcctgatgg tgggcgcggt gctgttcgtt     300
   gccggctccg tgggctccgc gtttgcgacc agcgtcgaaa tgctgctggt ggcaaggatc     360
   gttctcgggg tcgccgtggg gatcgcctct tataccgcgc cgctgtacct gtcggaaatg     420
   gcgagcgaaa acgtgcgcgg caagatgatc agcatgtatc agctgatggt gacgctgggt     480
   atcgtgatgg cgtttctctc cgacaccgcg ttcagctaca gcggcaactg gcgggcgatg     540
   cttggcgtac tggcgctgcc ggcggtggtg ctgattattc tggtgatctt cctgccgaac     600
   agcccgcgct ggctggcgga aaaagggcgt cacgtggaag cggaagaggt gctgcggatg     660
   ctgcgcgaca cgtcagaaaa agcccgtgac gagctcaacg agatccgcga aagcctgaag     720
   ctgaagcagg gcggctgggc gctgtttaag gtcaaccgca acgtgcggcg ggcggtattc     780
   ctcggcatgc tgttgcaggc gatgcagcag tttaccggta tgaatatcat catgtactac     840
   gcgccgcgca tctttaaaat ggcgggcttc accaccaccg aacagcagat ggtcgcgacc     900
   ctggtggttg gcctgacctt tatgttcgcc acctttatcg ccgtctttac cgtcgataag     960
   gccggacgta agccggcgct gaaaatcggt tttagcgtga tggccatcgg cacgctggtg    1020
   ctgggctact gtctgatgca gtttgataac ggcaccgcct ccagcggtct ctcctggctg    1080
   tcggtgggga tgaccatgat gtgtatcgcc ggctatgcga tgagcgccgc gccggtggtg    1140
   tggatcctgt gttcggaaat tcagccgctg aagtgccgcg atttcggcat cacctgctca    1200
   accaccacca actgggtgtc gaacatgatt atcggcgcga ccttcctgac gctgctggac    1260
   gcgatcggcg cggcaggaac cttctggctt tataccgcgc tgaacgtcgc ctttatcggc    1320
   gtgacgttct ggctgatccc ggaaaccaaa aacgtcaccc tggagcatat tgaacgcagg    1380
   ctgatgtccg gcgagaagct gcgcaatatc ggcaat                               1416
<210> 16
   <211> 472
   <212> PRT
   <213> Klebsiella oxytoca
<400> 16
   Met Thr Thr Leu Ser His Asp Ser Thr Thr Met Pro Arg Thr Gln Arg
   1               5                   10                  15
   Asp Thr Arg Arg Met Asn Gln Phe Val Ser Ile Ala Ala Ala Val Ala
               20                  25                  30
   Gly Leu Leu Phe Gly Leu Asp Ile Gly Val Ile Ala Gly Ala Leu Pro
           35                  40                  45
   Phe Ile Thr Asp His Phe Val Leu Ser Ser Arg Leu Gln Glu Trp Val
       50                  55                  60
   Val Ser Ser Met Met Leu Gly Ala Ala Ile Gly Ala Leu Phe Asn Gly
   65 70 75 80
   Trp Leu Ser Phe Arg Leu Gly Arg Lys Tyr Ser Leu Met Val Gly Ala
   85 90 95
   Val Leu Phe Val Ala Gly Ser Val Gly Ser Ala Phe Ala Thr Ser Val
   100 105 110
   Glu Met Leu Leu Val Ala Arg Ile Val Leu Gly Val Ala Val Gly Ile
   115 120 125
   Ala Ser Tyr Thr Ala Pro Leu Tyr Leu Ser Glu Met Ala Ser Glu Asn
   130 135 140
   Val Arg Gly Lys Met Ile Ser Met Tyr Gln Leu Met Val Thr Leu Gly
   145 150 155 160
   Ile Val Met Ala Phe Leu Ser Asp Thr Ala Phe Ser Tyr Ser Gly Asn
   165 170 175
   Trp Arg Ala Met Leu Gly Val Leu Ala Leu Pro Ala Val Val Leu Ile
   180 185 190
   Ile Leu Val Ile Phe Leu Pro Asn Ser Pro Arg Trp Leu Ala Glu Lys
   195 200 205
   Gly Arg His Val Glu Ala Glu Glu Val Leu Arg Met Leu Arg Asp Thr
   210 215 220
   Ser Glu Lys Ala Arg Asp Glu Leu Asn Glu Ile Arg Glu Ser Leu Lys
   225 230 235 240
   Leu Lys Gln Gly Gly Trp Ala Leu Phe Lys Val Asn Arg Asn Val Arg
   245 250 255
   Arg Ala Val Phe Leu Gly Met Leu Leu Gln Ala Met Gln Gln Phe Thr
   260 265 270
   Gly Met Asn Ile Ile Met Tyr Tyr Ala Pro Arg Ile Phe Lys Met Ala
   275 280 285
   Gly Phe Thr Thr Thr Glu Gln Gln Met Val Ala Thr Leu Val Val Gly
   290 295 300
   Leu Thr Phe Met Phe Ala Thr Phe Ile Ala Val Phe Thr Val Asp Lys
   305 310 315 320
   Ala Gly Arg Lys Pro Ala Leu Lys Ile Gly Phe Ser Val Met Ala Ile
   325 330 335
   Gly Thr Leu Val Leu Gly Tyr Cys Leu Met Gln Phe Asp Asn Gly Thr
   340 345 350
   Ala Ser Ser Gly Leu Ser Trp Leu Ser Val Gly Met Thr Met Met Cys
   355 360 365
   Ile Ala Gly Tyr Ala Met Ser Ala Ala Pro Val Val Trp Ile Leu Cys
   370 375 380
   Ser Glu Ile Gln Pro Leu Lys Cys Arg Asp Phe Gly Ile Thr Cys Ser
   385 390 395 400
   Thr Thr Thr Asn Trp Val Ser Asn Met Ile Ile Gly Ala Thr Phe Leu
   405 410 415
   Thr Leu Leu Asp Ala Ile Gly Ala Ala Gly Thr Phe Trp Leu Tyr Thr
   420 425 430
   Ala Leu Asn Val Ala Phe Ile Gly Val Thr Phe Trp Leu Ile Pro Glu
   435 440 445
   Thr Lys Asn Val Thr Leu Glu His Ile Glu Arg Arg Leu Met Ser Gly
   450 455 460
   Glu Lys Leu Arg Asn Ile Gly Asn
   465 470
<210> 17
   <211> 1413
   <212> DNA
   <213> Enterobacter cancerogenus
<400> 17
   atgacatctc tcaatgactc taccctcatg cccgcggcgc tgcgcgacac ccgccgcatg 60
   aaccagtttg tctccgtcgc ggcggccgta gcgggtctgc tgtttgggct ggatatcggc 120
   gttatcgccg gtgcgctgcc gtttatcacc gatcatttca cgttaagtca tcgcctgcag 180
   gagtgggtgg tgagcagcat gatgctgggc gccgcaattg gggcgttgtt caacggctgg 240
   ctctcgttcc gcctgggacg aaagtacagc ctgatggtcg gggcgatcct gtttgtggcc 300
   ggttcactgg ggtcggcgtt tgccacaagc gttgaggtgc tgttgctctc ccgcgtgctg 360
   cttggcgtgg cggtggggat cgcctcctac accgcgccgc tgtatctctc cgaaatggcg 420
   agcgagaacg tgcgcggcaa gatgatcagc atgtatcagc tgatggtgac gctcggcatc 480
   gtgctggcgt ttctttccga tacctggttc agctacaccg gtaactggcg cgccatgctc 540
   ggcgtgctgg cgttgcccgc gctgttgctg atggtgctgg tgattttcct gccgaacagc 600
   ccgcgctggc tggcgcaaaa aggccgccac gtcgaggcgg aagaagtgct gcgaatgctg 660
   cgtgacacct ctgaaaaagc gcgtgaagag ttgaacgaga tccgcgaaag cctgaagctg 720
   aagcagggcg gctgggcgct gtttaaggtc aaccgcaacg tgcgccgcgc cgtgtttctg 780
   ggaatgctct tgcaggcgat gcagcagttt acgggcatga acatcatcat gtactacgcc 840
   ccgcgcatct ttaaaatggc gggcttcacc acgaccgagc agcagatgat cgccaccctg 900
   gtggtcgggc tgacctttat gttcgccacc tttattgccg tatttaccgt cgataaagcc 960
   ggacgtaaac cggcgctgaa aattggcttt agcgtgatgg cgctcggtac gctgatcctc 1020
   ggctactgcc tgatgcagtt tgatcagggc acggcatcga gcgggctttc ctggctctcc 1080
   gtcggtatga ccatgatgtg cattgccggt tatgcaatga gcgccgcgcc ggtggtgtgg 1140
   atcctgtgct ctgaaattca gccgctaaaa tgccgcgact ttggtatcac ctgttccacc 1200
   accaccaact gggtgtcgaa catgattatc ggtgcgacct tcctgacgct gctggatgcc 1260
   attggtgcag cgggaacatt ctggctctac acggtgctga acgtggcgtt tattggcgta 1320
   acgttctggc tgatcccaga aaccaaaggg gtgacgctgg agcacattga acgcaagctg 1380
   atggcggggg agaagttaaa aaacataggc gtg 1413
<210> 18
   <211> 471
   <212> PRT
   <213> Enterobacter cancerogenus
<400> 18
   Met Thr Ser Leu Asn Asp Ser Thr Leu Met Pro Ala Ala Leu Arg Asp
   1               5                   10                  15
   Thr Arg Arg Met Asn Gln Phe Val Ser Val Ala Ala Ala Val Ala Gly
               20                  25                  30
   Leu Leu Phe Gly Leu Asp Ile Gly Val Ile Ala Gly Ala Leu Pro Phe
           35                  40                  45
   Ile Thr Asp His Phe Thr Leu Ser His Arg Leu Gln Glu Trp Val Val
       50                  55                  60
   Ser Ser Met Met Leu Gly Ala Ala Ile Gly Ala Leu Phe Asn Gly Trp
   65                  70                  75                  80
   Leu Ser Phe Arg Leu Gly Arg Lys Tyr Ser Leu Met Val Gly Ala Ile
                   85                  90                  95
   Leu Phe Val Ala Gly Ser Leu Gly Ser Ala Phe Ala Thr Ser Val Glu
               100                 105                 110
   Val Leu Leu Leu Ser Arg Val Leu Leu Gly Val Ala Val Gly Ile Ala
           115                 120                 125
   Ser Tyr Thr Ala Pro Leu Tyr Leu Ser Glu Met Ala Ser Glu Asn Val
       130                 135                 140
   Arg Gly Lys Met Ile Ser Met Tyr Gln Leu Met Val Thr Leu Gly Ile
   145                 150                 155                 160
   Val Leu Ala Phe Leu Ser Asp Thr Trp Phe Ser Tyr Thr Gly Asn Trp
                   165                 170                 175
   Arg Ala Met Leu Gly Val Leu Ala Leu Pro Ala Leu Leu Leu Met Val
               180                 185                 190
   Leu Val Ile Phe Leu Pro Asn Ser Pro Arg Trp Leu Ala Gln Lys Gly
           195                 200                 205
   Arg His Val Glu Ala Glu Glu Val Leu Arg Met Leu Arg Asp Thr Ser
       210                 215                 220
   Glu Lys Ala Arg Glu Glu Leu Asn Glu Ile Arg Glu Ser Leu Lys Leu
   225                 230                 235                 240
   Lys Gln Gly Gly Trp Ala Leu Phe Lys Val Asn Arg Asn Val Arg Arg
                   245                 250                 255
   Ala Val Phe Leu Gly Met Leu Leu Gln Ala Met Gln Gln Phe Thr Gly
               260                 265                 270
   Met Asn Ile Ile Met Tyr Tyr Ala Pro Arg Ile Phe Lys Met Ala Gly
           275                 280                 285
   Phe Thr Thr Thr Glu Gln Gln Met Ile Ala Thr Leu Val Val Gly Leu
       290                 295                 300
   Thr Phe Met Phe Ala Thr Phe Ile Ala Val Phe Thr Val Asp Lys Ala
   305                 310                 315                 320
   Gly Arg Lys Pro Ala Leu Lys Ile Gly Phe Ser Val Met Ala Leu Gly
                   325                 330                 335
   Thr Leu Ile Leu Gly Tyr Cys Leu Met Gln Phe Asp Gln Gly Thr Ala
               340                 345                 350
   Ser Ser Gly Leu Ser Trp Leu Ser Val Gly Met Thr Met Met Cys Ile
           355                 360                 365
   Ala Gly Tyr Ala Met Ser Ala Ala Pro Val Val Trp Ile Leu Cys Ser
       370                 375                 380
   Glu Ile Gln Pro Leu Lys Cys Arg Asp Phe Gly Ile Thr Cys Ser Thr
   385 390 395 400
   Thr Thr Asn Trp Val Ser Asn Met Ile Ile Gly Ala Thr Phe Leu Thr
   405 410 415
   Leu Leu Asp Ala Ile Gly Ala Ala Gly Thr Phe Trp Leu Tyr Thr Val
   420 425 430
   Leu Asn Val Ala Phe Ile Gly Val Thr Phe Trp Leu Ile Pro Glu Thr
   435 440 445
   Lys Gly Val Thr Leu Glu His Ile Glu Arg Lys Leu Met Ala Gly Glu
   450 455 460
   Lys Leu Lys Asn Ile Gly Val
   465 470
<210> 19
   <211> 1392
   <212> DNA
   <213> Bacillus amyloliquefaciens
<400> 19
   atgaagaatc acccggcacc aattggctca aatgtacctg tcactcggca gcattccaag 60
   tggtttgtca ttctcatctc atgcgcggcc ggactgggag ggcttttgta cggttatgac 120
   acggcggtga tttccggcgc tatcggtttc ctgaaagatt tgtaccgctt aagtcctttt 180
   atggaagggc tcgtgatttc aagcattatg atcggcggtg ttttcggcgt cgggatttcc 240
   ggatttttga gtgaccgttt cggacggaga aagattttga tggcagcggc gctgttgttt 300
   gcggtgtcag cggttgtctc tgcgctttct caaagtgtgt cttccttagt gatcgccaga 360
   gtcatcggcg gtctgggaat cggcatgggc tcctcgcttt ctgtcacgta tattaccgaa 420
   gccgctccgc cggccatacg cggcagtctg tcttcactgt atcagctgtt tacgatatta 480
   gggatatccg gcacttattt tattaacctt gccgtccagc agtccggctc gtatgaatgg 540
   ggagtgcaca ccggctggcg gtggatgctc gcttacggca tgattccgtc cgtcatcttt 600
   tttatcgtgc tgcttatcgt gccggaaagt ccgcgctggc ttgcgaaagc ggggcgccgg 660
   aatgaagccc tcgccgtgct gacgcgcatt aacggcgagc agaccgcgaa agaagaaatc 720
   aaacaaatcg aaacgtcttt acaattagaa aaaatgggtt cattgtctca gctgtttaag 780
   ccggggctga gaaaagcgct tgtgatcggg attctgctgg ctttattcaa tcaggtcatc 840
   ggcatgaacg caattacgta ttacgggccg gaaattttca aaatgatggg cttcggacag 900
   aatgcggggt ttatcacgac atgcatcgtc ggtgtcgttg aagtgatttt caccattatc 960
   gcggttcttt tagtcgataa ggtaggccgg aaaaaactga tgggggtcgg atctgccttt 1020
   atggcgctgt tcatgatctt aatcggggca tccttttatt ttcagctggc gagcggtccg 1080
   gctttagtcg tcatcatatt gggattcgtc gccgctttct gcgtatcagt cgggccgatt 1140
   acatggatca tgatttcgga aatctttccg aaccacctcc gcgcacgcgc cgccggtatt 1200
   gcgacgatat tcttatgggg ggcgaactgg gcgatcggcc agttcgtgcc gatgatgatc 1260
   agcgggttag ggcttgcgta caccttctgg atattcgccg tcattaatat tctctgtttc 1320
   ttgtttgtcg tgacgatctg ccctgagacg aaaaataaat cattagaaga aatagaaaaa 1380
   ctctggataa aa 1392
<210> 20
   <211> 464
   <212> PRT
   <213> Bacillus amyloliquefaciens
<400> 20
   Met Lys Asn His Pro Ala Pro Ile Gly Ser Asn Val Pro Val Thr Arg
   1               5                   10                  15
   Gln His Ser Lys Trp Phe Val Ile Leu Ile Ser Cys Ala Ala Gly Leu
               20                  25                  30
   Gly Gly Leu Leu Tyr Gly Tyr Asp Thr Ala Val Ile Ser Gly Ala Ile
           35                  40                  45
   Gly Phe Leu Lys Asp Leu Tyr Arg Leu Ser Pro Phe Met Glu Gly Leu
       50                  55                  60
   Val Ile Ser Ser Ile Met Ile Gly Gly Val Phe Gly Val Gly Ile Ser
   65                  70                  75                  80
   Gly Phe Leu Ser Asp Arg Phe Gly Arg Arg Lys Ile Leu Met Ala Ala
                   85                  90                  95
   Ala Leu Leu Phe Ala Val Ser Ala Val Val Ser Ala Leu Ser Gln Ser
               100                 105                 110
   Val Ser Ser Leu Val Ile Ala Arg Val Ile Gly Gly Leu Gly Ile Gly
           115                 120                 125
   Met Gly Ser Ser Leu Ser Val Thr Tyr Ile Thr Glu Ala Ala Pro Pro
       130                 135                 140
   Ala Ile Arg Gly Ser Leu Ser Ser Leu Tyr Gln Leu Phe Thr Ile Leu
   145                 150                 155                 160
   Gly Ile Ser Gly Thr Tyr Phe Ile Asn Leu Ala Val Gln Gln Ser Gly
                   165                 170                 175
   Ser Tyr Glu Trp Gly Val His Thr Gly Trp Arg Trp Met Leu Ala Tyr
   180 185 190
   Gly Met Ile Pro Ser Val Ile Phe Phe Ile Val Leu Leu Ile Val Pro
   195 200 205
   Glu Ser Pro Arg Trp Leu Ala Lys Ala Gly Arg Arg Asn Glu Ala Leu
   210 215 220
   Ala Val Leu Thr Arg Ile Asn Gly Glu Gln Thr Ala Lys Glu Glu Ile
   225 230 235 240
   Lys Gln Ile Glu Thr Ser Leu Gln Leu Glu Lys Met Gly Ser Leu Ser
   245 250 255
   Gln Leu Phe Lys Pro Gly Leu Arg Lys Ala Leu Val Ile Gly Ile Leu
   260 265 270
   Leu Ala Leu Phe Asn Gln Val Ile Gly Met Asn Ala Ile Thr Tyr Tyr
   275 280 285
   Gly Pro Glu Ile Phe Lys Met Met Gly Phe Gly Gln Asn Ala Gly Phe
   290 295 300
   Ile Thr Thr Cys Ile Val Gly Val Val Glu Val Ile Phe Thr Ile Ile
   305 310 315 320
   Ala Val Leu Leu Val Asp Lys Val Gly Arg Lys Lys Leu Met Gly Val
   325 330 335
   Gly Ser Ala Phe Met Ala Leu Phe Met Ile Leu Ile Gly Ala Ser Phe
   340 345 350
   Tyr Phe Gln Leu Ala Ser Gly Pro Ala Leu Val Val Ile Ile Leu Gly
   355 360 365
   Phe Val Ala Ala Phe Cys Val Ser Val Gly Pro Ile Thr Trp Ile Met
   370 375 380
   Ile Ser Glu Ile Phe Pro Asn His Leu Arg Ala Arg Ala Ala Gly Ile
   385 390 395 400
   Ala Thr Ile Phe Leu Trp Gly Ala Asn Trp Ala Ile Gly Gln Phe Val
   405 410 415
   Pro Met Met Ile Ser Gly Leu Gly Leu Ala Tyr Thr Phe Trp Ile Phe
   420 425 430
   Ala Val Ile Asn Ile Leu Cys Phe Leu Phe Val Val Thr Ile Cys Pro
   435 440 445
   Glu Thr Lys Asn Lys Ser Leu Glu Glu Ile Glu Lys Leu Trp Ile Lys
   450 455 460
<210> 21
   <211> 1500
   <212> DNA
   <213> Escherichia coli
<400> 21
   atgacgattt ttgataatta tgaagtgtgg tttgtcattg gcagccagca tctgtatggc    60
   ccggaaaccc tgcgtcaggt cacccaacat gccgagcacg tcgttaatgc gctgaatacg   120
   gaagcgaaac tgccctgcaa actggtgttg aaaccgctgg gcaccacgcc ggatgaaatc   180
   accgctattt gccgcgacgc gaattacgac gatcgttgcg ctggtctggt ggtgtggctg   240
   cacaccttct ccccggccaa aatgtggatc aacggcctga ccatgctcaa caaaccgttg   300
   ctgcaattcc acacccagtt caacgcggcg ctgccgtggg acagtatcga tatggacttt   360
   atgaacctga accagactgc acatggcggt cgcgagttcg gcttcattgg cgcgcgtatg   420
   cgtcagcaac atgccgtggt taccggtcac tggcaggata aacaagccca tgagcgtatc   480
   ggctcctgga tgcgtcaggc ggtctctaaa caggataccc gtcatctgaa agtctgccga   540
   tttggcgata acatgcgtga agtggcggtc accgatggcg ataaagttgc cgcacagatc   600
   aagttcggtt tctccgtcaa tacctgggcg gttggcgatc tggtgcaggt ggtgaactcc   660
   atcagcgacg gcgatgttaa cgcgctggtc gatgagtacg aaagctgcta caccatgacg   720
   cctgccacac aaatccacgg caaaaaacga cagaacgtgc tggaagcggc gcgtattgag   780
   ctggggatga agcgtttcct ggaacaaggt ggcttccacg cgttcaccac cacctttgaa   840
   gatttgcacg gtctgaaaca gcttcctggt ctggccgtac agcgtctgat gcagcagggt   900
   tacggctttg cgggcgaagg cgactggaaa actgccgccc tgcttcgcat catgaaggtg   960
   atgtcaaccg gtctgcaggg cggcacctcc tttatggagg actacaccta tcacttcgag  1020
   aaaggtaatg acctggtgct cggctcccat atgctggaag tctgcccgtc gatcgccgca  1080
   gaagagaaac cgatcctcga cgttcagcat ctcggtattg gtggtaagga cgatcctgcc  1140
   cgcctgatct tcaataccca aaccggccca gcgattgtcg ccagcttgat tgatctcggc  1200
   gatcgttacc gtctactggt taactgcatc gacacggtga aaacaccgca ctccctgccg  1260
   aaactgccgg tggcgaatgc gctgtggaaa gcgcaaccgg atctgccaac tgcttccgaa  1320
   gcgtggatcc tcgctggtgg cgcgcaccat accgtcttca gccatgcact gaacctcaac  1380
   gatatgcgcc aattcgccga gatgcacgac attgaaatca cggtgattga taacgacaca  1440
   cgcctgccag cgtttaaaga cgcgctgcgc tggaacgaag tgtattacgg atttcgtcgc  1500
<210> 22
   <211> 500
   <212> PRT
   <213> Escherichia coli
<400> 22
   Met Thr Ile Phe Asp Asn Tyr Glu Val Trp Phe Val Ile Gly Ser Gln
   1               5                   10                  15
   His Leu Tyr Gly Pro Glu Thr Leu Arg Gln Val Thr Gln His Ala Glu
               20                  25                  30
   His Val Val Asn Ala Leu Asn Thr Glu Ala Lys Leu Pro Cys Lys Leu
           35                  40                  45
   Val Leu Lys Pro Leu Gly Thr Thr Pro Asp Glu Ile Thr Ala Ile Cys
       50                  55                  60
   Arg Asp Ala Asn Tyr Asp Asp Arg Cys Ala Gly Leu Val Val Trp Leu
   65                  70                  75                  80
   His Thr Phe Ser Pro Ala Lys Met Trp Ile Asn Gly Leu Thr Met Leu
                   85                  90                  95
   Asn Lys Pro Leu Leu Gln Phe His Thr Gln Phe Asn Ala Ala Leu Pro
               100                 105                 110
   Trp Asp Ser Ile Asp Met Asp Phe Met Asn Leu Asn Gln Thr Ala His
           115                 120                 125
   Gly Gly Arg Glu Phe Gly Phe Ile Gly Ala Arg Met Arg Gln Gln His
       130                 135                 140
   Ala Val Val Thr Gly His Trp Gln Asp Lys Gln Ala His Glu Arg Ile
   145                 150                 155                 160
   Gly Ser Trp Met Arg Gln Ala Val Ser Lys Gln Asp Thr Arg His Leu
                   165                 170                 175
   Lys Val Cys Arg Phe Gly Asp Asn Met Arg Glu Val Ala Val Thr Asp
               180                 185                 190
   Gly Asp Lys Val Ala Ala Gln Ile Lys Phe Gly Phe Ser Val Asn Thr
           195                 200                 205
   Trp Ala Val Gly Asp Leu Val Gln Val Val Asn Ser Ile Ser Asp Gly
       210                 215                 220
   Asp Val Asn Ala Leu Val Asp Glu Tyr Glu Ser Cys Tyr Thr Met Thr
   225                 230                 235                 240
   Pro Ala Thr Gln Ile His Gly Lys Lys Arg Gln Asn Val Leu Glu Ala
                   245                 250                 255
   Ala Arg Ile Glu Leu Gly Met Lys Arg Phe Leu Glu Gln Gly Gly Phe
               260                 265                 270
   His Ala Phe Thr Thr Thr Phe Glu Asp Leu His Gly Leu Lys Gln Leu
           275                 280                 285
   Pro Gly Leu Ala Val Gln Arg Leu Met Gln Gln Gly Tyr Gly Phe Ala
       290                 295                 300
   Gly Glu Gly Asp Trp Lys Thr Ala Ala Leu Leu Arg Ile Met Lys Val
   305                 310                 315                 320
   Met Ser Thr Gly Leu Gln Gly Gly Thr Ser Phe Met Glu Asp Tyr Thr
                   325                 330                 335
   Tyr His Phe Glu Lys Gly Asn Asp Leu Val Leu Gly Ser His Met Leu
               340                 345                 350
   Glu Val Cys Pro Ser Ile Ala Ala Glu Glu Lys Pro Ile Leu Asp Val
           355                 360                 365
   Gln His Leu Gly Ile Gly Gly Lys Asp Asp Pro Ala Arg Leu Ile Phe
       370                 375                 380
   Asn Thr Gln Thr Gly Pro Ala Ile Val Ala Ser Leu Ile Asp Leu Gly
   385                 390                 395                 400
   Asp Arg Tyr Arg Leu Leu Val Asn Cys Ile Asp Thr Val Lys Thr Pro
                   405                 410                 415
   His Ser Leu Pro Lys Leu Pro Val Ala Asn Ala Leu Trp Lys Ala Gln
               420                 425                 430
   Pro Asp Leu Pro Thr Ala Ser Glu Ala Trp Ile Leu Ala Gly Gly Ala
           435                 440                 445
   His His Thr Val Phe Ser His Ala Leu Asn Leu Asn Asp Met Arg Gln
       450                 455                 460
   Phe Ala Glu Met His Asp Ile Glu Ile Thr Val Ile Asp Asn Asp Thr
   465                 470                 475                 480
   Arg Leu Pro Ala Phe Lys Asp Ala Leu Arg Trp Asn Glu Val Tyr Tyr
                   485                 490                 495
   Gly Phe Arg Arg
   500
<210> 23
   <211> 1698
   <212> DNA
   <213> Escherichia coli
<400> 23
   atggcgattg caattggcct cgattttggc agtgattctg tgcgagcttt ggcggtggac     60
   tgcgctaccg gtgaagagat cgccaccagc gtagagtggt atccccgttg gcagaaaggg    120
   caattttgtg atgccccgaa taaccagttc cgtcatcatc cgcgtgacta cattgagtca    180
   atggaagcgg cactgaaaac cgtgcttgca gagcttagcg tcgaacagcg cgcagctgtg    240
   gtcgggattg gcgttgacag taccggctcg acgcccgcac cgattgatgc cgacggaaac    300
   gtgctggcgc tgcgcccgga gtttgccgaa aacccgaacg cgatgttcgt attgtggaaa    360
   gaccacactg cggttgaaga agcggaagag attacccgtt tgtgccacgc gccgggcaac    420
   gttgactact cccgctacat tggtggtatt tattccagcg aatggttctg ggcaaaaatc    480
   ctgcatgtga ctcgccagga cagcgccgtg gcgcaatctg ccgcatcgtg gattgagctg    540
   tgcgactggg tgccagctct gctttccggt accacccgcc cgcaggatat tcgtcgcgga    600
   cgttgcagcg ccgggcataa atctctgtgg cacgaaagct ggggcggcct gccgccagcc    660
   agtttctttg atgagctgga cccgatcctc aatcgccatt tgccttcccc gctgttcact    720
   gacacttgga ctgccgatat tccggtgggc accttatgcc cggaatgggc gcagcgtctc    780
   ggcctgcctg aaagcgtggt gatttccggc ggcgcgtttg actgccatat gggcgcagtt    840
   ggcgcaggcg cacagcctaa cgcactggta aaagttatcg gtacttccac ctgcgacatt    900
   ctgattgccg acaaacagag cgttggcgag cgggcagtta aaggtatttg cggtcaggtt    960
   gatggcagcg tggtgcctgg atttatcggt ctggaagcag gccaatcggc gtttggtgat   1020
   atctacgcct ggtttggtcg cgtactcggc tggccgctgg aacagcttgc cgcccagcat   1080
   ccggaactga aaacgcaaat caacgccagc cagaaacaac tgcttccggc gctgaccgaa   1140
   gcatgggcca aaaatccgtc tctggatcac ctgccggtgg tgctcgactg gtttaacggc   1200
   cgccgcacac cgaacgctaa ccaacgcctg aaaggggtga ttaccgatct taacctcgct   1260
   accgacgctc cgctgctgtt cggcggtttg attgctgcca ccgcctttgg cgcacgcgca   1320
   atcatggagt gctttaccga tcaggggatc gccgttaata acgtgatggc actgggcggc   1380
   atcgcgcgga aaaaccaggt cattatgcag gcctgctgcg acgtgctgaa tcgcccgctg   1440
   caaattgttg cctctgacca gtgctgtgcg ctcggtgcgg cgatttttgc tgccgtcgcc   1500
   gcgaaagtgc acgcagacat cccatcagct cagcaaaaaa tggccagtgc ggtagagaaa   1560
   accctgcaac cgtgcagcga gcaggcacaa cgctttgaac agctttatcg ccgctatcag   1620
   caatgggcga tgagcgccga acaacactat cttccaactt ccgccccggc acaggctgcc   1680
   caggccgttg cgactcta                                                  1698
<210> 24
   <211> 566
   <212> PRT
   <213> Escherichia coli
<400> 24
   Met Ala Ile Ala Ile Gly Leu Asp Phe Gly Ser Asp Ser Val Arg Ala
   1               5                   10                  15
   Leu Ala Val Asp Cys Ala Thr Gly Glu Glu Ile Ala Thr Ser Val Glu
               20                  25                  30
   Trp Tyr Pro Arg Trp Gln Lys Gly Gln Phe Cys Asp Ala Pro Asn Asn
           35                  40                  45
   Gln Phe Arg His His Pro Arg Asp Tyr Ile Glu Ser Met Glu Ala Ala
       50                  55                  60
   Leu Lys Thr Val Leu Ala Glu Leu Ser Val Glu Gln Arg Ala Ala Val
   65                  70                  75                  80
   Val Gly Ile Gly Val Asp Ser Thr Gly Ser Thr Pro Ala Pro Ile Asp
                   85                  90                  95
   Ala Asp Gly Asn Val Leu Ala Leu Arg Pro Glu Phe Ala Glu Asn Pro
               100                 105                 110
   Asn Ala Met Phe Val Leu Trp Lys Asp His Thr Ala Val Glu Glu Ala
           115                 120                 125
   Glu Glu Ile Thr Arg Leu Cys His Ala Pro Gly Asn Val Asp Tyr Ser
       130                 135                 140
   Arg Tyr Ile Gly Gly Ile Tyr Ser Ser Glu Trp Phe Trp Ala Lys Ile
   145                 150                 155                 160
   Leu His Val Thr Arg Gln Asp Ser Ala Val Ala Gln Ser Ala Ala Ser
                   165                 170                 175
   Trp Ile Glu Leu Cys Asp Trp Val Pro Ala Leu Leu Ser Gly Thr Thr
               180                 185                 190
   Arg Pro Gln Asp Ile Arg Arg Gly Arg Cys Ser Ala Gly His Lys Ser
           195                 200                 205
   Leu Trp His Glu Ser Trp Gly Gly Leu Pro Pro Ala Ser Phe Phe Asp
   210 215 220
   Glu Leu Asp Pro Ile Leu Asn Arg His Leu Pro Ser Pro Leu Phe Thr
   225 230 235 240
   Asp Thr Trp Thr Ala Asp Ile Pro Val Gly Thr Leu Cys Pro Glu Trp
   245 250 255
   Ala Gln Arg Leu Gly Leu Pro Glu Ser Val Val Ile Ser Gly Gly Ala
   260 265 270
   Phe Asp Cys His Met Gly Ala Val Gly Ala Gly Ala Gln Pro Asn Ala
   275 280 285
   Leu Val Lys Val Ile Gly Thr Ser Thr Cys Asp Ile Leu Ile Ala Asp
   290 295 300
   Lys Gln Ser Val Gly Glu Arg Ala Val Lys Gly Ile Cys Gly Gln Val
   305 310 315 320
   Asp Gly Ser Val Val Pro Gly Phe Ile Gly Leu Glu Ala Gly Gln Ser
   325 330 335
   Ala Phe Gly Asp Ile Tyr Ala Trp Phe Gly Arg Val Leu Gly Trp Pro
   340 345 350
   Leu Glu Gln Leu Ala Ala Gln His Pro Glu Leu Lys Thr Gln Ile Asn
   355 360 365
   Ala Ser Gln Lys Gln Leu Leu Pro Ala Leu Thr Glu Ala Trp Ala Lys
   370 375 380
   Asn Pro Ser Leu Asp His Leu Pro Val Val Leu Asp Trp Phe Asn Gly
   385 390 395 400
   Arg Arg Thr Pro Asn Ala Asn Gln Arg Leu Lys Gly Val Ile Thr Asp
   405 410 415
   Leu Asn Leu Ala Thr Asp Ala Pro Leu Leu Phe Gly Gly Leu Ile Ala
   420 425 430
   Ala Thr Ala Phe Gly Ala Arg Ala Ile Met Glu Cys Phe Thr Asp Gln
   435 440 445
   Gly Ile Ala Val Asn Asn Val Met Ala Leu Gly Gly Ile Ala Arg Lys
   450 455 460
   Asn Gln Val Ile Met Gln Ala Cys Cys Asp Val Leu Asn Arg Pro Leu
   465 470 475 480
   Gln Ile Val Ala Ser Asp Gln Cys Cys Ala Leu Gly Ala Ala Ile Phe
   485 490 495
   Ala Ala Val Ala Ala Lys Val His Ala Asp Ile Pro Ser Ala Gln Gln
   500 505 510
   Lys Met Ala Ser Ala Val Glu Lys Thr Leu Gln Pro Cys Ser Glu Gln
   515 520 525
   Ala Gln Arg Phe Glu Gln Leu Tyr Arg Arg Tyr Gln Gln Trp Ala Met
   530 535 540
   Ser Ala Glu Gln His Tyr Leu Pro Thr Ser Ala Pro Ala Gln Ala Ala
   545 550 555 560
   Gln Ala Val Ala Thr Leu
   565
<210> 25
   <211> 693
   <212> DNA
   <213> Escherichia coli
<400> 25
   atgttagaag atctcaaacg ccaggtatta gaagccaacc tggcgctgcc aaaacacaac 60
   ctggtcacgc tcacatgggg caacgtcagc gccgttgatc gcgagcgcgg cgtctttgtg 120
   atcaaacctt ccggcgtcga ttacagcgtc atgaccgctg acgatatggt cgtggttagc 180
   atcgaaaccg gtgaagtggt tgaaggtacg aaaaagccct cctccgacac gccaactcac 240
   cggctgctct atcaggcatt cccctccatt ggcggcattg tgcatacgca ctcgcgccac 300
   gccaccatct gggcgcaggc gggtcagtcg attccagcaa ccggcaccac ccacgccgac 360
   tatttctacg gcaccattcc ctgtacccgc aaaatgaccg acgcagaaat caacggcgaa 420
   tatgagtggg aaaccggtaa cgtcatcgta gaaacctttg aaaaacaggg tatcgatgca 480
   gcgcaaatgc ccggcgttct ggtccattcc cacggcccgt ttgcatgggg caaaaatgcc 540
   gaagatgcgg tgcataacgc catcgtgctg gaagaggtcg cttatatggg gatattctgc 600
   cgtcagttag cgccgcagtt accggatatg cagcaaacgc tgctggataa acactatctg 660
   cgtaagcatg gcgcgaaggc atattacggg cag 693
<210> 26
   <211> 231
   <212> PRT
   <213> Escherichia coli
<400> 26
   Met Leu Glu Asp Leu Lys Arg Gln Val Leu Glu Ala Asn Leu Ala Leu
   1               5                   10                  15
   Pro Lys His Asn Leu Val Thr Leu Thr Trp Gly Asn Val Ser Ala Val
               20                  25                  30
   Asp Arg Glu Arg Gly Val Phe Val Ile Lys Pro Ser Gly Val Asp Tyr
           35                  40                  45
   Ser Val Met Thr Ala Asp Asp Met Val Val Val Ser Ile Glu Thr Gly
       50                  55                  60
   Glu Val Val Glu Gly Thr Lys Lys Pro Ser Ser Asp Thr Pro Thr His
   65                  70                  75                  80
   Arg Leu Leu Tyr Gln Ala Phe Pro Ser Ile Gly Gly Ile Val His Thr
                   85                  90                  95
   His Ser Arg His Ala Thr Ile Trp Ala Gln Ala Gly Gln Ser Ile Pro
               100                 105                 110
   Ala Thr Gly Thr Thr His Ala Asp Tyr Phe Tyr Gly Thr Ile Pro Cys
           115                 120                 125
   Thr Arg Lys Met Thr Asp Ala Glu Ile Asn Gly Glu Tyr Glu Trp Glu
       130                 135                 140
   Thr Gly Asn Val Ile Val Glu Thr Phe Glu Lys Gln Gly Ile Asp Ala
   145                 150                 155                 160
   Ala Gln Met Pro Gly Val Leu Val His Ser His Gly Pro Phe Ala Trp
                   165                 170                 175
   Gly Lys Asn Ala Glu Asp Ala Val His Asn Ala Ile Val Leu Glu Glu
               180                 185                 190
   Val Ala Tyr Met Gly Ile Phe Cys Arg Gln Leu Ala Pro Gln Leu Pro
           195                 200                 205
   Asp Met Gln Gln Thr Leu Leu Asp Lys His Tyr Leu Arg Lys His Gly
       210                 215                 220
   Ala Lys Ala Tyr Tyr Gly Gln
   225                 230
<210> 27
   <211> 3226
   <212> DNA
   <213> artificial sequence
<220>
   <223> araA-araB PCR fragment
<400> 27
   aaccatggcg attgcaattg gcctcgattt tggcagtgat tctgtgcgag ctttggcggt 60
   ggactgcgct accggtgaag agatcgccac cagcgtagag tggtatcccc gttggcagaa 120
   agggcaattt tgtgatgccc cgaataacca gttccgtcat catccgcgtg actacattga 180
   gtcaatggaa gcggcactga aaaccgtgct tgcagagctt agcgtcgaac agcgcgcagc 240
   tgtggtcggg attggcgttg acagtaccgg ctcgacgccc gcaccgattg atgccgacgg 300
   aaacgtgctg gcgctgcgcc cggagtttgc cgaaaacccg aacgcgatgt tcgtattgtg 360
   gaaagaccac actgcggttg aagaagcgga agagattacc cgtttgtgcc acgcgccggg 420
   caacgttgac tactcccgct acattggtgg tatttattcc agcgaatggt tctgggcaaa 480
   aatcctgcat gtgactcgcc aggacagcgc cgtggcgcaa tctgccgcat cgtggattga 540
   gctgtgcgac tgggtgccag ctctgctttc cggtaccacc cgcccgcagg atattcgtcg 600
   cggacgttgc agcgccgggc ataaatctct gtggcacgaa agctggggcg gcctgccgcc 660
   agccagtttc tttgatgagc tggacccgat cctcaatcgc catttgcctt ccccgctgtt 720
   cactgacact tggactgccg atattccggt gggcacctta tgcccggaat gggcgcagcg 780
   tctcggcctg cctgaaagcg tggtgatttc cggcggcgcg tttgactgcc atatgggcgc 840
   agttggcgca ggcgcacagc ctaacgcact ggtaaaagtt atcggtactt ccacctgcga 900
   cattctgatt gccgacaaac agagcgttgg cgagcgggca gttaaaggta tttgcggtca 960
   ggttgatggc agcgtggtgc ctggatttat cggtctggaa gcaggccaat cggcgtttgg 1020
   tgatatctac gcctggtttg gtcgcgtact cggctggccg ctggaacagc ttgccgccca 1080
   gcatccggaa ctgaaaacgc aaatcaacgc cagccagaaa caactgcttc cggcgctgac 1140
   cgaagcatgg gccaaaaatc cgtctctgga tcacctgccg gtggtgctcg actggtttaa 1200
   cggccgccgc acaccgaacg ctaaccaacg cctgaaaggg gtgattaccg atcttaacct 1260
   cgctaccgac gctccgctgc tgttcggcgg tttgattgct gccaccgcct ttggcgcacg 1320
   cgcaatcatg gagtgcttta ccgatcaggg gatcgccgtt aataacgtga tggcactggg 1380
   cggcatcgcg cggaaaaacc aggtcattat gcaggcctgc tgcgacgtgc tgaatcgccc 1440
   gctgcaaatt gttgcctctg accagtgctg tgcgctcggt gcggcgattt ttgctgccgt 1500
   cgccgcgaaa gtgcacgcag acatcccatc agctcagcaa aaaatggcca gtgcggtaga 1560
   gaaaaccctg caaccgtgca gcgagcaggc acaacgcttt gaacagcttt atcgccgcta 1620
   tcagcaatgg gcgatgagcg ccgaacaaca ctatcttcca acttccgccc cggcacaggc 1680
   tgcccaggcc gttgcgactc tataaggaca cgataatgac gatttttgat aattatgaag 1740
   tgtggtttgt cattggcagc cagcatctgt atggcccgga aaccctgcgt caggtcaccc 1800
   aacatgccga gcacgtcgtt aatgcgctga atacggaagc gaaactgccc tgcaaactgg 1860
   tgttgaaacc gctgggcacc acgccggatg aaatcaccgc tatttgccgc gacgcgaatt 1920
   acgacgatcg ttgcgctggt ctggtggtgt ggctgcacac cttctccccg gccaaaatgt 1980
   ggatcaacgg cctgaccatg ctcaacaaac cgttgctgca attccacacc cagttcaacg 2040
   cggcgctgcc gtgggacagt atcgatatgg actttatgaa cctgaaccag actgcacatg 2100
   gcggtcgcga gttcggcttc attggcgcgc gtatgcgtca gcaacatgcc gtggttaccg 2160
   gtcactggca ggataaacaa gcccatgagc gtatcggctc ctggatgcgt caggcggtct 2220
   ctaaacagga tacccgtcat ctgaaagtct gccgatttgg cgataacatg cgtgaagtgg 2280
   cggtcaccga tggcgataaa gttgccgcac agatcaagtt cggtttctcc gtcaatacct 2340
   gggcggttgg cgatctggtg caggtggtga actccatcag cgacggcgat gttaacgcgc 2400
   tggtcgatga gtacgaaagc tgctacacca tgacgcctgc cacacaaatc cacggcaaaa 2460
   aacgacagaa cgtgctggaa gcggcgcgta ttgagctggg gatgaagcgt ttcctggaac 2520
   aaggtggctt ccacgcgttc accaccacct ttgaagattt gcacggtctg aaacagcttc 2580
   ctggtctggc cgtacagcgt ctgatgcagc agggttacgg ctttgcgggc gaaggcgact 2640
   ggaaaactgc cgccctgctt cgcatcatga aggtgatgtc aaccggtctg cagggcggca 2700
   cctcctttat ggaggactac acctatcact tcgagaaagg taatgacctg gtgctcggct 2760
   cccatatgct ggaagtctgc ccgtcgatcg ccgcagaaga gaaaccgatc ctcgacgttc 2820
   agcatctcgg tattggtggt aaggacgatc ctgcccgcct gatcttcaat acccaaaccg 2880
   gcccagcgat tgtcgccagc ttgattgatc tcggcgatcg ttaccgtcta ctggttaact 2940
   gcatcgacac ggtgaaaaca ccgcactccc tgccgaaact gccggtggcg aatgcgctgt 3000
   ggaaagcgca accggatctg ccaactgctt ccgaagcgtg gatcctcgct ggtggcgcgc 3060
   accataccgt cttcagccat gcactgaacc tcaacgatat gcgccaattc gccgagatgc 3120
   acgacattga aatcacggtg attgataacg acacacgcct gccagcgttt aaagacgcgc 3180
   tgcgctggaa cgaagtgtat tacggatttc gtcgctaagt ctagag 3226
<210> 28
   <211> 25
   <212> DNA
   <213> artificial sequence
<220>
   <223> primer
<400> 28
   aaccatggcg attgcaattg gcctc 25
<210> 29
   <211> 32
   <212> DNA
   <213> artificial sequence
<220>
   <223> primer
<400> 29
   ctctagactt agcgacgaaa tccgtaatac ac 32
<210> 30
   <211> 889
   <212> DNA
   <213> artificial sequence
<220>
   <223> araD PCR fragment
<400> 30
   gtctagagaa ggagtcaaca tgttagaaga tctcaaacgc caggtattag aagccaacct 60
   ggcgctgcca aaacacaacc tggtcacgct cacatggggc aacgtcagcg ccgttgatcg 120
   cgagcgcggc gtctttgtga tcaaaccttc cggcgtcgat tacagcgtca tgaccgctga 180
   cgatatggtc gtggttagca tcgaaaccgg tgaagtggtt gaaggtacga aaaagccctc 240
   ctccgacacg ccaactcacc ggctgctcta tcaggcattc ccctccattg gcggcattgt 300
   gcatacgcac tcgcgccacg ccaccatctg ggcgcaggcg ggtcagtcga ttccagcaac 360
   cggcaccacc cacgccgact atttctacgg caccattccc tgtacccgca aaatgaccga 420
   cgcagaaatc aacggcgaat atgagtggga aaccggtaac gtcatcgtag aaacctttga 480
   aaaacagggt atcgatgcag cgcaaatgcc cggcgttctg gtccattccc acggcccgtt 540
   tgcatggggc aaaaatgccg aagatgcggt gcataacgcc atcgtgctgg aagaggtcgc 600
   ttatatgggg atattctgcc gtcagttagc gccgcagtta ccggatatgc agcaaacgct 660
   gctggataaa cactatctgc gtaagcatgg cgcgaaggca tattacgggc agtaatgact 720
   gtataaaacc acagccaatc aaacgaaacc aggctatact caagcctggt tttttgatgg 780
   attttcagcg tggcgcaggc aggttttatc ttaacccgac actggcggga caccccgcaa 840
   gggacagaag tctccttctg gctggcgacg gacaacgggc caagcttgg 889
<210> 31
   <211> 32
   <212> DNA
   <213> artificial sequence
<220>
   <223> primer
<400> 31
   gtctagagaa ggagtcaaca tgttagaaga tc 32
<210> 32
   <211> 28
   <212> DNA
   <213> artificial sequence
<220>
   <223> primer
<400> 32
   ccaagcttgg cccgttgtcc gtcgccag 28
<210> 33
   <211> 303
   <212> DNA
   <213> Zymomonas mobilis
<400> 33
   tcgatcaaca acccgaatcc tatcgtaatg atgttttgcc cgatcagcct caatcgacaa 60
   ttttacgcgt ttcgatcgaa gcagggacga caattggctg ggaacggtat actggaataa 120
   atggtcttcg ttatggtatt gatgtttttg gtgcatcggc cccggcgaat gatctatatg 180
   ctcatttcgg cttgaccgca gtcggcatca cgaacaaggt gttggccgcg atcgccggta 240
   agtcggcacg ttaaaaaata gctatggaat ataatagcta cttaataagt taggagaata 300
   aac 303
<210> 34
   <211> 34
   <212> DNA
   <213> artificial sequence
<220>
   <223> primer
<400> 34
   gggagctcac tagttcgatc aacaacccga atcc 34
<210> 35
   <211> 29
   <212> DNA
   <213> artificial sequence
<220>
   <223> primer
<400> 35
   agccatggtt attctcctaa cttattaag 29
<210> 36
   <211> 323
   <212> DNA
   <213> artificial sequence
<220>
   <223> Pgap PCR fragment
<400> 36
   gggagctcac tagttcgatc aacaacccga atcctatcgt aatgatgttt tgcccgatca 60
   gcctcaatcg acaattttac gcgtttcgat cgaagcaggg acgacaattg gctgggaacg 120
   gtatactgga ataaatggtc ttcgttatgg tattgatgtt tttggtgcat cggccccggc 180
   gaatgatcta tatgctcatt tcggcttgac cgcagtcggc atcacgaaca aggtgttggc 240
   cgcgatcgcc ggtaagtcgg cacgttaaaa aatagctatg gaatataata gctacttaat 300
   aagttaggag aataaccatg gct 323
<210> 37
   <211> 35
   <212> DNA
   <213> artificial sequence
<220>
   <223> primer
<400> 37
   ctactcattt atcgatggag cacaggatga cgcct 35
<210> 38
   <211> 34
   <212> DNA
   <213> artificial sequence
<220>
   <223> primer
<400> 38
   catcttacta cgcgttggca ggtcagcaag tgcc 34
<210> 39
   <211> 36
   <212> DNA
   <213> artificial sequence
<220>
   <223> mutagenesis oligo
<400> 39
   aagttaggag aataaacatg gcgattgcaa ttggcc 36
<210> 40
   <211> 36
   <212> DNA
   <213> artificial sequence
<220>
   <223> mutagenesis oligo
<400> 40
   ggccaattgc aatcgccatg tttattctcc taactt 36
<210> 41
   <211> 9884
   <212> DNA
   <213> artificial sequence
<220>
   <223> constructed plasmid
<400> 41
   ctagttcgat caacaacccg aatcctatcg taatgatgtt ttgcccgatc agcctcaatc     60
   gacaatttta cgcgtttcga tcgaagcagg gacgacaatt ggctgggaac ggtatactgg    120
   aataaatggt cttcgttatg gtattgatgt ttttggtgca tcggccccgg cgaatgatct 180
   atatgctcat ttcggcttga ccgcagtcgg catcacgaac aaggtgttgg ccgcgatcgc 240
   cggtaagtcg gcacgttaaa aaatagctat ggaatataat agctacttaa taagttagga 300
   gaataaacat ggcgattgca attggcctcg attttggcag tgattctgtg cgagctttgg 360
   cggtggactg cgctaccggt gaagagatcg ccaccagcgt agagtggtat ccccgttggc 420
   agaaagggca attttgtgat gccccgaata accagttccg tcatcatccg cgtgactaca 480
   ttgagtcaat ggaagcggca ctgaaaaccg tgcttgcaga gcttagcgtc gaacagcgcg 540
   cagctgtggt cgggattggc gttgacagta ccggctcgac gcccgcaccg attgatgccg 600
   acggaaacgt gctggcgctg cgcccggagt ttgccgaaaa cccgaacgcg atgttcgtat 660
   tgtggaaaga ccacactgcg gttgaagaag cggaagagat tacccgtttg tgccacgcgc 720
   cgggcaacgt tgactactcc cgctacattg gtggtattta ttccagcgaa tggttctggg 780
   caaaaatcct gcatgtgact cgccaggaca gcgccgtggc gcaatctgcc gcatcgtgga 840
   ttgagctgtg cgactgggtg ccagctctgc tttccggtac cacccgcccg caggatattc 900
   gtcgcggacg ttgcagcgcc gggcataaat ctctgtggca cgaaagctgg ggcggcctgc 960
   cgccagccag tttctttgat gagctggacc cgatcctcaa tcgccatttg ccttccccgc 1020
   tgttcactga cacttggact gccgatattc cggtgggcac cttatgcccg gaatgggcgc 1080
   agcgtctcgg cctgcctgaa agcgtggtga tttccggcgg cgcgtttgac tgccatatgg 1140
   gcgcagttgg cgcaggcgca cagcctaacg cactggtaaa agttatcggt acttccacct 1200
   gcgacattct gattgccgac aaacagagcg ttggcgagcg ggcagttaaa ggtatttgcg 1260
   gtcaggttga tggcagcgtg gtgcctggat ttatcggtct ggaagcaggc caatcggcgt 1320
   ttggtgatat ctacgcctgg tttggtcgcg tactcggctg gccgctggaa cagcttgccg 1380
   cccagcatcc ggaactgaaa acgcaaatca acgccagcca gaaacaactg cttccggcgc 1440
   tgaccgaagc atgggccaaa aatccgtctc tggatcacct gccggtggtg ctcgactggt 1500
   ttaacggccg ccgcacaccg aacgctaacc aacgcctgaa aggggtgatt accgatctta 1560
   acctcgctac cgacgctccg ctgctgttcg gcggtttgat tgctgccacc gcctttggcg 1620
   cacgcgcaat catggagtgc tttaccgatc aggggatcgc cgttaataac gtgatggcac 1680
   tgggcggcat cgcgcggaaa aaccaggtca ttatgcaggc ctgctgcgac gtgctgaatc 1740
   gcccgctgca aattgttgcc tctgaccagt gctgtgcgct cggtgcggcg atttttgctg 1800
   ccgtcgccgc gaaagtgcac gcagacatcc catcagctca gcaaaaaatg gccagtgcgg 1860
   tagagaaaac cctgcaaccg tgcagcgagc aggcacaacg ctttgaacag ctttatcgcc 1920
   gctatcagca atgggcgatg agcgccgaac aacactatct tccaacttcc gccccggcac 1980
   aggctgccca ggccgttgcg actctataag gacacgataa tgacgatttt tgataattat 2040
   gaagtgtggt ttgtcattgg cagccagcat ctgtatggcc cggaaaccct gcgtcaggtc 2100
   acccaacatg ccgagcacgt cgttaatgcg ctgaatacgg aagcgaaact gccctgcaaa 2160
   ctggtgttga aaccgctggg caccacgccg gatgaaatca ccgctatttg ccgcgacgcg 2220
   aattacgacg atcgttgcgc tggtctggtg gtgtggctgc acaccttctc cccggccaaa 2280
   atgtggatca acggcctgac catgctcaac aaaccgttgc tgcaattcca cacccagttc 2340
   aacgcggcgc tgccgtggga cagtatcgat atggacttta tgaacctgaa ccagactgca 2400
   catggcggtc gcgagttcgg cttcattggc gcgcgtatgc gtcagcaaca tgccgtggtt 2460
   accggtcact ggcaggataa acaagcccat gagcgtatcg gctcctggat gcgtcaggcg 2520
   gtctctaaac aggatacccg tcatctgaaa gtctgccgat ttggcgataa catgcgtgaa 2580
   gtggcggtca ccgatggcga taaagttgcc gcacagatca agttcggttt ctccgtcaat 2640
   acctgggcgg ttggcgatct ggtgcaggtg gtgaactcca tcagcgacgg cgatgttaac 2700
   gcgctggtcg atgagtacga aagctgctac accatgacgc ctgccacaca aatccacggc 2760
   aaaaaacgac agaacgtgct ggaagcggcg cgtattgagc tggggatgaa gcgtttcctg 2820
   gaacaaggtg gcttccacgc gttcaccacc acctttgaag atttgcacgg tctgaaacag 2880
   cttcctggtc tggccgtaca gcgtctgatg cagcagggtt acggctttgc gggcgaaggc 2940
   gactggaaaa ctgccgccct gcttcgcatc atgaaggtga tgtcaaccgg tctgcagggc 3000
   ggcacctcct ttatggagga ctacacctat cacttcgaga aaggtaatga cctggtgctc 3060
   ggctcccata tgctggaagt ctgcccgtcg atcgccgcag aagagaaacc gatcctcgac 3120
   gttcagcatc tcggtattgg tggtaaggac gatcctgccc gcctgatctt caatacccaa 3180
   accggcccag cgattgtcgc cagcttgatt gatctcggcg atcgttaccg tctactggtt 3240
   aactgcatcg acacggtgaa aacaccgcac tccctgccga aactgccggt ggcgaatgcg 3300
   ctgtggaaag cgcaaccgga tctgccaact gcttccgaag cgtggatcct cgctggtggc 3360
   gcgcaccata ccgtcttcag ccatgcactg aacctcaacg atatgcgcca attcgccgag 3420
   atgcacgaca ttgaaatcac ggtgattgat aacgacacac gcctgccagc gtttaaagac 3480
   gcgctgcgct ggaacgaagt gtattacgga tttcgtcgct aagtctagag aaggagtcaa 3540
   catgttagaa gatctcaaac gccaggtatt agaagccaac ctggcgctgc caaaacacaa 3600
   cctggtcacg ctcacatggg gcaacgtcag cgccgttgat cgcgagcgcg gcgtctttgt 3660
   gatcaaacct tccggcgtcg attacagcgt catgaccgct gacgatatgg tcgtggttag 3720
   catcgaaacc ggtgaagtgg ttgaaggtac gaaaaagccc tcctccgaca cgccaactca 3780
   ccggctgctc tatcaggcat tcccctccat tggcggcatt gtgcatacgc actcgcgcca 3840
   cgccaccatc tgggcgcagg cgggtcagtc gattccagca accggcacca cccacgccga 3900
   ctatttctac ggcaccattc cctgtacccg caaaatgacc gacgcagaaa tcaacggcga 3960
   atatgagtgg gaaaccggta acgtcatcgt agaaaccttt gaaaaacagg gtatcgatgc 4020
   agcgcaaatg cccggcgttc tggtccattc ccacggcccg tttgcatggg gcaaaaatgc 4080
   cgaagatgcg gtgcataacg ccatcgtgct ggaagaggtc gcttatatgg ggatattctg 4140
   ccgtcagtta gcgccgcagt taccggatat gcagcaaacg ctgctggata aacactatct 4200
   gcgtaagcat ggcgcgaagg catattacgg gcagtaatga ctgtataaaa ccacagccaa 4260
   tcaaacgaaa ccaggctata ctcaagcctg gttttttgat ggattttcag cgtggcgcag 4320
   gcaggtttta tcttaacccg acactggcgg gacaccccgc aagggacaga agtctccttc 4380
   tggctggcga cggacaacgg gccaagcttg gaagggcgaa ttctgcagat atccatcaca 4440
   ctggcggccg ctaattccgg atgagcattc atcaggcggg caagaatgtg aataaaggcc 4500
   ggataaaact tgtgcttatt tttctttacg gtctttaaaa aggccgtaat atccagctga 4560
   acggtctggt tataggtaca ttgagcaact gactgaaatg cctcaaaatg ttctttacga 4620
   tgccattggg atatatcaac ggtggtatat ccagtgattt ttttctccat tttagcttcc 4680
   ttagctcctg aaaatctcga taactcaaaa aatacgcccg gtagtgatct tatttcatta 4740
   tggtgaaagt tggaacctct tacgtgccga tcaacgtctc attttcgcca aaagttggcc 4800
   cagggcttcc cggtatcaac agggacacca ggatttattt attctgcgaa gtgatcttcc 4860
   gtcacaggta tttattcggc gcaaagtgcg tcgggtgatg ctgccaactt actgatttag 4920
   tgtatgatgg tgtttttgag gtgctccagt ggcttctgtt tctatcagct gtccctcctg 4980
   ttcagctact gacggggtgg tgcgtaacgg caaaagcacc gccggacatc agcgctagcg 5040
   gagtgtatac tggcttacta tgttggcact gatgagggtg tcagtgaagt gcttcatgtg 5100
   gcaggagaaa aaaggctgca ccggtgcgtc agcagaatat gtgatacagg atatattccg 5160
   cttcctcgct cactgactcg ctacgctcgg tcgttcgact gcggcgagcg gaaatggctt 5220
   acgaacgggg cggagatttc ctggaagatg ccaggaagat acttaacagg gaagtgagag 5280
   ggccgcggca aagccgtttt tccataggct ccgcccccct gacaagcatc acgaaatctg 5340
   acgctcaaat cagtggtggc gaaacccgac aggactataa agataccagg cgtttccccc 5400
   tggcggctcc ctcgtgcgct ctcctgttcc tgcctttcgg tttaccggtg tcattccgct 5460
   gttatggccg cgtttgtctc attccacgcc tgacactcag ttccgggtag gcagttcgct 5520
   ccaagctgga ctgtatgcac gaaccccccg ttcagtccga ccgctgcgcc ttatccggta 5580
   actatcgtct tgagtccaac ccggaaagac atgcaaaagc accactggca gcagccactg 5640
   gtaattgatt tagaggagtt agtcttgaag tcatgcgccg gttaaggcta aactgaaagg 5700
   acaagttttg gtgactgcgc tcctccaagc cagttacctc ggttcaaaga gttggtagct 5760
   cagagaacct tcgaaaaacc gccctgcaag gcggtttttt cgttttcaga gcaagagatt 5820
   acgcgcagac caaaacgatc tcaagaagat catcttatta atcagataaa atatttctag 5880
   atttcagtgc aatttatctc ttcaaatgta gcacctgaag tcagccccat acgatataag 5940
   ttgtaattct catgtttgac agcttatcat cgatggagca caggatgacg cctaacaatt 6000
   cattcaagcc gacaccgctt cgcggcgcgg cttaattcag gagttaaaca tcatgaggga 6060
   agcggtgatc gccgaagtat cgactcaact atcagaggta gttggcgtca tcgagcgcca 6120
   tctcgaaccg acgttgctgg ccgtacattt gtacggctcc gcagtggatg gcggcctgaa 6180
   gccacacagt gatattgatt tgctggttac ggtgactgta aggcttgatg aaacaacgcg 6240
   gcgagctttg atcaacgacc ttttggaaac ttcggcttcc cctggagaga gcgagattct 6300
   ccgcgctgta gaagtcacca ttgttgtgca cgacgacatc attccgtggc gttatccagc 6360
   taagcgcgaa ctgcaatttg gagaatggca gcgcaatgac attcttgcag gtatcttcga 6420
   gccagccacg atcgacattg atctggctat cttgctgaca aaagcaagag aacatagcgt 6480
   tgccttggta ggtccagcgg cggaggaact ctttgatccg gttcctgaac aggatctatt 6540
   tgaggcgcta aatgaaacct taacgctatg gaactcgccg cccgactggg ctggcgatga 6600
   gcgaaatgta gtgcttacgt tgtcccgcat ttggtacagc gcagtaaccg gcaaaatcgc 6660
   gccgaaggat gtcgctgccg actgggcaat ggagcgcctg ccggcccagt atcagcccgt 6720
   catacttgaa gctaggcagg cttatcttgg acaagaagat cgcttggcct cgcgcgcaga 6780
   tcagttggaa gaatttgttc actacgtgaa aggcgagatc accaaggtag tcggcaaata 6840
   atgtctaaca attcgttcaa gccgacgccg cttcgcggcg cggcttaact caagcgttag 6900
   agagctgggg aagactatgc gcgatctgtt gaaggtggtt ctaagcctcg tacttgcgat 6960
   ggcatcgggg caggcacttg ctgacctgcc aacgcgcctt tgtagtcttg gcctgttgtg 7020
   tgcatgagca aatcaatggc accaccccct cctttttgag ctgaatggtc ataaaattta 7080
   taattatcta tcgtaattcg gaatctatgt tcagggtctc gccattgctt tttgtctgct 7140
   gggtcaagtt ccatgcctaa ggtttttaag acatcagaaa gaggtattgc acgcatgcta 7200
   tcagcttttc ttctagctaa tgacagggct tcctctgctc tatctgctcg ttttttttct 7260
   tccacatatc tcgccgcttt gtcagccagc ggctgtatta cggaaagtgc cgatttttgg 7320
   gcttttaggc gttctttttc tgcccattct tccttatttg taaaaattga gggtgggatg 7380
   ggtgcctgaa tcttgggatc tagctgtaaa gttttgttga tatttccgta atgtctttgg 7440
   actctttgat gcgttgcttt tgaacctttt acgcctctgg ccagccctag aggctccata 7500
   gaagccgcat aatccgtctg gagggcagaa agggcttttc gaccatcaaa ccatctcgat 7560
   gcgtttaaac ggcctgtatc ggggtctcta ggcaccataa agccggttaa gtggggtgtt 7620
   gtttcatcag catgtagctg aagagataca aggttgtttt ctccaaaggt ttgttccgcc 7680
   cattgctggg tgattgtttt ccagtgttcg agtttttcag gagtggcctg ttttgaccat 7740
   tctggagaca taccaaagaa cagttctatg gcctgcacac cgttttttct aagaggcttt 7800
   cccgtttctt tctgaatttt attcagcata gatttaacat ctgctgatgg gtcagtagag 7860
   cctttgagta tttcgtttag ttcttttcta tctgggtcag cgttttgtgt ttcgcggcct 7920
   cgcgtcatat gcaggctcgc ggctttaatc gtgccaactg ttttatgttt ttcaaaccta 7980
   aagattgcat agttcggcat gttttaactg ctttaatttg agaaaagacc agaggaaata 8040
   atccagccta tatttctttc cctagtagcg aactggaatt gtttttccga aggaaaaaag 8100
   caattccgta gtgagtactg aatttattct gattcgtctt gcttttggag cgtctttttg 8160
   cgttctataa ctgttgtgaa agctacgcgg tcgccattga aaacgaaatt aggattaata 8220
   aaataccatc cttggcgaac atgctttgca atgattttag ctttttctaa ttcggctaga 8280
   cctcttgcaa aggtagcttg agatagtgcc agtttttttt cttgtgcgtt aagaaagtcc 8340
   tctaaaacga atttgtctaa agggacgagg tctttgctga tgcctttgtc ttgaagtatc 8400
   caaaccagaa cgctgaaagc ttttattcca gcggctccta gttcaaaagt tagcgcgata 8460
   ttggtgctaa ataattttac aaattcttca ctatcaacac gtctgtaagt cgtcacatga 8520
   gtgccttgca tctcaccagt ggcttgattg accagaatgt tatcatctcg tcctaatcga 8580
   gataactgaa ccctctgact tttaactggc acaaccatac cttcgatgaa aggattctcg 8640
   tcatatctga ttggctgctt tctcaatttt gtcgccatat ttgataaacc tttaatcaaa 8700
   aaaaccacat tttttgatta tacctattca tcgaatgagg caaggtctat caattttacc 8760
   cctttttttg atagacggtt taatcaatat tgatagaccc cttcacagat tctgaaaatc 8820
   gacttcccta ttttagggat attttcacga ttccctttct tagttcttcc tagtggggaa 8880
   attcgttgaa tcctgcctcg gaaaaaccat gagaaagctg ttggttatat acacgggcaa 8940
   agccacccta tttttagcta ctggggaaag agataaggca gggtatttgt aaaattaaaa 9000
   ccggattttt cgctttacgg tttgtttagg cgcaactgtc tttttaagac cgcgtttaac 9060
   catcaaaaga tcgttccaat cttttccgtg tatcatctgt tctttaggtg ggagccagtt 9120
   ttcaactttt tttgttggaa acgcggcttt aatcgctccg actaatagcg atgctgctct 9180
   ttgtcctaca gcatcccaat cataggcaat atggacagaa gatgcctttt caacgatttt 9240
   tcggagagtt ttagtaagag acgttcttac gccgctggtg cttaataatt ttacgccagc 9300
   tttaattttt tctgggctta aaaagccgac tactgaaatc gcgtctatcg cactttcagc 9360
   gatataaaga tcatactttt cgtcattttt tacattgatg ctgccagtaa aatgggcttc 9420
   gcgactgctt cccaaggcta accctttaaa accactgctt gttccgcgta attctgcgcc 9480
   ctgaagtgta tctttatcgt catacatcaa gaaggctaca ttaccgcgat catctgttcg 9540
   gatagagtca ggaatattgt taaatgatat tcctcgggca gcgttgggtc ctggccacgg 9600
   gtgcgcatga tcgtgctcct gtcgttgagg acccggctag gctggcgggg ttgccttact 9660
   ggttagcaga atgaatcacc gatacgcgag cgaacgtgaa gcgactgctg ctgcaaaacg 9720
   tctgcgacct gagcaacaac atgaatggtc ttcggtttcc gtgtttcgta aagtctggaa 9780
   acgcggaagt cccctacgtg ctgctgaagt tgcccgcaac agagagtgga accaaccggt 9840
   gataccacga tactatgact gagagtcaac gccatgggag ctca 9884
<210> 42
   <211> 34
   <212> DNA
   <213> artificial sequence
<220>
   <223> primer
<400> 42
   atgggagctc gtttttctat ccccatcacc tcgg 34
<210> 43
   <211> 35
   <212> DNA
   <213> artificial sequence
<220>
   <223> primer
<400> 43
   atcgactagt gggtcataat atgggcaaag acgct 35
<210> 44
   <211> 895
   <212> DNA
   <213> artificial sequence
<220>
   <223> LDH-L PCR fragment
<400> 44
   atgggagctc gtttttctat ccccatcacc tcggttttgt tgacaaaaaa aggtggccac      60
   taaattggct ttccgcaccg atgggatgat ttttattctt tgctattctt cgctctttgc      120
   ccaattcatt aaaagcggaa atcatcacca aagatagaag acgcagcctt caccatttca      180
   gattgccctt ctcgggcatt ttctgctgct agaatcctct taaaaatatt aaattccact      240
   ctattggtaa tatgtttccc tctttaggga acaaataaag cccttctttg ttctataaaa      300
   gttagcttac cgattttaca aaaaataata ccgcttcatt caatcggtaa tacatatctt      360
   ttttcttcaa aaaacttttc aagagggtgt ctatgcgcgt cgcaatattc agttccaaaa      420
   actatgacca tcattctatt gaaaaagaaa atgaacatta tggccatgac cttgtttttc      480
   tgaatgagcg gcttaccaaa gagacagcag aaaaagccaa agacgcagaa gctgtttgta      540
   tctttgtgaa tgacgaagcc aatgccgaag tgctggaaat tttggcaggc ttaggcatca      600
   agttggttgc tcttcgttgc gccggttata acaatgtcga tctcgatgcg gccaaaaagc      660
   tgaatatcaa ggttgtgcgc gtgcctgcct attcgcccta ttcggttgcc gaatatgcag      720
   tagggatgtt gctcaccctg aatcggcaaa tttcacgcgg tttgaagcgg gttcgggaaa      780
   ataacttctc cttggaaggt ttgattggcc ttgatgtgca tgacaaaaca gtcggcatta      840
   tcggtgttgg tcatatcggg agcgtctttg cccatattat gacccactag tcgat           895
<210> 45
   <211> 33
   <212> DNA
   <213> artificial sequence
<220>
   <223> primer
<400> 45
   gcgaattcat ggttttggtg ccaatgttat cgc 33
<210> 46
   <211> 35
   <212> DNA
   <213> artificial sequence
<220>
   <223> primer
<400> 46
   ttaggcggcc gcgcggctga catacatctt gcgaa 35
<210> 47
   <211> 1169
   <212> DNA
   <213> artificial sequence
<220>
   <223> LDH-R PCR fragment
<400> 47
   gcgaattcat ggttttggtg ccaatgttat cgcctataaa ccgcatccag accccgaatt      60
   ggcgaaaaag gtcggtttcc gcttcacctc tctcgatgaa gtgatcgaga ccagcgacat     120
   catttcgctt cactgtccgc tcacgccaga aaatcatcac atgattaatg aagaaacact     180
   ggcaagggca aaaaaaggct tttacctcgt caataccagt cgcggcggct tggttgatac     240
   caaggcggtg attaaatcgc tgaaagccaa acatctcggc ggttatgcgg cggatgttta     300
   cgaagaggag gggcctttat tcttcgaaaa tcacgctgac gatattatcg aagatgatat     360
   tctcgaaagg ttgatcgctt tcccgaatgt ggttttcacg ggacatcagg cctttttgac     420
   gaaagaggcc ttatcaaaca ttgctcacag tattctacaa gatatcagcg atgccgaagc     480
   tggaaaagaa atgccggatg cgcttgttta gtagacaagc gacaattaac cttttgaaga     540
   tcataatgat caaatttttg ggttaattcg gtagttatgg cataggctat tacgcgctaa     600
   ttgatatcaa aaaaaagcat agccggacat cataccggct atgtttttta ttaggaaaaa     660
   atttcctttc accttgctta gccatcgccg cattatttaa tcaatatgcc gagtttttct     720
   tgaaatccct atcttacacc aaggccaaca agggaatcat ccatactcgg tgtcctatcc     780
   tatgactttt taaattttct ccaaatttac taaaatcacg ccatctcagc ggctgctatt     840
   ttcaaaaagc gcctctcaaa accgcttttt cctgctcaaa tatcggatcc caaaattccc     900
   tcaaaaaagg caggtatttt tttacaaaat cgcccctaat atctctcaat ccgctgcctt     960
   gttcatatgt ttttgcaaat gatttttatt aaactttttt aggcgtattt ttatcaagaa    1020
   aatttaaata atcacatttt tattatttta gatttaagta ttgatacaag tgatatctat    1080
   aaatgttttt ataactttct ggatcgtaat cggctggcaa tcgttttccc tatattcgca    1140
   agatgtatgt cagccgcgcg gccgcctaa                                      1169
<210> 48
   <211> 1098
   <212> DNA
   <213> artificial sequence
<220>
   <223> LoxPw-aadA-LoxPw PCR fragment
<400> 48
   ataacttcgt ataatgtatg ctatacgaag ttatgcggcc gcagcacagg atgacgccta 60
   acaattcatt caagccgaca ccgcttcgcg gcgcggctta attcaggagt taaacatcat 120
   gagggaagcg gtgatcgccg aagtatcgac tcaactatca gaggtagttg gcgtcatcga 180
   gcgccatctc gaaccgacgt tgctggccgt acatttgtac ggctccgcag tggatggcgg 240
   cctgaagcca cacagtgata ttgatttgct ggttacggtg actgtaaggc ttgatgaaac 300
   aacgcggcga gctttgatca acgacctttt ggaaacttcg gcttcccctg gagagagcga 360
   gattctccgc gctgtagaag tcaccattgt tgtgcacgac gacatcattc cgtggcgtta 420
   tccagctaag cgcgaactgc aatttggaga atggcagcgc aatgacattc ttgcaggtat 480
   cttcgagcca gccacgatcg acattgatct ggctatcttg ctgacaaaag caagagaaca 540
   tagcgttgcc ttggtaggtc cagcggcgga ggaactcttt gatccggttc ctgaacagga 600
   tctatttgag gcgctaaatg aaaccttaac gctatggaac tcgccgcccg actgggctgg 660
   cgatgagcga aatgtagtgc ttacgttgtc ccgcatttgg tacagcgcag taaccggcaa 720
   aatcgcgccg aaggatgtcg ctgccgactg ggcaatggag cgcctgccgg cccagtatca 780
   gcccgtcata cttgaagcta ggcaggctta tcttggacaa gaagatcgct tggcctcgcg 840
   cgcagatcag ttggaagaat ttgttcacta cgtgaaaggc gagatcacca aggtagtcgg 900
   caaataatgt ctaacaattc gttcaagccg acgccgcttc gcggcgcggc ttaactcaag 960
   cgttagagag ctggggaaga ctatgcgcga tctgttgaag gtggttctaa gcctcgtact 1020
   tgcgatggca tcggggcagg cacttgctga cctgccttaa ttaaataact tcgtataatg 1080
   tatgctatac gaagttat 1098
<210> 49
   <211> 10441
   <212> DNA
   <213> artificial sequence
<220>
   <223> constructed plasmid
<400> 49
   ctagttcgat caacaacccg aatcctatcg taatgatgtt ttgcccgatc agcctcaatc 60
   gacaatttta cgcgtttcga tcgaagcagg gacgacaatt ggctgggaac ggtatactgg 120
   aataaatggt cttcgttatg gtattgatgt ttttggtgca tcggccccgg cgaatgatct 180
   atatgctcat ttcggcttga ccgcagtcgg catcacgaac aaggtgttgg ccgcgatcgc 240
   cggtaagtcg gcacgttaaa aaatagctat ggaatataat agctacttaa taagttagga 300
   gaataaacat ggcgattgca attggcctcg attttggcag tgattctgtg cgagctttgg 360
   cggtggactg cgctaccggt gaagagatcg ccaccagcgt agagtggtat ccccgttggc 420
   agaaagggca attttgtgat gccccgaata accagttccg tcatcatccg cgtgactaca 480
   ttgagtcaat ggaagcggca ctgaaaaccg tgcttgcaga gcttagcgtc gaacagcgcg 540
   cagctgtggt cgggattggc gttgacagta ccggctcgac gcccgcaccg attgatgccg 600
   acggaaacgt gctggcgctg cgcccggagt ttgccgaaaa cccgaacgcg atgttcgtat 660
   tgtggaaaga ccacactgcg gttgaagaag cggaagagat tacccgtttg tgccacgcgc 720
   cgggcaacgt tgactactcc cgctacattg gtggtattta ttccagcgaa tggttctggg 780
   caaaaatcct gcatgtgact cgccaggaca gcgccgtggc gcaatctgcc gcatcgtgga 840
   ttgagctgtg cgactgggtg ccagctctgc tttccggtac cacccgcccg caggatattc 900
   gtcgcggacg ttgcagcgcc gggcataaat ctctgtggca cgaaagctgg ggcggcctgc 960
   cgccagccag tttctttgat gagctggacc cgatcctcaa tcgccatttg ccttccccgc 1020
   tgttcactga cacttggact gccgatattc cggtgggcac cttatgcccg gaatgggcgc 1080
   agcgtctcgg cctgcctgaa agcgtggtga tttccggcgg cgcgtttgac tgccatatgg 1140
   gcgcagttgg cgcaggcgca cagcctaacg cactggtaaa agttatcggt acttccacct 1200
   gcgacattct gattgccgac aaacagagcg ttggcgagcg ggcagttaaa ggtatttgcg 1260
   gtcaggttga tggcagcgtg gtgcctggat ttatcggtct ggaagcaggc caatcggcgt 1320
   ttggtgatat ctacgcctgg tttggtcgcg tactcggctg gccgctggaa cagcttgccg 1380
   cccagcatcc ggaactgaaa acgcaaatca acgccagcca gaaacaactg cttccggcgc 1440
   tgaccgaagc atgggccaaa aatccgtctc tggatcacct gccggtggtg ctcgactggt 1500
   ttaacggccg ccgcacaccg aacgctaacc aacgcctgaa aggggtgatt accgatctta 1560
   acctcgctac cgacgctccg ctgctgttcg gcggtttgat tgctgccacc gcctttggcg 1620
   cacgcgcaat catggagtgc tttaccgatc aggggatcgc cgttaataac gtgatggcac 1680
   tgggcggcat cgcgcggaaa aaccaggtca ttatgcaggc ctgctgcgac gtgctgaatc 1740
   gcccgctgca aattgttgcc tctgaccagt gctgtgcgct cggtgcggcg atttttgctg 1800
   ccgtcgccgc gaaagtgcac gcagacatcc catcagctca gcaaaaaatg gccagtgcgg 1860
   tagagaaaac cctgcaaccg tgcagcgagc aggcacaacg ctttgaacag ctttatcgcc 1920
   gctatcagca atgggcgatg agcgccgaac aacactatct tccaacttcc gccccggcac 1980
   aggctgccca ggccgttgcg actctataag gacacgataa tgacgatttt tgataattat 2040
   gaagtgtggt ttgtcattgg cagccagcat ctgtatggcc cggaaaccct gcgtcaggtc 2100
   acccaacatg ccgagcacgt cgttaatgcg ctgaatacgg aagcgaaact gccctgcaaa 2160
   ctggtgttga aaccgctggg caccacgccg gatgaaatca ccgctatttg ccgcgacgcg 2220
   aattacgacg atcgttgcgc tggtctggtg gtgtggctgc acaccttctc cccggccaaa 2280
   atgtggatca acggcctgac catgctcaac aaaccgttgc tgcaattcca cacccagttc 2340
   aacgcggcgc tgccgtggga cagtatcgat atggacttta tgaacctgaa ccagactgca 2400
   catggcggtc gcgagttcgg cttcattggc gcgcgtatgc gtcagcaaca tgccgtggtt 2460
   accggtcact ggcaggataa acaagcccat gagcgtatcg gctcctggat gcgtcaggcg 2520
   gtctctaaac aggatacccg tcatctgaaa gtctgccgat ttggcgataa catgcgtgaa 2580
   gtggcggtca ccgatggcga taaagttgcc gcacagatca agttcggttt ctccgtcaat 2640
   acctgggcgg ttggcgatct ggtgcaggtg gtgaactcca tcagcgacgg cgatgttaac 2700
   gcgctggtcg atgagtacga aagctgctac accatgacgc ctgccacaca aatccacggc 2760
   aaaaaacgac agaacgtgct ggaagcggcg cgtattgagc tggggatgaa gcgtttcctg 2820
   gaacaaggtg gcttccacgc gttcaccacc acctttgaag atttgcacgg tctgaaacag 2880
   cttcctggtc tggccgtaca gcgtctgatg cagcagggtt acggctttgc gggcgaaggc 2940
   gactggaaaa ctgccgccct gcttcgcatc atgaaggtga tgtcaaccgg tctgcagggc 3000
   ggcacctcct ttatggagga ctacacctat cacttcgaga aaggtaatga cctggtgctc 3060
   ggctcccata tgctggaagt ctgcccgtcg atcgccgcag aagagaaacc gatcctcgac 3120
   gttcagcatc tcggtattgg tggtaaggac gatcctgccc gcctgatctt caatacccaa 3180
   accggcccag cgattgtcgc cagcttgatt gatctcggcg atcgttaccg tctactggtt 3240
   aactgcatcg acacggtgaa aacaccgcac tccctgccga aactgccggt ggcgaatgcg 3300
   ctgtggaaag cgcaaccgga tctgccaact gcttccgaag cgtggatcct cgctggtggc 3360
   gcgcaccata ccgtcttcag ccatgcactg aacctcaacg atatgcgcca attcgccgag 3420
   atgcacgaca ttgaaatcac ggtgattgat aacgacacac gcctgccagc gtttaaagac 3480
   gcgctgcgct ggaacgaagt gtattacgga tttcgtcgct aagtctagag aaggagtcaa 3540
   catgttagaa gatctcaaac gccaggtatt agaagccaac ctggcgctgc caaaacacaa 3600
   cctggtcacg ctcacatggg gcaacgtcag cgccgttgat cgcgagcgcg gcgtctttgt 3660
   gatcaaacct tccggcgtcg attacagcgt catgaccgct gacgatatgg tcgtggttag 3720
   catcgaaacc ggtgaagtgg ttgaaggtac gaaaaagccc tcctccgaca cgccaactca 3780
   ccggctgctc tatcaggcat tcccctccat tggcggcatt gtgcatacgc actcgcgcca 3840
   cgccaccatc tgggcgcagg cgggtcagtc gattccagca accggcacca cccacgccga 3900
   ctatttctac ggcaccattc cctgtacccg caaaatgacc gacgcagaaa tcaacggcga 3960
   atatgagtgg gaaaccggta acgtcatcgt agaaaccttt gaaaaacagg gtatcgatgc 4020
   agcgcaaatg cccggcgttc tggtccattc ccacggcccg tttgcatggg gcaaaaatgc 4080
   cgaagatgcg gtgcataacg ccatcgtgct ggaagaggtc gcttatatgg ggatattctg 4140
   ccgtcagtta gcgccgcagt taccggatat gcagcaaacg ctgctggata aacactatct 4200
   gcgtaagcat ggcgcgaagg catattacgg gcagtaatga ctgtataaaa ccacagccaa 4260
   tcaaacgaaa ccaggctata ctcaagcctg gttttttgat ggattttcag cgtggcgcag 4320
   gcaggtttta tcttaacccg acactggcgg gacaccccgc aagggacaga agtctccttc 4380
   tggctggcga cggacaacgg gccaagcttg gaagggcgaa ttcgcgatcg cataacttcg 4440
   tataatgtat gctatacgaa gttatgcggc cgcagcacag gatgacgcct aacaattcat 4500
   tcaagccgac accgcttcgc ggcgcggctt aattcaggag ttaaacatca tgagggaagc 4560
   ggtgatcgcc gaagtatcga ctcaactatc agaggtagtt ggcgtcatcg agcgccatct 4620
   cgaaccgacg ttgctggccg tacatttgta cggctccgca gtggatggcg gcctgaagcc 4680
   acacagtgat attgatttgc tggttacggt gactgtaagg cttgatgaaa caacgcggcg 4740
   agctttgatc aacgaccttt tggaaacttc ggcttcccct ggagagagcg agattctccg 4800
   cgctgtagaa gtcaccattg ttgtgcacga cgacatcatt ccgtggcgtt atccagctaa 4860
   gcgcgaactg caatttggag aatggcagcg caatgacatt cttgcaggta tcttcgagcc 4920
   agccacgatc gacattgatc tggctatctt gctgacaaaa gcaagagaac atagcgttgc 4980
   cttggtaggt ccagcggcgg aggaactctt tgatccggtt cctgaacagg atctatttga 5040
   ggcgctaaat gaaaccttaa cgctatggaa ctcgccgccc gactgggctg gcgatgagcg 5100
   aaatgtagtg cttacgttgt cccgcatttg gtacagcgca gtaaccggca aaatcgcgcc 5160
   gaaggatgtc gctgccgact gggcaatgga gcgcctgccg gcccagtatc agcccgtcat 5220
   acttgaagct aggcaggctt atcttggaca agaagatcgc ttggcctcgc gcgcagatca 5280
   gttggaagaa tttgttcact acgtgaaagg cgagatcacc aaggtagtcg gcaaataatg 5340
   tctaacaatt cgttcaagcc gacgccgctt cgcggcgcgg cttaactcaa gcgttagaga 5400
   gctggggaag actatgcgcg atctgttgaa ggtggttcta agcctcgtac ttgcgatggc 5460
   atcggggcag gcacttgctg acctgcctta attaaataac ttcgtataat gtatgctata 5520
   cgaagttatg gccggccaat tcatggtttt ggtgccaatg ttatcgccta taaaccgcat 5580
   ccagaccccg aattggcgaa aaaggtcggt ttccgcttca cctctctcga tgaagtgatc 5640
   gagaccagcg acatcatttc gcttcactgt ccgctcacgc cagaaaatca tcacatgatt 5700
   aatgaagaaa cactggcaag ggcaaaaaaa ggcttttacc tcgtcaatac cagtcgcggc 5760
   ggcttggttg ataccaaggc ggtgattaaa tcgctgaaag ccaaacatct cggcggttat 5820
   gcggcggatg tttacgaaga ggaggggcct ttattcttcg aaaatcacgc tgacgatatt 5880
   atcgaagatg atattctcga aaggttgatc gctttcccga atgtggtttt cacgggacat 5940
   caggcctttt tgacgaaaga ggccttatca aacattgctc acagtattct acaagatatc 6000
   agcgatgccg aagctggaaa agaaatgccg gatgcgcttg tttagtagac aagcgacaat 6060
   taaccttttg aagatcataa tgatcaaatt tttgggttaa ttcggtagtt atggcatagg 6120
   ctattacgcg ctaattgata tcaaaaaaaa gcatagccgg acatcatacc ggctatgttt 6180
   tttattagga aaaaatttcc tttcaccttg cttagccatc gccgcattat ttaatcaata 6240
   tgccgagttt ttcttgaaat ccctatctta caccaaggcc aacaagggaa tcatccatac 6300
   tcggtgtcct atcctatgac tttttaaatt ttctccaaat ttactaaaat cacgccatct 6360
   cagcggctgc tattttcaaa aagcgcctct caaaaccgct ttttcctgct caaatatcgg 6420
   atcccaaaat tccctcaaaa aaggcagggt attttttaca aaatcgcccc taatatctct 6480
   caatccgctg ccttgttcat atgtttttgc aaatgatttt tattaaactt ttttaggcgt 6540
   atttttatca agaaaattta aataatcaca tttttattat tttagattta agtattgata 6600
   caagtgatat ctataaatgt ttttataact ttctggatcg taatcggctg gcaatcgttt 6660
   tccctatatt cgcaagatgt atgtcagccg cgcggccgct ggtacccaat tcgccctata 6720
   gtgagtcgta ttacgcgcgc tcactggccg tcgttttaca acgtcgtgac tgggaaaacc 6780
   ctggcgttac ccaacttaat cgccttgcag cacatccccc tttcgccagc tggcgtaata 6840
   gcgaagaggc ccgcaccgat cgcccttccc aacagttgcg cagcctgaat ggcgaatggg 6900
   acgcgccctg tagcggcgca ttaagcgcgg cgggtgtggt ggttacgcgc agcgtgaccg 6960
   ctacacttgc cagcgcccta gcgcccgctc ctttcgcttt cttcccttcc tttctcgcca 7020
   cgttcgccgg ctttccccgt caagctctaa atcgggggct ccctttaggg ttccgattta 7080
   gtgctttacg gcacctcgac cccaaaaaac ttgattaggg tgatggttca cgtagtgggc 7140
   catcgccctg atagacggtt tttcgccctt tgacgttgga gtccacgttc tttaatagtg 7200
   gactcttgtt ccaaactgga acaacactca accctatctc ggtctattct tttgatttat 7260
   aagggatttt gccgatttcg gcctattggt taaaaaatga gctgatttaa caaaaattta 7320
   acgcgaattt taacaaaata ttaacgctta caatttaggt ggcacttttc ggggaaatgt 7380
   gcgcggaacc cctatttgtt tatttttcta aatacattca aatatgtatc cgctcatgag 7440
   acaataaccc tgataaatgc ttcaataata ttgaaaaagg aagagtatga gtattcaaca 7500
   tttccgtgtc gcccttattc ccttttttgc ggcattttgc cttcctgttt ttgctcaccc 7560
   agaaacgctg gtgaaagtaa aagatgctga agatcagttg ggtgcacgag tgggttacat 7620
   cgaactggat ctcaacagcg gtaagatcct tgagagtttt cgccccgaag aacgttttcc 7680
   aatgatgagc acttttaaag ttctgctatg tggcgcggta ttatcccgta ttgacgccgg 7740
   gcaagagcaa ctcggtcgcc gcatacacta ttctcagaat gacttggttg agtactcacc 7800
   agtcacagaa aagcatctta cggatggcat gacagtaaga gaattatgca gtgctgccat 7860
   aaccatgagt gataacactg cggccaactt acttctgaca acgatcggag gaccgaagga 7920
   gctaaccgct tttttgcaca acatggggga tcatgtaact cgccttgatc gttgggaacc 7980
   ggagctgaat gaagccatac caaacgacga gcgtgacacc acgatgcctg tagcaatggc 8040
   aacaacgttg cgcaaactat taactggcga actacttact ctagcttccc ggcaacaatt 8100
   aatagactgg atggaggcgg ataaagttgc aggaccactt ctgcgctcgg cccttccggc 8160
   tggctggttt attgctgata aatctggagc cggtgagcgt gggtctcgcg gtatcattgc 8220
   agcactgggg ccagatggta agccctcccg tatcgtagtt atctacacga cggggagtca 8280
   ggcaactatg gatgaacgaa atagacagat cgctgagata ggtgcctcac tgattaagca 8340
   ttggtaactg tcagaccaag tttactcata tatactttag attgatttaa aacttcattt 8400
   ttaatttaaa aggatctagg tgaagatcct ttttgataat ctcatgacca aaatccctta 8460
   acgtgagttt tcgttccact gagcgtcaga ccccgtagaa aagatcaaag gatcttcttg 8520
   agatcctttt tttctgcgcg taatctgctg cttgcaaaca aaaaaaccac cgctaccagc 8580
   ggtggtttgt ttgccggatc aagagctacc aactcttttt ccgaaggtaa ctggcttcag 8640
   cagagcgcag ataccaaata ctgtccttct agtgtagccg tagttaggcc accacttcaa 8700
   gaactctgta gcaccgccta catacctcgc tctgctaatc ctgttaccag tggctgctgc 8760
   cagtggcgat aagtcgtgtc ttaccgggtt ggactcaaga cgatagttac cggataaggc 8820
   gcagcggtcg ggctgaacgg ggggttcgtg cacacagccc agcttggagc gaacgaccta 8880
   caccgaactg agatacctac agcgtgagct atgagaaagc gccacgcttc ccgaagggag 8940
   aaaggcggac aggtatccgg taagcggcag ggtcggaaca ggagagcgca cgagggagct 9000
   tccaggggga aacgcctggt atctttatag tcctgtcggg tttcgccacc tctgacttga 9060
   gcgtcgattt ttgtgatgct cgtcaggggg gcggagccta tggaaaaacg ccagcaacgc 9120
   ggccttttta cggttcctgg ccttttgctg gccttttgct cacatgttct ttcctgcgtt 9180
   atcccctgat tctgtggata accgtattac cgcctttgag tgagctgata ccgctcgccg 9240
   cagccgaacg accgagcgca gcgagtcagt gagcgaggaa gcggaagagc gcccaatacg 9300
   caaaccgcct ctccccgcgc gttggccgat tcattaatgc agctggcacg acaggtttcc 9360
   cgactggaaa gcgggcagtg agcgcaacgc aattaatgtg agttagctca ctcattaggc 9420
   accccaggct ttacacttta tgcttccggc tcgtatgttg tgtggaattg tgagcggata 9480
   acaatttcac acaggaaaca gctatgacca tgattacgcc aagcgcgcaa ttaaccctca 9540
   ctaaagggaa caaaagctgg agctcgtttt tctatcccca tcacctcggt tttgttgaca 9600
   aaaaaaggtg gccactaaat tggctttccg caccgatggg atgattttta ttctttgcta 9660
   ttcttcgctc tttgcccaat tcattaaaag cggaaatcat caccaaagat agaagacgca   9720
   gccttcacca tttcagattg cccttctcgg gcattttctg ctgctagaat cctcttaaaa   9780
   atattaaatt ccactctatt ggtaatatgt ttccctcttt agggaacaaa taaagccctt   9840
   ctttgttcta taaaagttag cttaccgatt ttacaaaaaa taataccgct tcattcaatc   9900
   ggtaatacat atcttttttc ttcaaaaaac ttttcaagag ggtgtctatg cgcgtcgcaa   9960
   tattcagttc caaaaactat gaccatcatt ctattgaaaa agaaaatgaa cattatggcc  10020
   atgaccttgt ttttctgaat gagcggctta ccaaagagac agcagaaaaa gccaaagacg  10080
   cagaagctgt ttgtatcttt gtgaatgacg aagccaatgc cgaagtgctg gaaattttgg  10140
   caggcttagg catcaagttg gttgctcttc gttgcgccgg ttataacaat gtcgatctcg  10200
   atgcggccaa aaagctgaat atcaaggttg tgcgcgtgcc tgcctattcg ccctattcgg  10260
   ttgccgaata tgcagtaggg atgttgctca ccctgaatcg gcaaatttca cgcggtttga  10320
   agcgggttcg ggaaaataac ttctccttgg aaggtttgat tggccttgat gtgcatgaca  10380
   aaacagtcgg cattatcggt gttggtcata tcgggagcgt ctttgcccat attatgaccc  10440
   a                                                                  10441
<210> 50
   <211> 20
   <212> DNA
   <213> artificial sequence
<220>
   <223> primer
<400> 50
   gccttgggct tttaaagcct 20
<210> 51
   <211> 22
   <212> DNA
   <213> artificial sequence
<220>
   <223> primer
<400> 51
   tcaatccacg atgcggcaga tt 22
<210> 52
   <211> 20
   <212> DNA
   <213> artificial sequence
<220>
   <223> primer
<400> 52
   ccagtatcag cccgtcatac 20
<210> 53
   <211> 26
   <212> DNA
   <213> artificial sequence
<220>
   <223> primer
<400> 53
   tctcggagag atagaggtca gtcgac 26
<210> 54
   <211> 27
   <212> DNA
   <213> artificial sequence
<220>
   <223> primer
<400> 54
   aaccatggtt actatcaata cggaatc 27
<210> 55
   <211> 27
   <212> DNA
   <213> artificial sequence
<220>
   <223> primer
<400> 55
   ttgaattcct gatgtgtgtt accgcaa 27
<210> 56
   <211> 1550
   <212> DNA
   <213> artificial sequence
<220>
   <223> araE PCR fragment
<400> 56
   aaccatggtt actatcaata cggaatctgc tttaacgcca cgttctttgc gggatacgcg 60
   gcgtatgaat atgtttgttt cggtagctgc tgcggtcgca ggattgttat ttggtcttga 120
   tatcggcgta atcgccggag cgttgccgtt cattaccgat cactttgtgc tgaccagtcg 180
   tttgcaggaa tgggtggtta gtagcatgat gctcggtgca gcaattggtg cgctgtttaa 240
   tggttggctg tcgttccgcc tggggcgtaa atacagcctg atggcggggg ccatcctgtt 300
   tgtactcggt tctatagggt ccgcttttgc gaccagcgta gagatgttaa tcgccgctcg 360
   tgtggtgctg ggcattgctg tcgggatcgc gtcttacacc gctcctctgt atctttctga 420
   aatggcaagt gaaaacgttc gcggtaagat gatcagtatg taccagttga tggtcacact 480
   cggcatcgtg ctggcgtttt tatccgatac agcgttcagt tatagcggta actggcgcgc 540
   aatgttgggg gttcttgctt taccagcagt tctgctgatt attctggtag tcttcctgcc 600
   aaatagcccg cgctggctgg cggaaaaggg gcgtcatatt gaggcggaag aagtattgcg 660
   tatgctgcgc gatacgtcgg aaaaagcgcg agaagaactc aacgaaattc gtgaaagcct 720
   gaagttaaaa cagggcggtt gggcactgtt taagatcaac cgtaacgtcc gtcgtgctgt 780
   gtttctcggt atgttgttgc aggcgatgca gcagtttacc ggtatgaaca tcatcatgta 840
   ctacgcgccg cgtatcttca aaatggcggg ctttacgacc acagaacaac agatgattgc 900
   gactctggtc gtagggctga cctttatgtt cgccaccttt attgcggtgt ttacggtaga 960
   taaagcaggg cgtaaaccgg ctctgaaaat tggtttcagc gtgatggcgt taggcactct 1020
   ggtgctgggc tattgcctga tgcagtttga taacggtacg gcttccagtg gcttgtcctg 1080
   gctctctgtt ggcatgacga tgatgtgtat tgccggttat gcgatgagcg ccgcgccagt 1140
   ggtgtggatc ctgtgctctg aaattcagcc gctgaaatgc cgcgatttcg gtattacctg 1200
   ttcgaccacc acgaactggg tgtcgaatat gattatcggc gcgaccttcc tgacactgct 1260
   tgatagcatt ggcgctgccg gtacgttctg gctctacact gcgctgaaca ttgcgtttgt 1320
   gggcattact ttctggctca ttccggaaac caaaaatgtc acgctggaac atatcgaacg 1380
   caaactgatg gcaggcgaga agttgagaaa tatcggcgtc tgatttcacg ggccggatgt 1440
   gctgtacatc cggccctttt ttcgttaata gagattgggc acttggccgt tgaggcgttt 1500
   gtctcgttcc ttattcagcc ttgttgcggt aacacacatc aggaattcaa 1550
<210> 57
   <211> 32
   <212> DNA
   <213> artificial sequence
<220>
   <223> primer
<400> 57
   aaccatggcg cacaaattta ctaaagccct gg 32
<210> 58
   <211> 30
   <212> DNA
   <213> artificial sequence
<220>
   <223> primer
<400> 58
   ccgaattcct tctcttttct tattgtgttg 30
<210> 59
   <211> 3744
   <212> DNA
   <213> artificial sequence
<220>
   <223> araFGH PCR fragment
<400> 59
   aaccatggcg cacaaattta ctaaagccct ggcagccatt ggtctggcag ccgttatgtc 60
   acaatccgct atggcggaga acctgaagct cggttttctg gtgaagcaac cggaagagcc 120
   gtggttccag accgaatgga agtttgccga taaagccggg aaggatttag ggtttgaggt 180
   tattaagatt gccgtgccgg atggcgaaaa aacattgaac gcgatcgaca gcctggctgc 240
   cagtggcgca aaaggtttcg ttatttgtac tccggacccc aaactcggct ctgccatcgt 300
   cgcgaaagcg cgtggctacg atatgaaagt cattgccgtg gatgaccagt ttgttaacgc 360
   caaaggtaag ccaatggata ccgttccgct ggtgatgatg gcggcgacta aaattggcga 420
   acgtcagggc caggaactgt ataaagagat gcagaaacgt ggctgggatg tcaaagaaag 480
   cgcggtgatg gcgattaccg ccaacgaact ggataccgcc cgccgccgta ctacgggatc 540
   tatggatgcg ctgaaagcgg ccggattccc ggaaaaacaa atttatcagg tacctaccaa 600
   atctaacgac atcccggggg catttgacgc tgccaactca atgctggttc aacatccgga 660
   agttaaacat tggctgatcg tcggtatgaa cgacagcacc gtgctgggcg gcgtacgcgc 720
   gacggaaggt cagggcttta aagcggccga tatcatcggc attggcatta acggtgtgga 780
   tgcggtgagc gaactgtcta aagcacaggc aaccggcttc tacggttccc tgctgccaag 840
   cccggacgta catggctata aatccagcga aatgctttac aactgggtag caaaagacgt 900
   tgaaccgcca aaatttaccg aagttaccga cgtggtactg atcacgcgtg acaactttaa 960
   agaagaactg gagaaaaaag gtttaggcgg taagtaattt gccggaaaaa ttcccctctg 1020
   catgatgcag agggggtgtg aacgaccagt gattcacgga gacgttatgc aacagtctac 1080
   cccgtatctc tcatttcgcg gcatcggtaa aacgtttccc ggcgttaagg cgctgacgga 1140
   tattagtttt gactgctatg ccggtcaggt tcatgcgttg atgggtgaaa atggcgcagg 1200
   aaaatcaact ctcttaaaaa tcctcagcgg caactatgcg ccaaccacgg gttctgtagt 1260
   gattaatggg caggaaatgt ccttttccga cacgaccgca gcacttaacg cgggcgtggc 1320
   gattatttac caggaactgc atctcgtgcc ggaaatgacc gtcgcggaaa acatctatct 1380
   cggccagctg ccgcataaag gcggcattgt gaatcgctca ttgctgaatt atgaggcggg 1440
   tttacaactt aaacatcttg gtatggatat tgacccggac acgccgctga aatatctctc 1500
   cattggtcag tggcagatgg ttgaaatcgc caaagcgctg gcgcgtaacg ccaaaattat 1560
   cgcctttgat gagccaacca gctccctctc tgcccgtgaa atcgacaatc ttttccgcgt 1620
   tattcgtgaa ctgcgaaaag aggggcgggt aatcttatac gtttctcacc gtatggaaga 1680
   aatatttgcc ctcagcgatg ccattactgt ctttaaagat ggacgttatg tcaaaacctt 1740
   taccgatatg cagcaggttg accacgacgc gctggtgcag gcgatggtcg ggcgcgacat 1800
   tggcgatatc tacggctggc aaccgcgtag ttatggcgag gagcgcctac gtcttgatgc 1860
   tgtgaaagca ccaggcgtgc gtacgccaat aagtctggcg gttcgcagtg gtgaaattgt 1920
   tgggctgttt ggtctggtag gggcggggcg tagcgaatta atgaaaggca tgtttggcgg 1980
   gacgcaaatc accgccggtc aggtttatat cgaccaacag ccgatcgata ttcgtaaacc 2040
   gagccacgcc attgccgcag gcatgatgct ctgcccggaa gatcgcaaag cggaaggcat 2100
   tattcccgtg cactccgttc gcgacaatat caacatcagt gccagacgta aacatgtgct 2160
   cggcggttgt gtaatcaaca acggttggga agaaaacaat gccgatcacc acattcgttc 2220
   gctcaacatc aaaacgccgg gcgcggagca actgatcatg aatctctcag gcggaaatca 2280
   gcaaaaagcc attctgggcc gctggttatc ggaagagatg aaggtcattt tgctggatga 2340
   acctacgcgc ggcattgatg ttggcgctaa gcacgaaata tataacgtaa tttatgcgct 2400
   ggcggcgcag ggcgtggcgg tgctgtttgc ctccagcgac ttacctgaag tcctcggcgt 2460
   tgccgaccgg attgtggtga tgcgggaagg tgaaatcgcc ggtgaattgt tacacgagca 2520
   ggcagatgag cgtcaggcac tgagccttgc gatgcctaaa gtcagccagg ctgttgcctg 2580
   agtaaggaga gtatgatgtc ttctgtttct acatcggggt ctggcgcacc taagtcgtca 2640
   ttcagcttcg ggcgtatctg ggatcagtac ggcatgctgg tggtgtttgc ggtgctcttt 2700
   atcgcctgtg ccatttttgt cccaaatttt gccaccttca ttaatatgaa agggttgggc 2760
   ctggcaattt ccatgtcggg gatggtggct tgtggcatgt tgttctgcct cgcttccggt 2820
   gactttgacc tttctgtcgc ctccgtaatt gcctgtgcgg gtgtcaccac ggcggtggtt 2880
   attaacctga ctgaaagcct gtggattggc gtggcagcgg ggttgttgct gggcgttctc 2940
   tgtggcctgg tcaatggctt tgttatcgcc aaactgaaaa taaatgctct gatcacgaca 3000
   ttggcaacga tgcagattgt tcgaggtctg gcgtacatca tttcagacgg taaagcggtc 3060
   ggtatcgaag atgaaagctt ctttgccctt ggttacgcca actggttcgg tctgcctgcg 3120
   ccaatctggc tcaccgtcgc gtgtctgatt atctttggtt tgctgctgaa taaaaccacc 3180
   tttggtcgta acaccctggc gattggcggg aacgaagagg ccgcgcgtct ggcgggtgta 3240
   ccggttgttc gcaccaaaat tattatcttt gttctctcag gcctggtatc agcgatagcc 3300
   ggaattattc tggcttcacg tatgaccagt gggcagccaa tgacgtcgat tggttatgag 3360
   ctgattgtta tctccgcctg cgttttaggt ggcgtttctc tgaaaggtgg catcggaaaa 3420
   atctcatatg tggtggcggg tatcttaatt ttaggcaccg tggaaaacgc catgaacctg 3480
   cttaatattt ctcctttcgc gcagtacgtg gttcgcggct taatcctgct ggcagcggtg 3540
   atcttcgacc gttacaagca aaaagcgaaa cgcactgtct gatgcttttt tctgcaacaa 3600
   tttagcgttt tttcccacca tagccaaccg ccataacggt tggctgttct tcgttgcaaa 3660
   tggcgacccc cgtcacactg tctatactta catgtctgta aagcgcgttc tgcgcaacac 3720
   aataagaaaa gagaaggaat tcgg 3744
<210> 60
   <211> 27
   <212> DNA
   <213> artificial sequence
<220>
   <223> primer
<400> 60
   gggagctcac tagtcgatct gtgctgt 27
<210> 61
   <211> 23
   <212> DNA
   <213> artificial sequence
<220>
   <223> primer
<400> 61
   agccatggtt acctccggga aac 23
<210> 62
   <211> 181
   <212> DNA
   <213> Actinoplanes missouriensis
<400> 62
   cgatctgtgc tgtttgccac ggtatgcagc accagcgcga gattatgggc tcgcacgctc 60
   gactgtcgga cgggggcact ggaacgagaa gtcaggcgag ccgtcacgcc cttgacaatg 120
   ccacatcctg agcaaataat tcaaccacta aacaaatcaa ccgcgtttcc cggaggtaac 180
   c 181
<210> 63
   <211> 201
   <212> DNA
   <213> artificial sequence
<220>
   <223> Pgi PCR fragment
<400> 63
   gggagctcac tagtcgatct gtgctgtttg ccacggtatg cagcaccagc gcgagattat 60
   gggctcgcac gctcgactgt cggacggggg cactggaacg agaagtcagg cgagccgtca 120
   cgcccttgac aatgccacat cctgagcaaa taattcaacc actaaacaaa tcaaccgcgt 180
   ttcccggagg taaccatggc t 201
<210> 64
   <211> 911
   <212> DNA
   <213> artificial sequence
<220>
   <223> chloramphenicol resistance marker
<400> 64
   gtgacggaag atcacttcgc agaataaata aatcctggtg tccctgttga taccgggaag 60
   ccctgggcca acttttggcg aaaatgagac gttgatcggc acgtaagagg ttccaacttt 120
   caccataatg aaataagatc actaccgggc gtattttttg agttatcgag attttcagga 180
   gctaaggaag ctaaaatgga gaaaaaaatc actggatata ccaccgttga tatatcccaa 240
   tggcatcgta aagaacattt tgaggcattt cagtcagttg ctcaatgtac ctataaccag 300
   accgttcagc tggatattac ggccttttta aagaccgtaa agaaaaataa gcacaagttt 360
   tatccggcct ttattcacat tcttgcccgc ctgatgaatg ctcatccgga attccgtatg 420
   gcaatgaaag acggtgagct ggtgatatgg gatagtgttc acccttgtta caccgttttc 480
   catgagcaaa ctgaaacgtt ttcatcgctc tggagtgaat accacgacga tttccggcag 540
   tttctacaca tatattcgca agatgtggcg tgttacggtg aaaacctggc ctatttccct 600
   aaagggttta ttgagaatat gtttttcgtc tcagccaatc cctgggtgag tttcaccagt 660
   tttgatttaa acgtggccaa tatggacaac ttcttcgccc ccgttttcac catgggcaaa 720
   tattatacgc aaggcgacaa ggtgctgatg ccgctggcga ttcaggttca tcatgccgtt 780
   tgtgatggct tccatgtcgg cagaatgctt aatgaattac aacagtactg cgatgagtgg 840
   cagggcgggg cgtaattttt ttaaggcagt tattggtgcc cttaaacgcc tggttgctac 900
   gcctgaataa g 911
<210> 65
   <211> 7224
   <212> DNA
   <213> artificial sequence
<220>
   <223> constructed plasmid
<400> 65
   ggcttactat gttggcactg atgagggtgt cagtgaagtg cttcatgtgg caggagaaaa      60
   aaggctgcac cggtgcgtca gcagaatatg tgatacagga tatattccgc ttcctcgctc     120
   actgactcgc tacgctcggt cgttcgactg cggcgagcgg aaatggctta cgaacggggc     180
   ggagatttcc tggaagatgc caggaagata cttaacaggg aagtgagagg gccgcggcaa     240
   agccgttttt ccataggctc cgcccccctg acaagcatca cgaaatctga cgctcaaatc     300
   agtggtggcg aaacccgaca ggactataaa gataccaggc gtttccccct ggcggctccc     360
   tcgtgcgctc tcctgttcct gcctttcggt ttaccggtgt cattccgctg ttatggccgc     420
   gtttgtctca ttccacgcct gacactcagt tccgggtagg cagttcgctc caagctggac     480
   tgtatgcacg aaccccccgt tcagtccgac cgctgcgcct tatccggtaa ctatcgtctt     540
   gagtccaacc cggaaagaca tgcaaaagca ccactggcag cagccactgg taattgattt     600
   agaggagtta gtcttgaagt catgcgccgg ttaaggctaa actgaaagga caagttttgg     660
   tgactgcgct cctccaagcc agttacctcg gttcaaagag ttggtagctc agagaacctt     720
   cgaaaaaccg ccctgcaagg cggttttttc gttttcagag caagagatta cgcgcagacc 780
   aaaacgatct caagaagatc atcttattaa tcagataaaa tatttctaga tttcagtgca 840
   atttatctct tcaaatgtag cacctgaagt cagccccata cgatataagt tgtaattctc 900
   atgtttgaca gcttatcatc gatggagcac aggatgacgc ctaacaattc attcaagccg 960
   acaccgcttc gcggcgcggc ttaattcagg agttaaacat catgagggaa gcggtgatcg 1020
   ccgaagtatc gactcaacta tcagaggtag ttggcgtcat cgagcgccat ctcgaaccga 1080
   cgttgctggc cgtacatttg tacggctccg cagtggatgg cggcctgaag ccacacagtg 1140
   atattgattt gctggttacg gtgactgtaa ggcttgatga aacaacgcgg cgagctttga 1200
   tcaacgacct tttggaaact tcggcttccc ctggagagag cgagattctc cgcgctgtag 1260
   aagtcaccat tgttgtgcac gacgacatca ttccgtggcg ttatccagct aagcgcgaac 1320
   tgcaatttgg agaatggcag cgcaatgaca ttcttgcagg tatcttcgag ccagccacga 1380
   tcgacattga tctggctatc ttgctgacaa aagcaagaga acatagcgtt gccttggtag 1440
   gtccagcggc ggaggaactc tttgatccgg ttcctgaaca ggatctattt gaggcgctaa 1500
   atgaaacctt aacgctatgg aactcgccgc ccgactgggc tggcgatgag cgaaatgtag 1560
   tgcttacgtt gtcccgcatt tggtacagcg cagtaaccgg caaaatcgcg ccgaaggatg 1620
   tcgctgccga ctgggcaatg gagcgcctgc cggcccagta tcagcccgtc atacttgaag 1680
   ctaggcaggc ttatcttgga caagaagatc gcttggcctc gcgcgcagat cagttggaag 1740
   aatttgttca ctacgtgaaa ggcgagatca ccaaggtagt cggcaaataa tgtctaacaa 1800
   ttcgttcaag ccgacgccgc ttcgcggcgc ggcttaactc aagcgttaga gagctgggga 1860
   agactatgcg cgatctgttg aaggtggttc taagcctcgt acttgcgatg gcatcggggc 1920
   aggcacttgc tgacctgcca acgcgccttt gtagtcttgg cctgttgtgt gcatgagcaa 1980
   atcaatggca ccaccccctc ctttttgagc tgaatggtca taaaatttat aattatctat 2040
   cgtaattcgg aatctatgtt cagggtctcg ccattgcttt ttgtctgctg ggtcaagttc 2100
   catgcctaag gtttttaaga catcagaaag aggtattgca cgcatgctat cagcttttct 2160
   tctagctaat gacagggctt cctctgctct atctgctcgt tttttttctt ccacatatct 2220
   cgccgctttg tcagccagcg gctgtattac ggaaagtgcc gatttttggg cttttaggcg 2280
   ttctttttct gcccattctt ccttatttgt aaaaattgag ggtgggatgg gtgcctgaat 2340
   cttgggatct agctgtaaag ttttgttgat atttccgtaa tgtctttgga ctctttgatg 2400
   cgttgctttt gaacctttta cgcctctggc cagccctaga ggctccatag aagccgcata 2460
   atccgtctgg agggcagaaa gggcttttcg accatcaaac catctcgatg cgtttaaacg 2520
   gcctgtatcg gggtctctag gcaccataaa gccggttaag tggggtgttg tttcatcagc 2580
   atgtagctga agagatacaa ggttgttttc tccaaaggtt tgttccgccc attgctgggt 2640
   gattgttttc cagtgttcga gtttttcagg agtggcctgt tttgaccatt ctggagacat    2700
   accaaagaac agttctatgg cctgcacacc gttttttcta agaggctttc ccgtttcttt   2760
   ctgaatttta ttcagcatag atttaacatc tgctgatggg tcagtagagc ctttgagtat 2820
   ttcgtttagt tcttttctat ctgggtcagc gttttgtgtt tcgcggcctc gcgtcatatg 2880
   caggctcgcg gctttaatcg tgccaactgt tttatgtttt tcaaacctaa agattgcata 2940
   gttcggcatg ttttaactgc tttaatttga gaaaagacca gaggaaataa tccagcctat 3000
   atttctttcc ctagtagcga actggaattg tttttccgaa ggaaaaaagc aattccgtag 3060
   tgagtactga atttattctg attcgtcttg cttttggagc gtctttttgc gttctataac 3120
   tgttgtgaaa gctacgcggt cgccattgaa aacgaaatta ggattaataa aataccatcc 3180
   ttggcgaaca tgctttgcaa tgattttagc tttttctaat tcggctagac ctcttgcaaa 3240
   ggtagcttga gatagtgcca gttttttttc ttgtgcgtta agaaagtcct ctaaaacgaa 3300
   tttgtctaaa gggacgaggt ctttgctgat gcctttgtct tgaagtatcc aaaccagaac 3360
   gctgaaagct tttattccag cggctcctag ttcaaaagtt agcgcgatat tggtgctaaa 3420
   taattttaca aattcttcac tatcaacacg tctgtaagtc gtcacatgag tgccttgcat 3480
   ctcaccagtg gcttgattga ccagaatgtt atcatctcgt cctaatcgag ataactgaac 3540
   cctctgactt ttaactggca caaccatacc ttcgatgaaa ggattctcgt catatctgat 3600
   tggctgcttt ctcaattttg tcgccatatt tgataaacct ttaatcaaaa aaaccacatt 3660
   ttttgattat acctattcat cgaatgaggc aaggtctatc aattttaccc ctttttttga 3720
   tagacggttt aatcaatatt gatagacccc ttcacagatt ctgaaaatcg acttccctat 3780
   tttagggata ttttcacgat tccctttctt agttcttcct agtggggaaa ttcgttgaat 3840
   cctgcctcgg aaaaaccatg agaaagctgt tggttatata cacgggcaaa gccaccctat 3900
   ttttagctac tggggaaaga gataaggcag ggtatttgta aaattaaaac cggatttttc 3960
   gctttacggt ttgtttaggc gcaactgtct ttttaagacc gcgtttaacc atcaaaagat 4020
   cgttccaatc ttttccgtgt atcatctgtt ctttaggtgg gagccagttt tcaacttttt 4080
   ttgttggaaa cgcggcttta atcgctccga ctaatagcga tgctgctctt tgtcctacag 4140
   catcccaatc ataggcaata tggacagaag atgccttttc aacgattttt cggagagttt 4200
   tagtaagaga cgttcttacg ccgctggtgc ttaataattt tacgccagct ttaatttttt 4260
   ctgggcttaa aaagccgact actgaaatcg cgtctatcgc actttcagcg atataaagat 4320
   catacttttc gtcatttttt acattgatgc tgccagtaaa atgggcttcg cgactgcttc 4380
   ccaaggctaa ccctttaaaa ccactgcttg ttccgcgtaa ttctgcgccc tgaagtgtat 4440
   ctttatcgtc atacatcaag aaggctacat taccgcgatc atctgttcgg atagagtcag 4500
   gaatattgtt aaatgatatt cctcggctag tcgatctgtg ctgtttgcca cggtatgcag 4560
   caccagcgcg agattatggg ctcgcacgct cgactgtcgg acgggggcac tggaacgaga 4620
   agtcaggcga gccgtcacgc ccttgacaat gccacatcct gagcaaataa ttcaaccact 4680
   aaacaaatca accgcgtttc ccggaggtaa ccatggttac tatcaatacg gaatctgctt 4740
   taacgccacg ttctttgcgg gatacgcggc gtatgaatat gtttgtttcg gtagctgctg 4800
   cggtcgcagg attgttattt ggtcttgata tcggcgtaat cgccggagcg ttgccgttca 4860
   ttaccgatca ctttgtgctg accagtcgtt tgcaggaatg ggtggttagt agcatgatgc 4920
   tcggtgcagc aattggtgcg ctgtttaatg gttggctgtc gttccgcctg gggcgtaaat 4980
   acagcctgat ggcgggggcc atcctgtttg tactcggttc tatagggtcc gcttttgcga 5040
   ccagcgtaga gatgttaatc gccgctcgtg tggtgctggg cattgctgtc gggatcgcgt 5100
   cttacaccgc tcctctgtat ctttctgaaa tggcaagtga aaacgttcgc ggtaagatga 5160
   tcagtatgta ccagttgatg gtcacactcg gcatcgtgct ggcgttttta tccgatacag 5220
   cgttcagtta tagcggtaac tggcgcgcaa tgttgggggt tcttgcttta ccagcagttc 5280
   tgctgattat tctggtagtc ttcctgccaa atagcccgcg ctggctggcg gaaaaggggc 5340
   gtcatattga ggcggaagaa gtattgcgta tgctgcgcga tacgtcggaa aaagcgcgag 5400
   aagaactcaa cgaaattcgt gaaagcctga agttaaaaca gggcggttgg gcactgttta 5460
   agatcaaccg taacgtccgt cgtgctgtgt ttctcggtat gttgttgcag gcgatgcagc 5520
   agtttaccgg tatgaacatc atcatgtact acgcgccgcg tatcttcaaa atggcgggct 5580
   ttacgaccac agaacaacag atgattgcga ctctggtcgt agggctgacc tttatgttcg 5640
   ccacctttat tgcggtgttt acggtagata aagcagggcg taaaccggct ctgaaaattg 5700
   gtttcagcgt gatggcgtta ggcactctgg tgctgggcta ttgcctgatg cagtttgata 5760
   acggtacggc ttccagtggc ttgtcctggc tctctgttgg catgacgatg atgtgtattg 5820
   ccggttatgc gatgagcgcc gcgccagtgg tgtggatcct gtgctctgaa attcagccgc 5880
   tgaaatgccg cgatttcggt attacctgtt cgaccaccac gaactgggtg tcgaatatga 5940
   ttatcggcgc gaccttcctg acactgcttg atagcattgg cgctgccggt acgttctggc 6000
   tctacactgc gctgaacatt gcgtttgtgg gcattacttt ctggctcatt ccggaaacca 6060
   aaaatgtcac gctggaacat atcgaacgca aactgatggc aggcgagaag ttgagaaata 6120
   tcggcgtctg atttcacggg ccggatgtgc tgtacatccg gccctttttt cgttaataga 6180
   gattgggcac ttggccgttg aggcgtttgt ctcgttcctt attcagcctt gttgcggtaa 6240
   cacacatcag gaattctgca gatatccatc acactggcgg ccgcgtgacg gaagatcact 6300
   tcgcagaata aataaatcct ggtgtccctg ttgataccgg gaagccctgg gccaactttt 6360
   ggcgaaaatg agacgttgat cggcacgtaa gaggttccaa ctttcaccat aatgaaataa 6420
   gatcactacc gggcgtattt tttgagttat cgagattttc aggagctaag gaagctaaaa 6480
   tggagaaaaa aatcactgga tataccaccg ttgatatatc ccaatggcat cgtaaagaac 6540
   attttgaggc atttcagtca gttgctcaat gtacctataa ccagaccgtt cagctggata 6600
   ttacggcctt tttaaagacc gtaaagaaaa ataagcacaa gttttatccg gcctttattc 6660
   acattcttgc ccgcctgatg aatgctcatc cggaattccg tatggcaatg aaagacggtg 6720
   agctggtgat atgggatagt gttcaccctt gttacaccgt tttccatgag caaactgaaa 6780
   cgttttcatc gctctggagt gaataccacg acgatttccg gcagtttcta cacatatatt 6840
   cgcaagatgt ggcgtgttac ggtgaaaacc tggcctattt ccctaaaggg tttattgaga 6900
   atatgttttt cgtctcagcc aatccctggg tgagtttcac cagttttgat ttaaacgtgg 6960
   ccaatatgga caacttcttc gcccccgttt tcaccatggg caaatattat acgcaaggcg 7020
   acaaggtgct gatgccgctg gcgattcagg ttcatcatgc cgtttgtgat ggcttccatg 7080
   tcggcagaat gcttaatgaa ttacaacagt actgcgatga gtggcagggc ggggcgtaat 7140
   ttttttaagg cagttattgg tgcccttaaa cgcctggttg ctacgcctga ataagttaat 7200
   taatgcgcta gcggagtgta tact 7224
<210> 66
   <211> 9418
   <212> DNA
   <213> artificial sequence
<220>
   <223> constructed plasmid
<400> 66
   ctagtcgatc tgtgctgttt gccacggtat gcagcaccag cgcgagatta tgggctcgca 60
   cgctcgactg tcggacgggg gcactggaac gagaagtcag gcgagccgtc acgcccttga 120
   caatgccaca tcctgagcaa ataattcaac cactaaacaa atcaaccgcg tttcccggag 180
   gtaaccatgg cgcacaaatt tactaaagcc ctggcagcca ttggtctggc agccgttatg 240
   tcacaatccg ctatggcgga gaacctgaag ctcggttttc tggtgaagca accggaagag 300
   ccgtggttcc agaccgaatg gaagtttgcc gataaagccg ggaaggattt agggtttgag 360
   gttattaaga ttgccgtgcc ggatggcgaa aaaacattga acgcgatcga cagcctggct 420
   gccagtggcg caaaaggttt cgttatttgt actccggacc ccaaactcgg ctctgccatc 480
   gtcgcgaaag cgcgtggcta cgatatgaaa gtcattgccg tggatgacca gtttgttaac 540
   gccaaaggta agccaatgga taccgttccg ctggtgatga tggcggcgac taaaattggc 600
   gaacgtcagg gccaggaact gtataaagag atgcagaaac gtggctggga tgtcaaagaa 660
   agcgcggtga tggcgattac cgccaacgaa ctggataccg cccgccgccg tactacggga 720
   tctatggatg cgctgaaagc ggccggattc ccggaaaaac aaatttatca ggtacctacc 780
   aaatctaacg acatcccggg ggcatttgac gctgccaact caatgctggt tcaacatccg 840
   gaagttaaac attggctgat cgtcggtatg aacgacagca ccgtgctggg cggcgtacgc 900
   gcgacggaag gtcagggctt taaagcggcc gatatcatcg gcattggcat taacggtgtg 960
   gatgcggtga gcgaactgtc taaagcacag gcaaccggct tctacggttc cctgctgcca 1020
   agcccggacg tacatggcta taaatccagc gaaatgcttt acaactgggt agcaaaagac 1080
   gttgaaccgc caaaatttac cgaagttacc gacgtggtac tgatcacgcg tgacaacttt 1140
   aaagaagaac tggagaaaaa aggtttaggc ggtaagtaat ttgccggaaa aattcccctc 1200
   tgcatgatgc agagggggtg tgaacgacca gtgattcacg gagacgttat gcaacagtct 1260
   accccgtatc tctcatttcg cggcatcggt aaaacgtttc ccggcgttaa ggcgctgacg 1320
   gatattagtt ttgactgcta tgccggtcag gttcatgcgt tgatgggtga aaatggcgca 1380
   ggaaaatcaa ctctcttaaa aatcctcagc ggcaactatg cgccaaccac gggttctgta 1440
   gtgattaatg ggcaggaaat gtccttttcc gacacgaccg cagcacttaa cgcgggcgtg 1500
   gcgattattt accaggaact gcatctcgtg ccggaaatga ccgtcgcgga aaacatctat 1560
   ctcggccagc tgccgcataa aggcggcatt gtgaatcgct cattgctgaa ttatgaggcg 1620
   ggtttacaac ttaaacatct tggtatggat attgacccgg acacgccgct gaaatatctc 1680
   tccattggtc agtggcagat ggttgaaatc gccaaagcgc tggcgcgtaa cgccaaaatt 1740
   atcgcctttg atgagccaac cagctccctc tctgcccgtg aaatcgacaa tcttttccgc 1800
   gttattcgtg aactgcgaaa agaggggcgg gtaatcttat acgtttctca ccgtatggaa 1860
   gaaatatttg ccctcagcga tgccattact gtctttaaag atggacgtta tgtcaaaacc 1920
   tttaccgata tgcagcaggt tgaccacgac gcgctggtgc aggcgatggt cgggcgcgac 1980
   attggcgata tctacggctg gcaaccgcgt agttatggcg aggagcgcct acgtcttgat 2040
   gctgtgaaag caccaggcgt gcgtacgcca ataagtctgg cggttcgcag tggtgaaatt 2100
   gttgggctgt ttggtctggt aggggcgggg cgtagcgaat taatgaaagg catgtttggc 2160
   gggacgcaaa tcaccgccgg tcaggtttat atcgaccaac agccgatcga tattcgtaaa 2220
   ccgagccacg ccattgccgc aggcatgatg ctctgcccgg aagatcgcaa agcggaaggc 2280
   attattcccg tgcactccgt tcgcgacaat atcaacatca gtgccagacg taaacatgtg 2340
   ctcggcggtt gtgtaatcaa caacggttgg gaagaaaaca atgccgatca ccacattcgt 2400
   tcgctcaaca tcaaaacgcc gggcgcggag caactgatca tgaatctctc aggcggaaat 2460
   cagcaaaaag ccattctggg ccgctggtta tcggaagaga tgaaggtcat tttgctggat 2520
   gaacctacgc gcggcattga tgttggcgct aagcacgaaa tatataacgt aatttatgcg 2580
   ctggcggcgc agggcgtggc ggtgctgttt gcctccagcg acttacctga agtcctcggc 2640
   gttgccgacc ggattgtggt gatgcgggaa ggtgaaatcg ccggtgaatt gttacacgag 2700
   caggcagatg agcgtcaggc actgagcctt gcgatgccta aagtcagcca ggctgttgcc 2760
   tgagtaagga gagtatgatg tcttctgttt ctacatcggg gtctggcgca cctaagtcgt 2820
   cattcagctt cgggcgtatc tgggatcagt acggcatgct ggtggtgttt gcggtgctct 2880
   ttatcgcctg tgccattttt gtcccaaatt ttgccacctt cattaatatg aaagggttgg 2940
   gcctggcaat ttccatgtcg gggatggtgg cttgtggcat gttgttctgc ctcgcttccg 3000
   gtgactttga cctttctgtc gcctccgtaa ttgcctgtgc gggtgtcacc acggcggtgg 3060
   ttattaacct gactgaaagc ctgtggattg gcgtggcagc ggggttgttg ctgggcgttc 3120
   tctgtggcct ggtcaatggc tttgttatcg ccaaactgaa aataaatgct ctgatcacga 3180
   cattggcaac gatgcagatt gttcgaggtc tggcgtacat catttcagac ggtaaagcgg 3240
   tcggtatcga agatgaaagc ttctttgccc ttggttacgc caactggttc ggtctgcctg 3300
   cgccaatctg gctcaccgtc gcgtgtctga ttatctttgg tttgctgctg aataaaacca 3360
   cctttggtcg taacaccctg gcgattggcg ggaacgaaga ggccgcgcgt ctggcgggtg 3420
   taccggttgt tcgcaccaaa attattatct ttgttctctc aggcctggta tcagcgatag 3480
   ccggaattat tctggcttca cgtatgacca gtgggcagcc aatgacgtcg attggttatg 3540
   agctgattgt tatctccgcc tgcgttttag gtggcgtttc tctgaaaggt ggcatcggaa 3600
   aaatctcata tgtggtggcg ggtatcttaa ttttaggcac cgtggaaaac gccatgaacc 3660
   tgcttaatat ttctcctttc gcgcagtacg tggttcgcgg cttaatcctg ctggcagcgg 3720
   tgatcttcga ccgttacaag caaaaagcga aacgcactgt ctgatgcttt tttctgcaac 3780
   aatttagcgt tttttcccac catagccaac cgccataacg gttggctgtt cttcgttgca 3840
   aatggcgacc cccgtcacac tgtctatact tacatgtctg taaagcgcgt tctgcgcaac 3900
   acaataagaa aagagaagga attctgcaga tatccatcac actggcggcc gcgtgacgga 3960
   agatcacttc gcagaataaa taaatcctgg tgtccctgtt gataccggga agccctgggc 4020
   caacttttgg cgaaaatgag acgttgatcg gcacgtaaga ggttccaact ttcaccataa 4080
   tgaaataaga tcactaccgg gcgtattttt tgagttatcg agattttcag gagctaagga 4140
   agctaaaatg gagaaaaaaa tcactggata taccaccgtt gatatatccc aatggcatcg 4200
   taaagaacat tttgaggcat ttcagtcagt tgctcaatgt acctataacc agaccgttca 4260
   gctggatatt acggcctttt taaagaccgt aaagaaaaat aagcacaagt tttatccggc 4320
   ctttattcac attcttgccc gcctgatgaa tgctcatccg gaattccgta tggcaatgaa 4380
   agacggtgag ctggtgatat gggatagtgt tcacccttgt tacaccgttt tccatgagca 4440
   aactgaaacg ttttcatcgc tctggagtga ataccacgac gatttccggc agtttctaca 4500
   catatattcg caagatgtgg cgtgttacgg tgaaaacctg gcctatttcc ctaaagggtt 4560
   tattgagaat atgtttttcg tctcagccaa tccctgggtg agtttcacca gttttgattt 4620
   aaacgtggcc aatatggaca acttcttcgc ccccgttttc accatgggca aatattatac 4680
   gcaaggcgac aaggtgctga tgccgctggc gattcaggtt catcatgccg tttgtgatgg 4740
   cttccatgtc ggcagaatgc ttaatgaatt acaacagtac tgcgatgagt ggcagggcgg 4800
   ggcgtaattt ttttaaggca gttattggtg cccttaaacg cctggttgct acgcctgaat 4860
   aagttaatta atgcgctagc ggagtgtata ctggcttact atgttggcac tgatgagggt 4920
   gtcagtgaag tgcttcatgt ggcaggagaa aaaaggctgc accggtgcgt cagcagaata 4980
   tgtgatacag gatatattcc gcttcctcgc tcactgactc gctacgctcg gtcgttcgac 5040
   tgcggcgagc ggaaatggct tacgaacggg gcggagattt cctggaagat gccaggaaga 5100
   tacttaacag ggaagtgaga gggccgcggc aaagccgttt ttccataggc tccgcccccc 5160
   tgacaagcat cacgaaatct gacgctcaaa tcagtggtgg cgaaacccga caggactata 5220
   aagataccag gcgtttcccc ctggcggctc cctcgtgcgc tctcctgttc ctgcctttcg 5280
   gtttaccggt gtcattccgc tgttatggcc gcgtttgtct cattccacgc ctgacactca 5340
   gttccgggta ggcagttcgc tccaagctgg actgtatgca cgaacccccc gttcagtccg 5400
   accgctgcgc cttatccggt aactatcgtc ttgagtccaa cccggaaaga catgcaaaag 5460
   caccactggc agcagccact ggtaattgat ttagaggagt tagtcttgaa gtcatgcgcc 5520
   ggttaaggct aaactgaaag gacaagtttt ggtgactgcg ctcctccaag ccagttacct 5580
   cggttcaaag agttggtagc tcagagaacc ttcgaaaaac cgccctgcaa ggcggttttt 5640
   tcgttttcag agcaagagat tacgcgcaga ccaaaacgat ctcaagaaga tcatcttatt 5700
   aatcagataa aatatttcta gatttcagtg caatttatct cttcaaatgt agcacctgaa 5760
   gtcagcccca tacgatataa gttgtaattc tcatgtttga cagcttatca tcgatggagc 5820
   acaggatgac gcctaacaat tcattcaagc cgacaccgct tcgcggcgcg gcttaattca 5880
   ggagttaaac atcatgaggg aagcggtgat cgccgaagta tcgactcaac tatcagaggt 5940
   agttggcgtc atcgagcgcc atctcgaacc gacgttgctg gccgtacatt tgtacggctc 6000
   cgcagtggat ggcggcctga agccacacag tgatattgat ttgctggtta cggtgactgt 6060
   aaggcttgat gaaacaacgc ggcgagcttt gatcaacgac cttttggaaa cttcggcttc 6120
   ccctggagag agcgagattc tccgcgctgt agaagtcacc attgttgtgc acgacgacat 6180
   cattccgtgg cgttatccag ctaagcgcga actgcaattt ggagaatggc agcgcaatga 6240
   cattcttgca ggtatcttcg agccagccac gatcgacatt gatctggcta tcttgctgac 6300
   aaaagcaaga gaacatagcg ttgccttggt aggtccagcg gcggaggaac tctttgatcc 6360
   ggttcctgaa caggatctat ttgaggcgct aaatgaaacc ttaacgctat ggaactcgcc 6420
   gcccgactgg gctggcgatg agcgaaatgt agtgcttacg ttgtcccgca tttggtacag 6480
   cgcagtaacc ggcaaaatcg cgccgaagga tgtcgctgcc gactgggcaa tggagcgcct 6540
   gccggcccag tatcagcccg tcatacttga agctaggcag gcttatcttg gacaagaaga 6600
   tcgcttggcc tcgcgcgcag atcagttgga agaatttgtt cactacgtga aaggcgagat 6660
   caccaaggta gtcggcaaat aatgtctaac aattcgttca agccgacgcc gcttcgcggc 6720
   gcggcttaac tcaagcgtta gagagctggg gaagactatg cgcgatctgt tgaaggtggt 6780
   tctaagcctc gtacttgcga tggcatcggg gcaggcactt gctgacctgc caacgcgcct 6840
   ttgtagtctt ggcctgttgt gtgcatgagc aaatcaatgg caccaccccc tcctttttga 6900
   gctgaatggt cataaaattt ataattatct atcgtaattc ggaatctatg ttcagggtct 6960
   cgccattgct ttttgtctgc tgggtcaagt tccatgccta aggtttttaa gacatcagaa 7020
   agaggtattg cacgcatgct atcagctttt cttctagcta atgacagggc ttcctctgct 7080
   ctatctgctc gttttttttc ttccacatat ctcgccgctt tgtcagccag cggctgtatt 7140
   acggaaagtg ccgatttttg ggcttttagg cgttcttttt ctgcccattc ttccttattt 7200
   gtaaaaattg agggtgggat gggtgcctga atcttgggat ctagctgtaa agttttgttg 7260
   atatttccgt aatgtctttg gactctttga tgcgttgctt ttgaaccttt tacgcctctg 7320
   gccagcccta gaggctccat agaagccgca taatccgtct ggagggcaga aagggctttt 7380
   cgaccatcaa accatctcga tgcgtttaaa cggcctgtat cggggtctct aggcaccata 7440
   aagccggtta agtggggtgt tgtttcatca gcatgtagct gaagagatac aaggttgttt 7500
   tctccaaagg tttgttccgc ccattgctgg gtgattgttt tccagtgttc gagtttttca 7560
   ggagtggcct gttttgacca ttctggagac ataccaaaga acagttctat ggcctgcaca 7620
   ccgttttttc taagaggctt tcccgtttct ttctgaattt tattcagcat agatttaaca 7680
   tctgctgatg ggtcagtaga gcctttgagt atttcgttta gttcttttct atctgggtca 7740
   gcgttttgtg tttcgcggcc tcgcgtcata tgcaggctcg cggctttaat cgtgccaact 7800
   gttttatgtt tttcaaacct aaagattgca tagttcggca tgttttaact gctttaattt 7860
   gagaaaagac cagaggaaat aatccagcct atatttcttt ccctagtagc gaactggaat 7920
   tgtttttccg aaggaaaaaa gcaattccgt agtgagtact gaatttattc tgattcgtct 7980
   tgcttttgga gcgtcttttt gcgttctata actgttgtga aagctacgcg gtcgccattg 8040
   aaaacgaaat taggattaat aaaataccat ccttggcgaa catgctttgc aatgatttta 8100
   gctttttcta attcggctag acctcttgca aaggtagctt gagatagtgc cagttttttt 8160
   tcttgtgcgt taagaaagtc ctctaaaacg aatttgtcta aagggacgag gtctttgctg 8220
   atgcctttgt cttgaagtat ccaaaccaga acgctgaaag cttttattcc agcggctcct 8280
   agttcaaaag ttagcgcgat attggtgcta aataatttta caaattcttc actatcaaca 8340
   cgtctgtaag tcgtcacatg agtgccttgc atctcaccag tggcttgatt gaccagaatg 8400
   ttatcatctc gtcctaatcg agataactga accctctgac ttttaactgg cacaaccata 8460
   ccttcgatga aaggattctc gtcatatctg attggctgct ttctcaattt tgtcgccata 8520
   tttgataaac ctttaatcaa aaaaaccaca ttttttgatt atacctattc atcgaatgag 8580
   gcaaggtcta tcaattttac cccttttttt gatagacggt ttaatcaata ttgatagacc 8640
   ccttcacaga ttctgaaaat cgacttccct attttaggga tattttcacg attccctttc 8700
   ttagttcttc ctagtgggga aattcgttga atcctgcctc ggaaaaacca tgagaaagct 8760
   gttggttata tacacgggca aagccaccct atttttagct actggggaaa gagataaggc   8820
   agggtatttg taaaattaaa accggatttt tcgctttacg gtttgtttag gcgcaactgt   8880
   ctttttaaga ccgcgtttaa ccatcaaaag atcgttccaa tcttttccgt gtatcatctg   8940
   ttctttaggt gggagccagt tttcaacttt ttttgttgga aacgcggctt taatcgctcc   9000
   gactaatagc gatgctgctc tttgtcctac agcatcccaa tcataggcaa tatggacaga   9060
   agatgccttt tcaacgattt ttcggagagt tttagtaaga gacgttctta cgccgctggt   9120
   gcttaataat tttacgccag ctttaatttt ttctgggctt aaaaagccga ctactgaaat   9180
   cgcgtctatc gcactttcag cgatataaag atcatacttt tcgtcatttt ttacattgat   9240
   gctgccagta aaatgggctt cgcgactgct tcccaaggct aaccctttaa aaccactgct   9300
   tgttccgcgt aattctgcgc cctgaagtgt atctttatcg tcatacatca agaaggctac   9360
   attaccgcga tcatctgttc ggatagagtc aggaatattg ttaaatgata ttcctcgg     9418